# EUROPEAN PATENT APPLICATION

(11) **EP 0 818 455 A1**
(43) Date of publication of application: **14.01.1998**
(21) Application number: 96907678.5
(22) Date of filing: 28.03.1996
(51) Int. Cl.: C07D 409/10, C07D 335/06, A01N 43/56

(54) **PYRAZOLE DERIVATIVES**

(30) Priority: 28.03.1995 JP 69760/95; 26.06.1995 JP 158842/95
(71) Applicant: IDEMITSU KOSAN COMPANY LIMITED, Tokyo 100 (JP)
(72) Inventor: SHIBATA, Mitsuru, Idemitsu Kosan Co. Ltd., Chiba-ken 299-02 (JP); SAKAMOTO, Masashi, Idemitsu Kosan Co. Ltd., Chiba-ken 299-02 (JP); KAMANO, Hideki, Idemitsu Kosan Co. Ltd., Chiba-ken 299-02 (JP); YAMAMOTO, Hiroshi, Idemitsu Kosan Co. Ltd., Chiba-ken 299-02 (JP)
(74) Representative: Türk, Gille, Hrabal, Leifert
(86) International application number: JP9600811
(87) International publication number: WO9630368

(57) **Abstract**

The present invention relates to pyrazole derivatives of the formula (I), 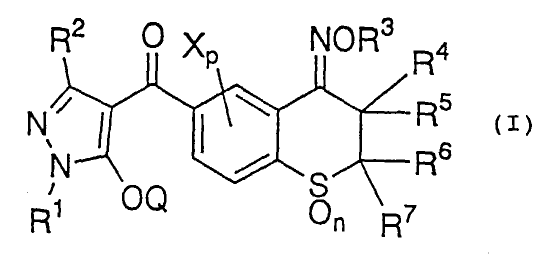 and herbicides containing them as active ingredients. According to the present invention, novel pyrazole derivatives and herbicides containing them as active ingredients which exhibit excellent selectivity between crops and weeds in foliar treatment and soil treatment are provided.

## Description

### Technical Field

The present invention relates to pyrazole derivatives and herbicides containing the pyrazole derivatives as active ingredients.

### Technical Background

It is indespensable to use a herbicide for protecting , and increasing the yields of, useful crops such as rice, wheat, barley, corn, soybean, cotton, beet and the like. In recent years in particular, there are desired selective herbicides which cause no phytotoxicity on the useful crops but selectively control weeds alone by foliar treatment of the crops and the weeds at the same time in a cultivated field where the useful crops and weeds are concurrently present.

During the planting time of corn, etc., triazine-based herbicides such as atrazine and acid anilide-based herbicides such as alachlor and metolachlor have been conventionally used as soil treating chemical. However, these herbicides require high dosage and are threfore causing environmental problems such as pollution of ground water.

In recent years, further, "non-tilled cropping" intended for soil conservation is promoted. "Non-tilled cropping" is a method of cultivating crops without plowing, and is against usual tilled cropping. In tilled cropping, fertile surface soil which is tilled (plowed) is washed away by rain, etc., which is not only a great farming problem but also a problem which leads to a kind of desertification. On the other hand, in non-tilled cropping, there is no problem of surface soil being washed away. However, without tilling, soil is hardened so that a chemical is scarcely infiltrated into the soli, and the effect of the chemical on soil treatment decreases. In the non-tilled cropping, therefore, there are demanded herbicides which have high herbicidal efficacy at a low dosage in soil treatment and which can be used as a single chemical for foliar treatment as well.

International Laid-open Patent Publication No. WO94/01431 discloses pyrazole derivatives having a thiochroman ring, represented by the following formula. 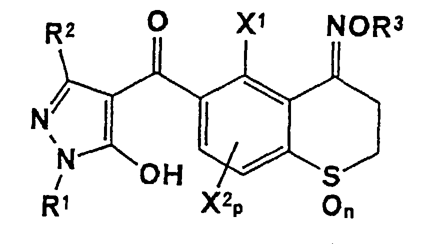 wherein each of X¹ and X² is a C₁∼C₄ alkyl group and p showing the number of substituents as X² is 0 or 1. Explanations of the other symbols are omitted.

Typical compound (A) of the above International Laid-open Patent Publication has the following structural formula.

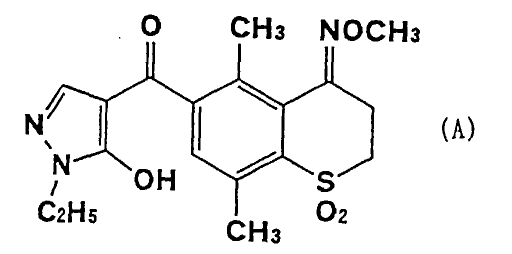

The above compound (A) shows excellent selectively between crops and weeds without damaging cultivated crops when used for the treatment of corn, wheat, barley, etc., at the stage of 1 ∼ 2 leaves. However, when the treatment is carried out at the stage of 3 ∼ 4 leaves, there is room for improvement concerning damage on crops.

### Disclosure of the Invention

It is a first object of the present invention to provide a novel pyrazole derivative which has herbicidal activity with high selectivity, or which is safe to cultivated crops such as corn and can control a broad range of weeds at a low dosage in foliar treatment, particularly, foliar treatment in non-tilled cropping.

Further, it is a second object of the present invention to provide an intermediate compound useful for the production of the above novel pyrazole derivative, and it is further a third object of the present invention to provide a herbicide containing the above novel pyrazole derivative as an active ingredient.

The present inventors have made diligent studies for creating novel pyrazole derivatives which can achieve the above objects, and have found the following. Compounds obtained by replacing one or both of X¹ and X² in the structural formula of the pyrazole derivative of the above International Laid-open Patent Publication by substituent(s) other than C₁∼C₄ alkyl group have remarkably high herbicidal spectrum against a broad range of weeds, have high safety to wheat, barley, corn, soybean, cotton, beet, rice, etc., and exhibit excellent selectivity between crops and weeds in any one of foliar treatment and soil treatment. The present invention has been completed on the basis of the above finding.

That is, the first object of the present invention is achieved by a pyrazole derivative of the formula (I) (to be sometimes referred to as "pyrazole derivative (I)" hereinafter), 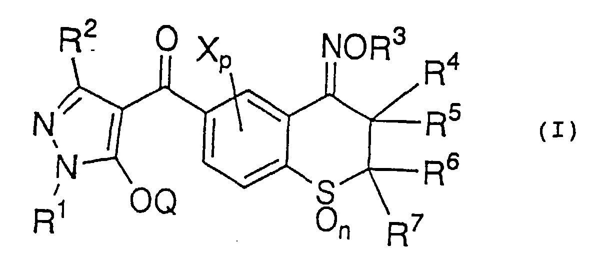 wherein:
R¹ is a C₁∼C₄ alkyl group, a C₂∼C₄ alkenyl group or a C₂∼C₄ haloalkenyl group;
R² is a hydrogen atom, a C₁∼C₄ alkyl group, a C₁∼C₄ haloalkyl group or a C₂∼C₄ alkoxyalkyl group;
X is a C₁∼C₄ alkyl group, a C₁∼C₄ haloalkyl group, a C₂∼C₄ alkoxyalkyl group, a halogen atom, a C₁∼C₄ alkoxy group or a C₁∼C₄ haloalkoxy group;
p is an integer of 0, 1 or 2;
R³ is a C₁∼C₄ alkyl group, a C₁∼C₄ haloalkyl group, a C₃∼C₆ cycloalkyl group, a C₃∼C₆ alkenylalkyl group, a C₃∼C₆ alkynylalkyl group, a C₃∼C₆ haloalkenylalkyl group or a C₂∼C₄ alkoxyalkyl group;
each of R⁴, R⁵, R⁶ and R⁷ is independently a hydrogen atom, a C₁∼C₄ alkyl group, a C₁∼C₄ haloalkyl group or a C₂∼C₄ alkoxyalkyl group;
n is an integer of 0, 1 or 2; and
Q is a hydrogen atom or a group of -A-B,
in which A is 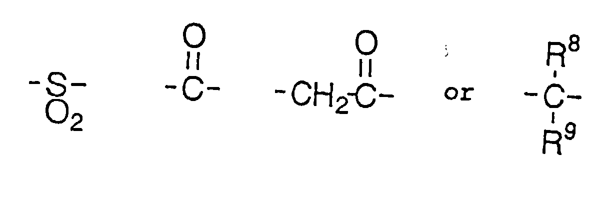
in which each of R⁸ and R⁹ is independently a hydrogen atom or a C₁∼C₄ alkyl group; and
B is a C₁∼C₁₂ alkyl group, a C₃-C₁₀ cycloalkyl group or a group of 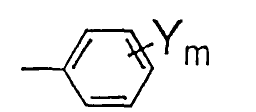
in which Y is a C₁∼C₄ alkyl group, a C₁∼C₄ alkoxy group, a C₁∼C₄ haloalkyl group, a nitro group or a halogen atom; and
m is an integer of 0, 1 or 2;
provided that compounds of the formula (I) wherein p is 1, R³ is a C₁∼C₄ alkyl group, X is a C₁∼C₄ alkyl group and all of R⁴, R⁵, R⁶ and R⁷ are hydrogen atoms and compounds of the formula (I) wherein p is 2, R³ is a C₁∼C₄ alkyl group, two Xs are both C₁∼C₄ alkyl groups and all of R⁴, R⁵, R⁶ and R⁷ are hydrogen atoms are excluded.

The second object of the present invention is achieved by a carboxylic acid of the formula (III) (to be sometimes referred to as "carboxylic acid (III)" hereinafter), 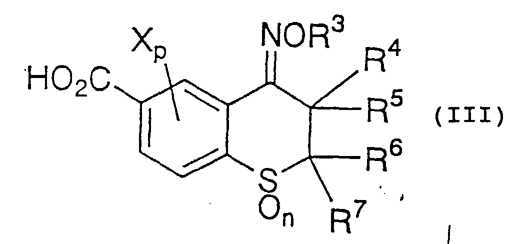 wherein:
X is a C₁∼C₄ alkyl group, a C₁∼C₄ haloalkyl group, a C₂∼C₄ alkoxyalkyl group, a halogen atom, a C₁∼C₄ alkoxy group or a C₁∼C₄ haloalkoxy group;
p is an integer of 0, 1 or 2;
R³ is a C₁∼C₄ alkyl group, a C₁∼C₄ haloalkyl group, a C₃∼C₆ cycloalkyl group, a C₃∼C₆ alkenylalkyl group, a C₃∼C₆ alkynylalkyl group, a C₃∼C₆ haloalkenylalkyl group or a C₂∼C₄ alkoxyalkyl group;
each of R⁴, R⁵, R⁶ and R⁷ is independently a hydrogen atom, a C₁∼C₄ alkyl group, a C₁∼C₄ haloalkyl group or a C₂∼C₄ alkoxyalkyl group; and
n is an integer of 0, 1 or 2;
provided that compounds of the formula (III) wherein p is 1, R³ is a C₁∼C₄ alkyl group, X is a C₁∼C₄ alkyl group and all of R⁴, R⁵, R⁶ and R⁷ are hydrogen atoms and compounds of the formula (III) wherein p is 2, R³ is a C₁∼C₄ alkyl group, two Xs are both C₁∼C₄ alkyl groups and all of R⁴, R⁵, R⁶ and R⁷ are hydrogen atoms are excluded.

The third object of the present invention is achieved by a herbicide containing the pyrazole derivative of the above formula (I) as an active ingredient (to be sometimes referred to as "herbicide of the present invention" hereinafter).

### Preferred Embodiments of the Invention

The novel pyrazole derivative of the present invention will be explained first.

The novel pyrazole derivative of the present invention is a compound of the formula (I).

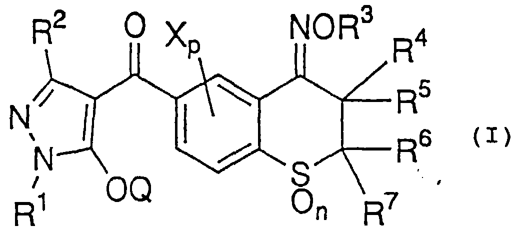

In the formula (I), R¹ is a C₁∼C₄ alkyl group, a C₂∼C₄ alkenyl group or a C₂∼C₄ haloalkenyl group. Specific examples of the C₁∼C₄ alkyl group include methyl, ethyl, propyl and butyl. Specific examples of the C₂∼C₄ alkenyl group include vinyl, allyl, 2-propenyl, 1-butylenyl and 2-butylenyl. The C₂∼C₄ haloalkenyl group is a group formed by replacing at least one hydrogen atom of the C₂∼C₄ alkenyl group with a halogen atom (e.g., chlorine, bromine, fluorine or iodine atom). Specific examples of the C₂∼C₄ haloalkenyl group include -CH=CHF, - CH=CHCl, -CH=CHBr, -CF=CHF, -CCl=CHCl, -CF=CF₂, -CH=CH-CH=CHF.

R¹ is preferably a C₁∼C₄ alkyl group, particularly preferably methyl or ethyl.

In the formula (I), R² is a hydrogen atom, a C₁∼C₄ alkyl group, a C₁∼C₄ haloalkyl group or a C₂∼C₄ alkoxyalkyl group. Specific examples of the C₁∼C₄ alkyl group include those explained concerning R¹. The C₁∼C₄ haloalkyl group is a group formed by replacing at least one hydrogen atom of the C₁∼C₄ alkyl group explained concerning R¹ with a halogen atom (e.g., chlorine, bromine, fluorine or iodine atom). Specific examples of the C₁∼C₄ haloalkyl group include -CH₂F, -CHF₂, -CF₃, -CH₂CF₃, -C₂F₅, -CH₂Cl, -CCl₃, -CHCl-CH₃, -CH₂CH₂Cl, -CHClCH₂Cl, -CH₂Br, -CHBrCH₃, -CH₂CH₂Br and CH₂I. Specific examples of the C₂∼C₄ alkoxyalkyl group include methoxymethyl, methoxyethyl, ethoxymethyl, n-propoxymethyl and i-propoxymethyl.

R² is preferably a hydrogen atom or a C₁∼C₄ alkyl group, particularly preferably a hydrogen atom or methyl.

In the formula (I), X is a C₁∼C₄ alkyl group, a C₁∼C₄ haloalkyl group, a C₂∼C₄ alkoxyalkyl group, a halogen atom, a C₁∼C₄ alkoxy group or a C₁∼C₄ haloalkoxy group. Specific examples of the C₁∼C₄ alkyl group, the C₁∼C₄ haloalkyl group and the C₂∼C₄ alkoxyalkyl group include those explained concerning R¹ or R². The C₁∼C₄ haloalkyl group is preferably trifluoromethyl. Specific examples of the halogen atom include chlorine, bromine, fluorine and iodine atoms. Specific examples of the C₁∼C₄ alkoxy group include methoxy, ethoxy, n-propoxy, i-propoxy, n-butoxy and i-butoxy. The C₁∼C₄ alkoxy group is preferably methoxy. The C₁∼C₄ haloalkoxy group is a group formed by replacing at least one hydrogen atom of the above C₁∼C₄ alkoxy group with a halogen atom (e.g., chlorine, bromine, fluorine or iodine atom). Specific examples of the C₁∼C₄ haloalkoxy group include -OCH₂F, -OCHF₂, -OCF₃, -OCH₂CF₃, -OC₂F₅, -OCH₂Cl, -OCCl₃, -OCHCl-CH₃, -OCH₂CH₂Cl, -OCHClCH₂Cl, -OCH₂Br, -OCHBrCH₃, -OCH₂CH₂Br and -OCH₂I.

X is preferably a C₁∼C₄ alkyl group or a halogen atom, particularly preferably methyl, chlorine or bromine.

The position on which X is substituted can be any one of the 5-, 7- and 8-positions on the thiochroman ring, while the position(s) for substituent(s) X is/are the 5- and/or 8-position(s).

In the formula (I), p is the number of substituent(s) X, and p is an integer of 0, 1 or 2, preferably 1 or 2.

When p = 1, i.e., when one X is positioned on the thiochroman ring, the position where X is substituted is preferably the 5-position on the thiochroman ring. When p = 1, preferably, X is a C₁∼C₄ alkyl group, a C₁∼C₄ haloalkyl group (preferably trifluoromethyl), a halogen atom (preferably chlorine atom) or a C₁∼C₄ alkoxy group (preferably methoxy). X is particularly preferably methyl or a chlorine atom.

When p = 2, i.e., when two Xs are positioned on the thiochroman ring, the positions where Xs are substituted are preferably the 5- and 8-positions on the thiochroman ring. When p = 2, the combination of the two Xs is preferably as follows. one X on the 5-position on the thiochroman ring is a C₁∼C₄ alkyl group (preferably methyl), a halogen atom (preferably chlorine or fluorine atom) or a C₁∼C₄ haloalkyl group (preferably trifluoromethyl) positioned, and the other X on the 8-position on the thiochroman ring is a halogen atom (preferably chlorine or fluorine atom), a C₁∼C₄ alkoxy group (preferably methoxy) or a C₁∼C₄ alkyl group (preferably methyl).

When p = 2, the combination of the two Xs is more preferably as follows. One X on the 5-position on the thiochroman ring is a C₁∼C₄ alkyl group (preferably methyl), and the other X on the 8-position on the thiochroman ring is a halogen atom (preferably chlorine or fluorine atom) or a C₁∼C₄ alkoxy group (preferably methoxy) positioned. Otherwise, one X on the 5-position on the thiochroman ring is a halogen atom (preferably chlorine atom), a C₁∼C₄ haloalkyl group (trifluoromethyl) or a C₁∼C₄ alkoxy group (preferably methoxy), and the other X on the 8-position on the thiochroman ring is a halogen atom (preferably chlorine or fluorine atom), a C₁∼C₄ alkoxy group (preferably methoxy) or a C₁∼C₄ alkyl group (preferably methyl).

When p = 2, the combination of the two Xs is particularly preferably as follows. One X on the 5-position on the thiochroman ring is a C₁∼C₄ alkyl group (preferably methyl) or a halogen atom (preferably chlorine or fluorine atom), and the other X on the 8-position on the thiochroman ring is halogen atom (preferably chlorine or fluorine atom).

In the formula (I), R³ is a C₁∼C₄ alkyl group, a C₁∼C₄ haloalkyl group, a C₃∼C₆ cycloalkyl group, a C₃∼C₆ alkenylalkyl group, a C₃∼C₆ alkynylalkyl group, a C₃∼C₆ haloalkenylalkyl group or a C₂∼C₄ alkoxyalkyl group. Specific examples of the C₁∼C₄ alkyl group, the C₁∼C₄ haloalkyl group and the C₂∼C₄ alkoxyalkyl group include those explained concerning R¹ or R².

Specific examples of the C₃∼C₆ cycloalkyl group include cyclopropyl, cyclobutyl, cyclopentyl and cyclohexyl. Specific examples of the C₃∼C₆ alkenylalkyl group include allyl, vinylethyl, -CH₂CH=C(CH₃)₂, -CH₂C(CH₃)=CH₂, -CH₂CH=CH-CH₃ and -CH₂CH=CH-C₂H₅. Specific examples of the C₃∼C₆ alkynylalkyl group include propargyl, 3-butynyl,

-CH₂-C→C-CH₃ and -CH(CH₃)-C→CH.

The C₃∼C₆ haloalkenylalkyl group is a group formed by replacing at least one hydrogen atom of the above C₃∼C₆ alkenylalkyl group with a halogen atom (e.g., chlorine, bromine, fluorine or iodine atom). Specific examples of the C₃∼C₆ haloalkenylalkyl group include -CH₂-CH=CHCl, -CH₂-CH=CHF, - CH₂-CCl=CH₂ and -CH₂-CF=CH₂.

R³ is preferably a C₁∼C₄ alkyl group (particularly preferably methyl), a C₁∼C₄ haloalkyl group (particularly preferably -CH₂CH₂F), a C₃∼C₆ alkenylalkyl group (particularly preferably allyl) or a C₃∼C₆ alkynylalkyl group (particularly preferably propargyl).

In the formula (I), each of R⁴, R⁵, R⁶ and R⁷ is independently a hydrogen atom, a C₁∼C₄ alkyl group, a C₁∼C₄ haloalkyl group or a C₂∼C₄ alkoxyalkyl group. Specific examples of the C₁∼C₄ alkyl group, the C₁∼C₄ haloalkyl group and the C₂∼C₄ alkoxyalkyl group include those explained concerning R¹ or R².

Each of R⁴, R⁵, R⁶ and R⁷ is, preferably, independently a hydrogen atom or a C₁∼C₄ alkyl group, and particularly preferably, a hydrogen atom.

In the formula (I), n is the number of oxygen atom bonding to the sulfur atom of the thiochroman ring, and n is an integer of 0, 1 or 2. That is, when n is 0, a sulfide is represented. When n is 1, a sulfoxide is represented. When n is 2, a sulfone is represented. n is preferably 0 or 2, particularly preferably 2, i.e., a sulfone is represented.

In the formula (I), Q is a hydrogen atom or a group of -A-B. In the group of -A-B, A is 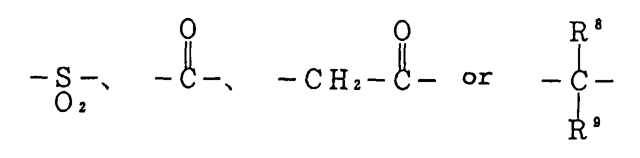 in which each of R⁸ and R⁹ is independently a hydrogen atom or a C₁∼C₄ alkyl group. Specific examples of the C₁∼C₄ alkyl group include those explained concerning R¹. Each of R⁸ and R⁹ is preferably methyl.

A is preferably

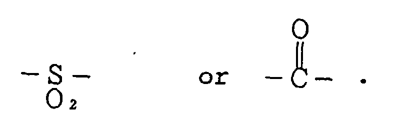

In the group of -A-B, B is a C₁∼C₁₂ alkyl group, a C₃∼C₁₀ cycloalkyl group or a group of 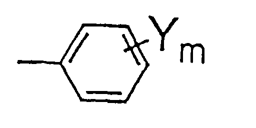 in which Y is a C₁∼C₄ alkyl group, a C₁∼C₄ alkoxy group, a C₁∼C₄ haloalkyl group, a nitro group or a halogen atom. m is the number of substituent(s) Y, and m is an integer of 0, 1 or 2. Specific examples of the C₁∼C₄ alkyl group, the C₁∼C₄ alkoxy group, the C₁∼C₄ haloalkyl group and the halogen atom include those explained concerning R¹, R² or X.

In B, an alkyl group having at least 3 carbon atoms out of the C₁∼C₁₂ alkyl group may be branched. Specific examples of the C₁∼C₁₂ alkyl group include pentyl, hexyl, heptyl, octyl, nonyl, decyl, undecyl and dodecyl in addition to the specific examples of the C₁∼C₄ alkyl group explained concerning R¹.

In B, specific examples of the C₃∼C₁₀ cycloalkyl group include cyclopropyl, cyclobutyl, cyclopentyl, cyclohexyl, cycloheptyl, cyclooctyl, cyclononyl and cyclodecyl.

B is preferably a C₁∼C₄ alkyl group, a halogen atom, a C₃∼C₇ cycloalkyl group or a group of 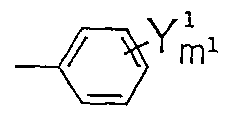 in which Y¹ is a C₁∼C₄ alkyl group or a halogen atom, m¹ is an integer of 0, 1 or 2. B is particularly preferably methyl, chlorine, cyclohexyl or toluyl.

Q is preferably a hydrogen atom or a group of -A-B in which a combination of A and B has any one of the following structures.

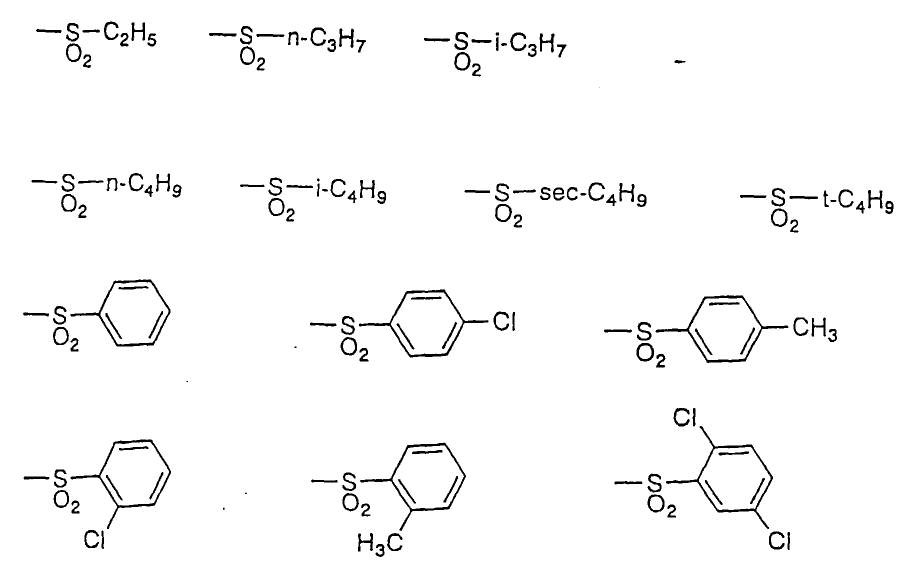

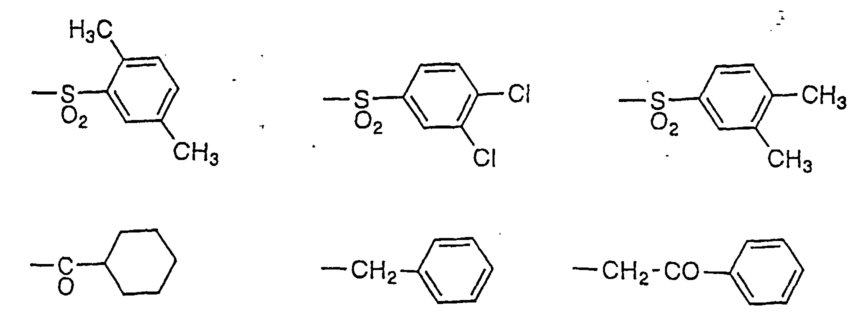

In the group of -A-B for Q, the combination of A and B is particularly preferably

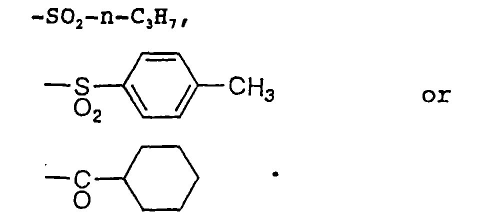

In addition, compounds of the formula (I) wherein p is 1, R³ is a C₁∼C₄ alkyl group, X is a C₁∼C₄ alkyl group and all of R⁴, R⁵, R⁶ and R⁷ are hydrogen atoms, and compounds of the formula (I) wherein p is 2, R³ is a C₁∼C₄ alkyl group, two Xs are both C₁∼C₄ alkyl groups and all of R⁴, R⁵, R⁶ and R⁷ are hydrogen atoms are excluded.

The combination of the substituents on the pyrazole derivative of the formula (I) preferably includes those shown in the following Table 1. In pyrazole derivatives (I) shown in Table 1, each of R⁴, R⁵, R⁶ and R⁷ is a hydrogen atom and n is 2.

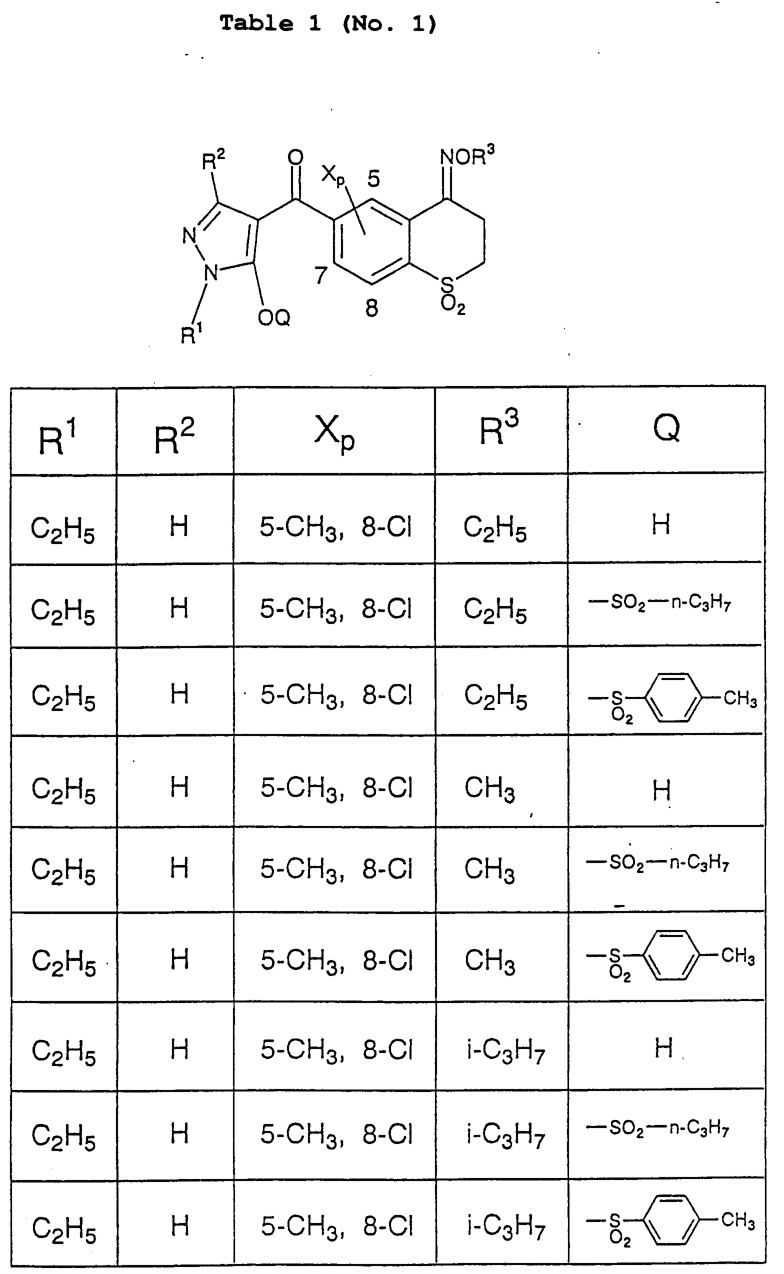

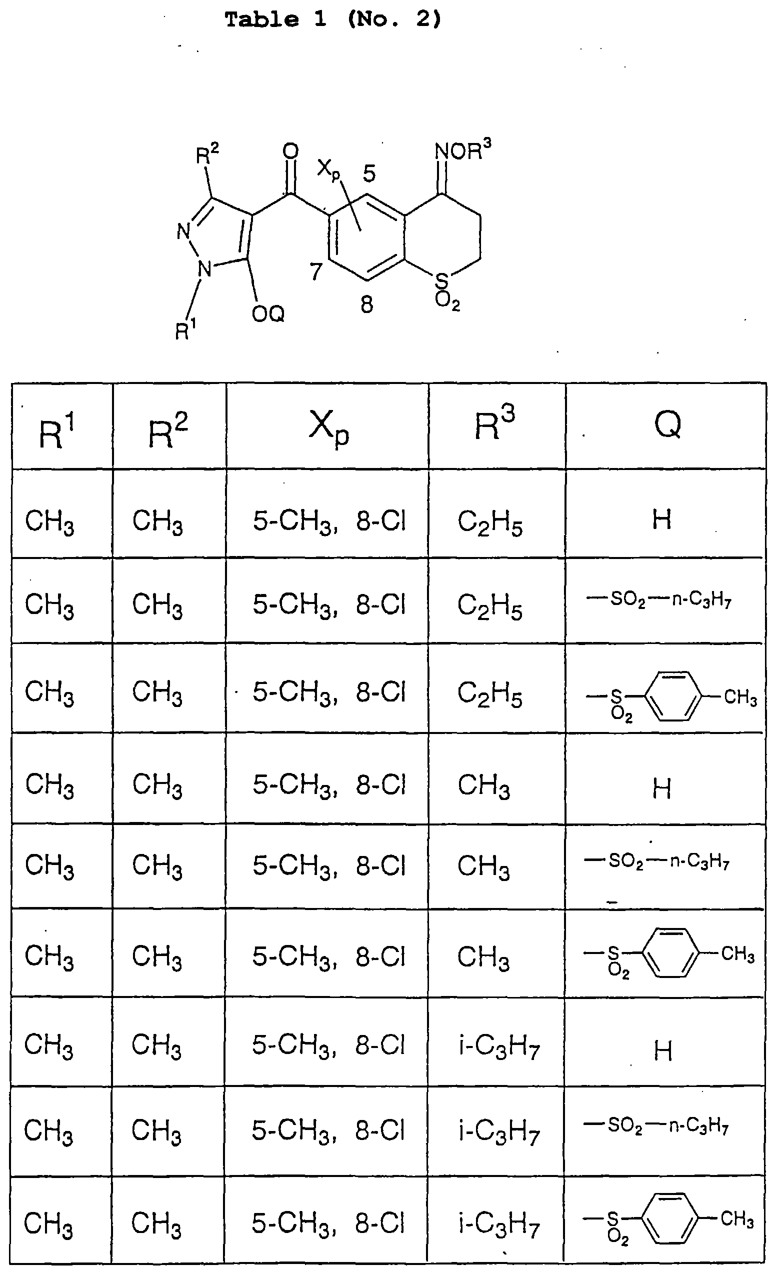

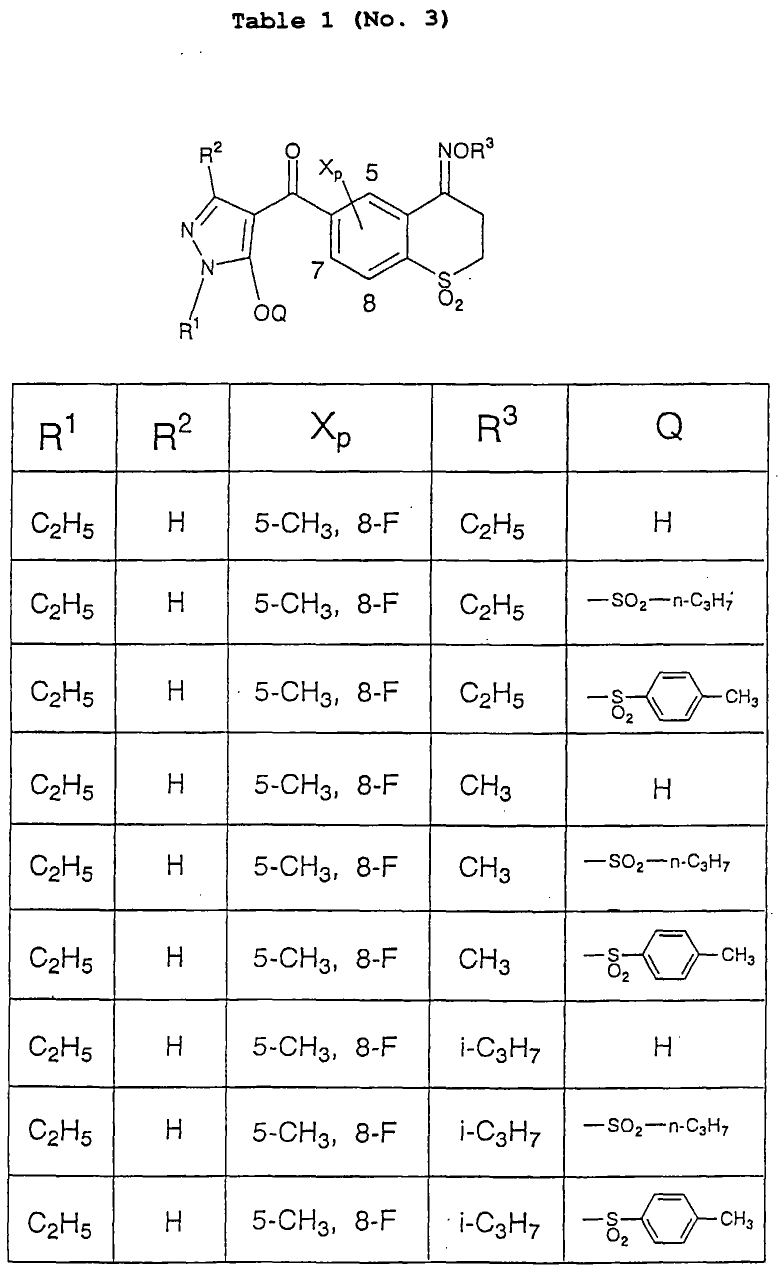

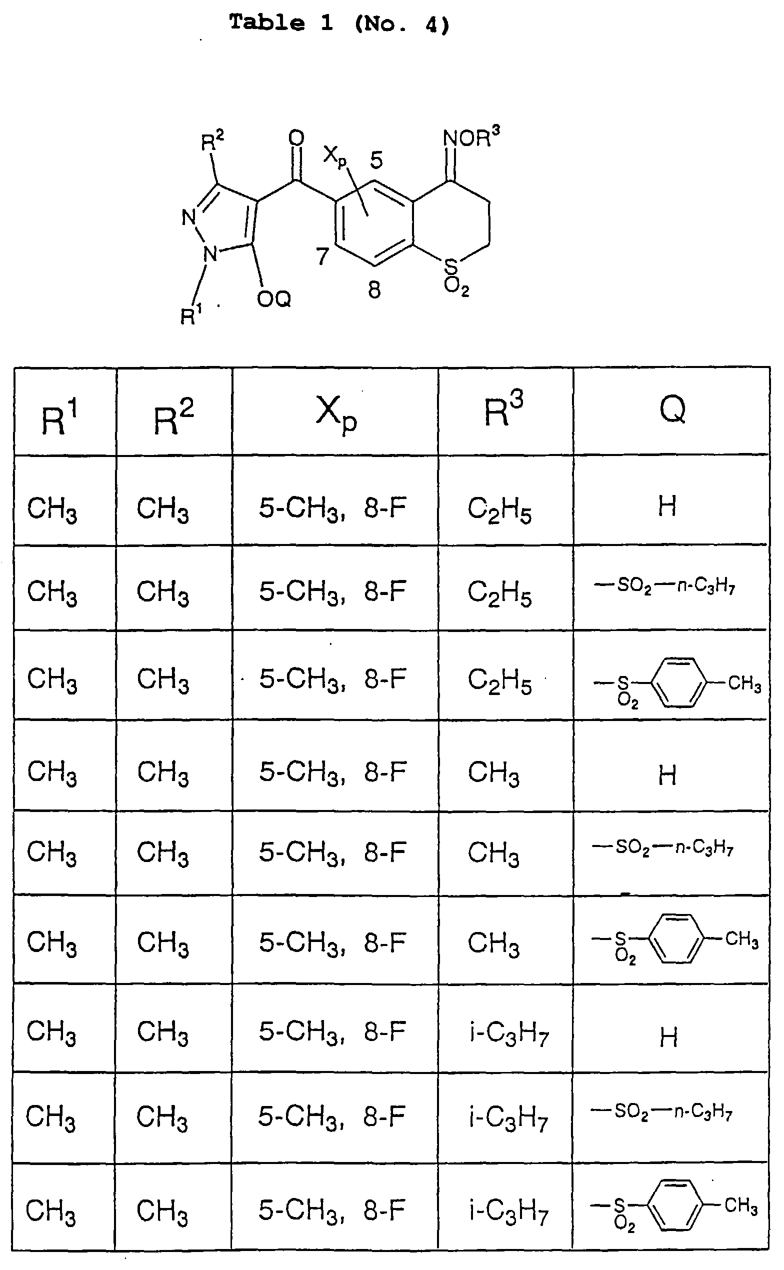

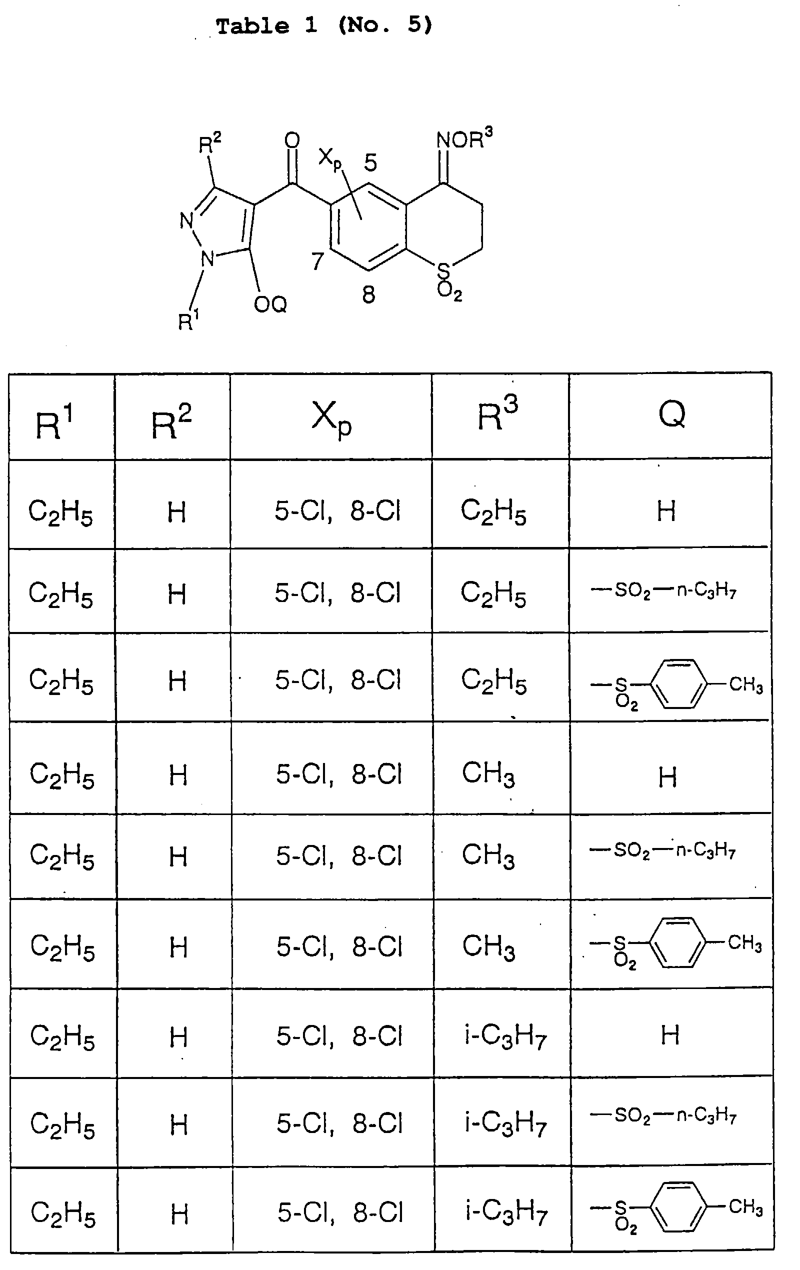

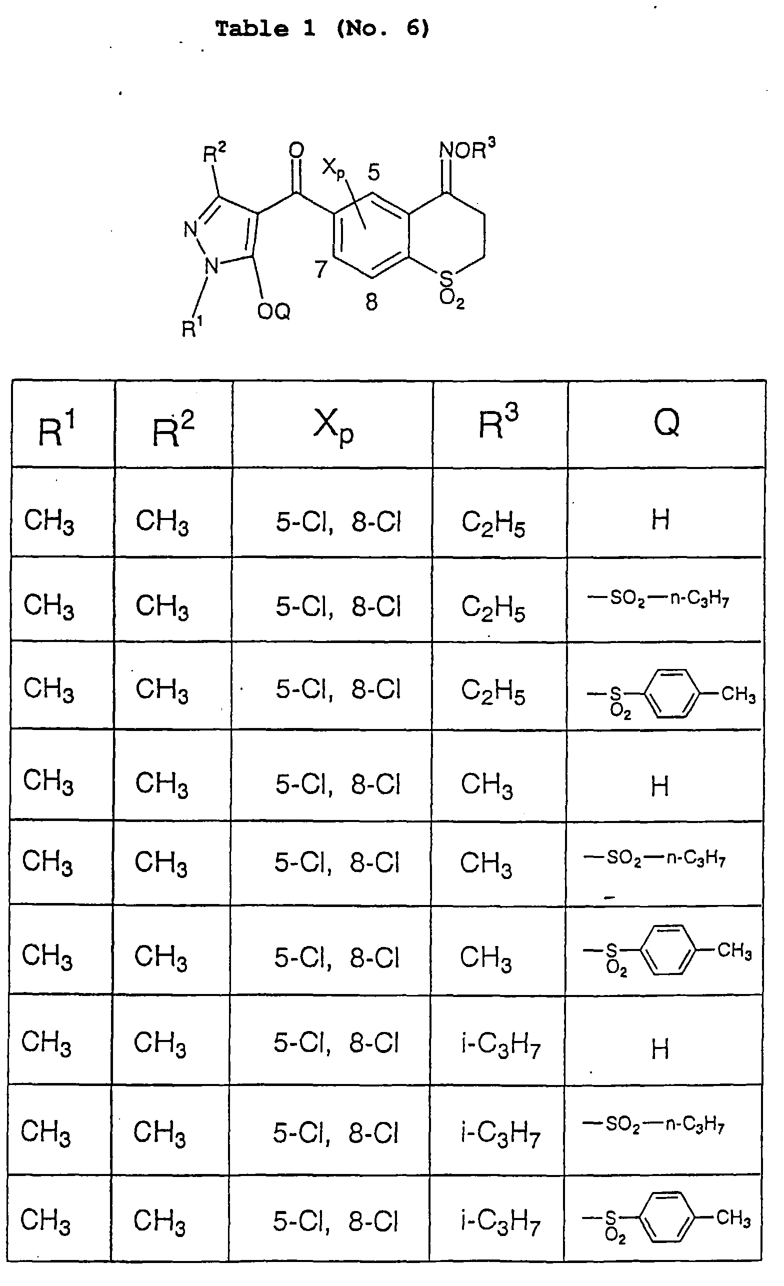

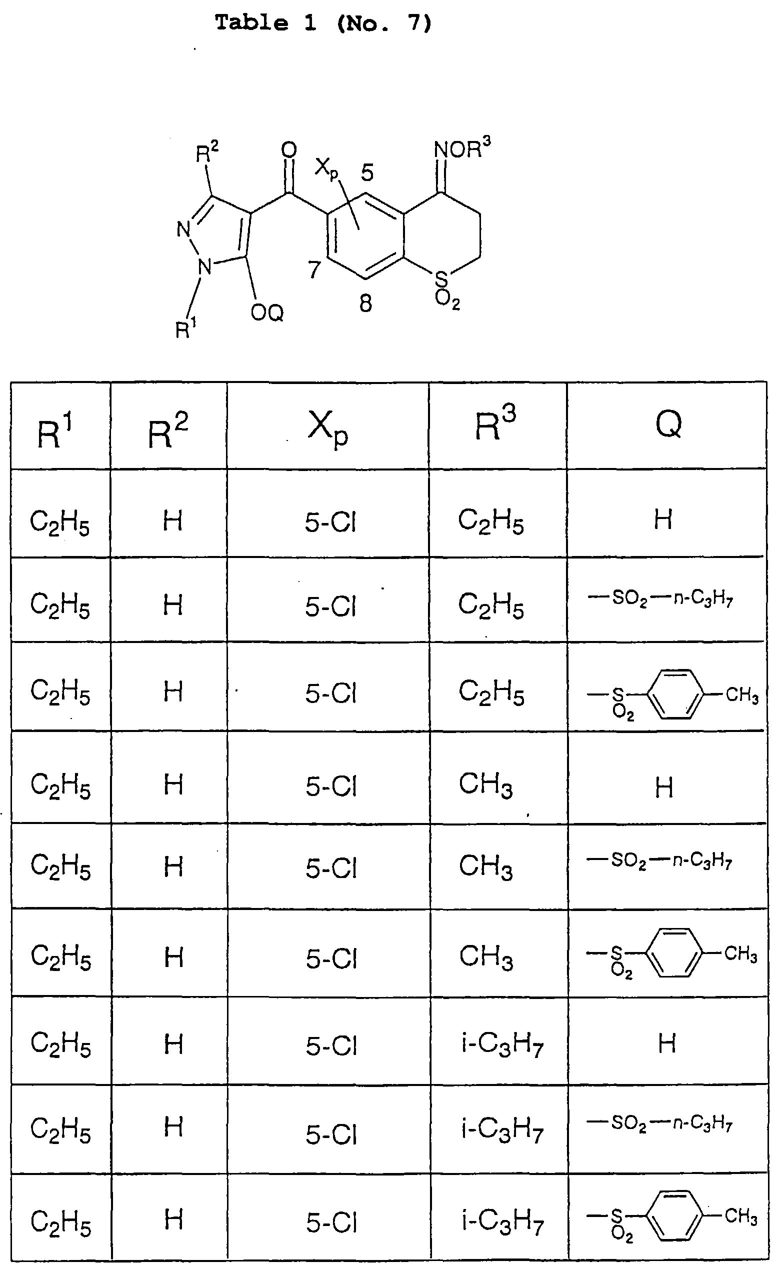

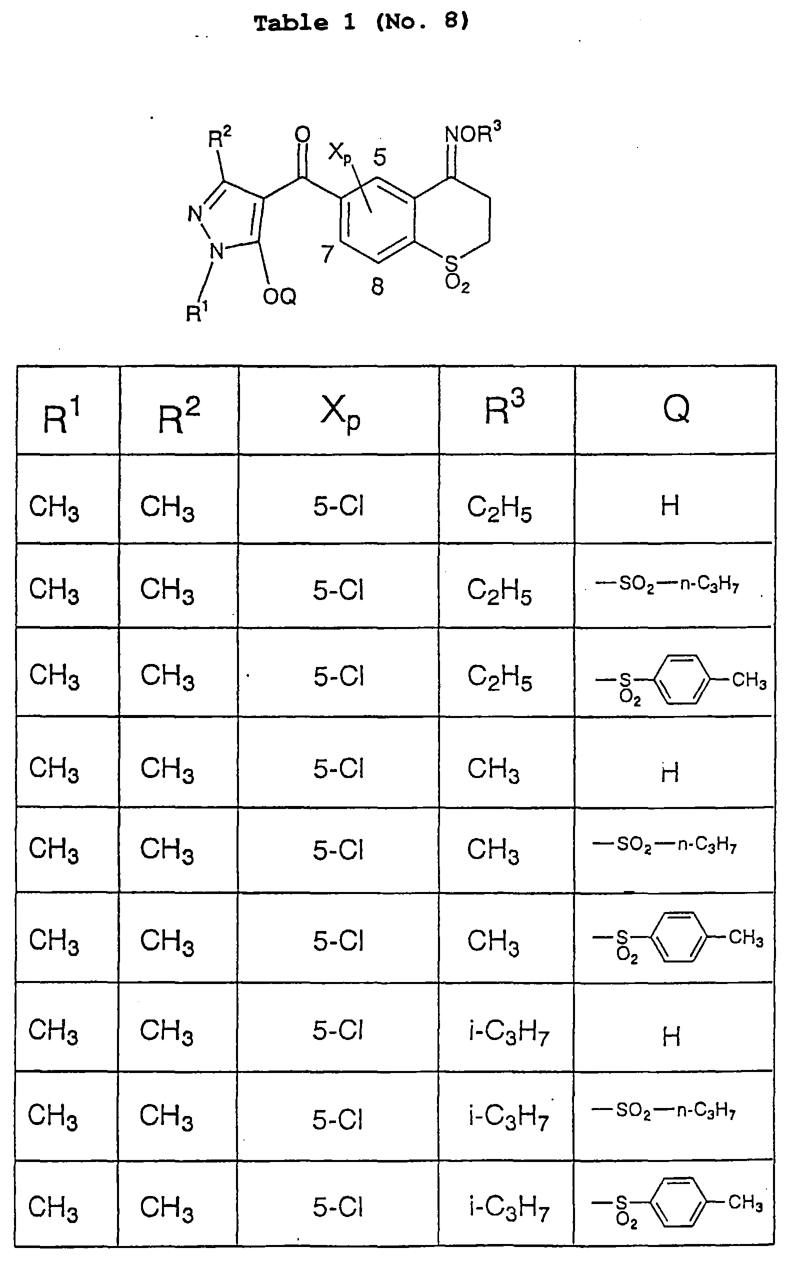

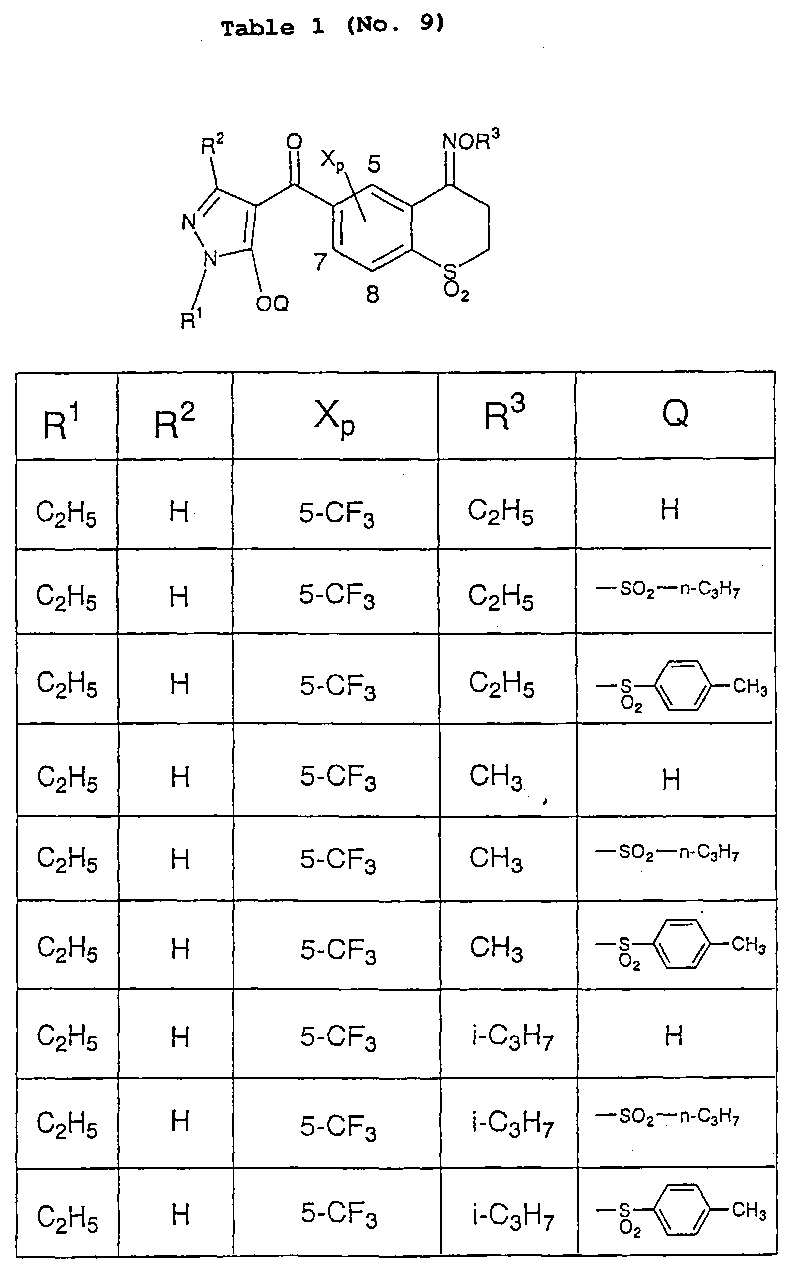

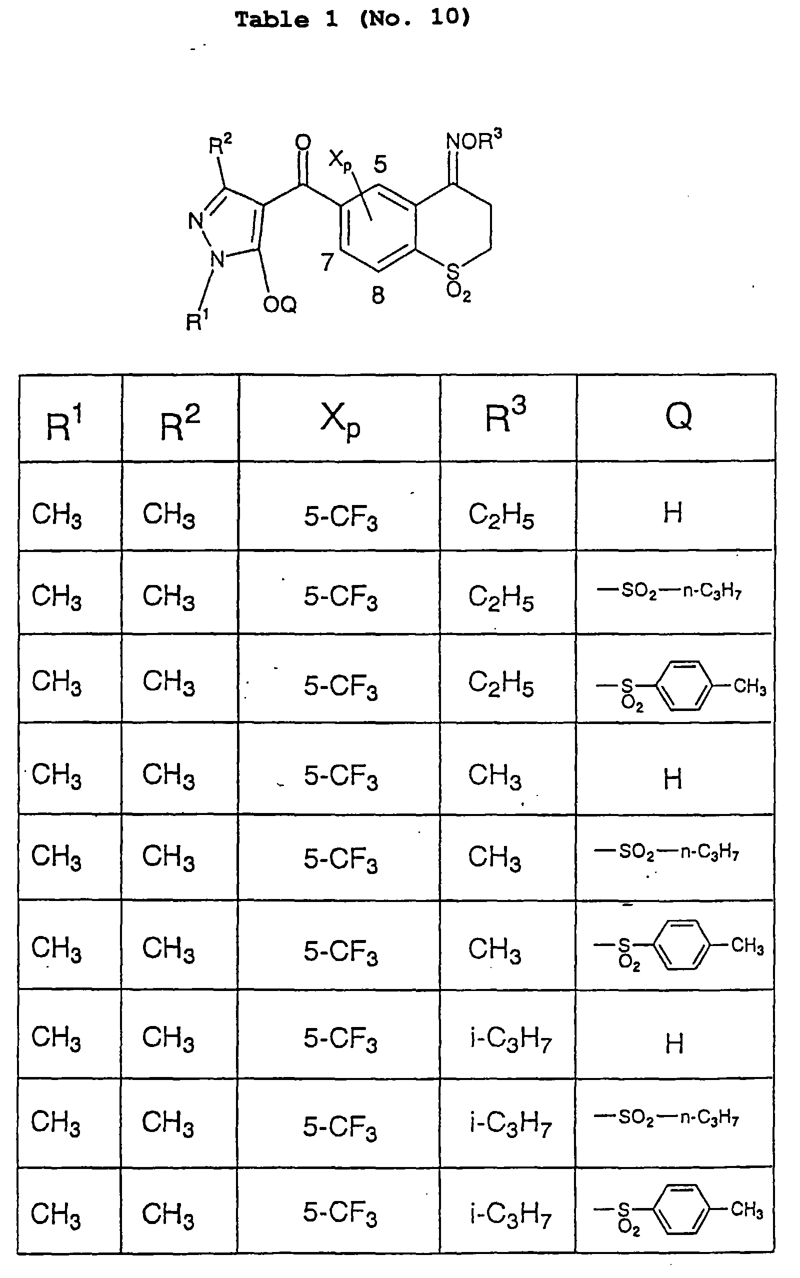

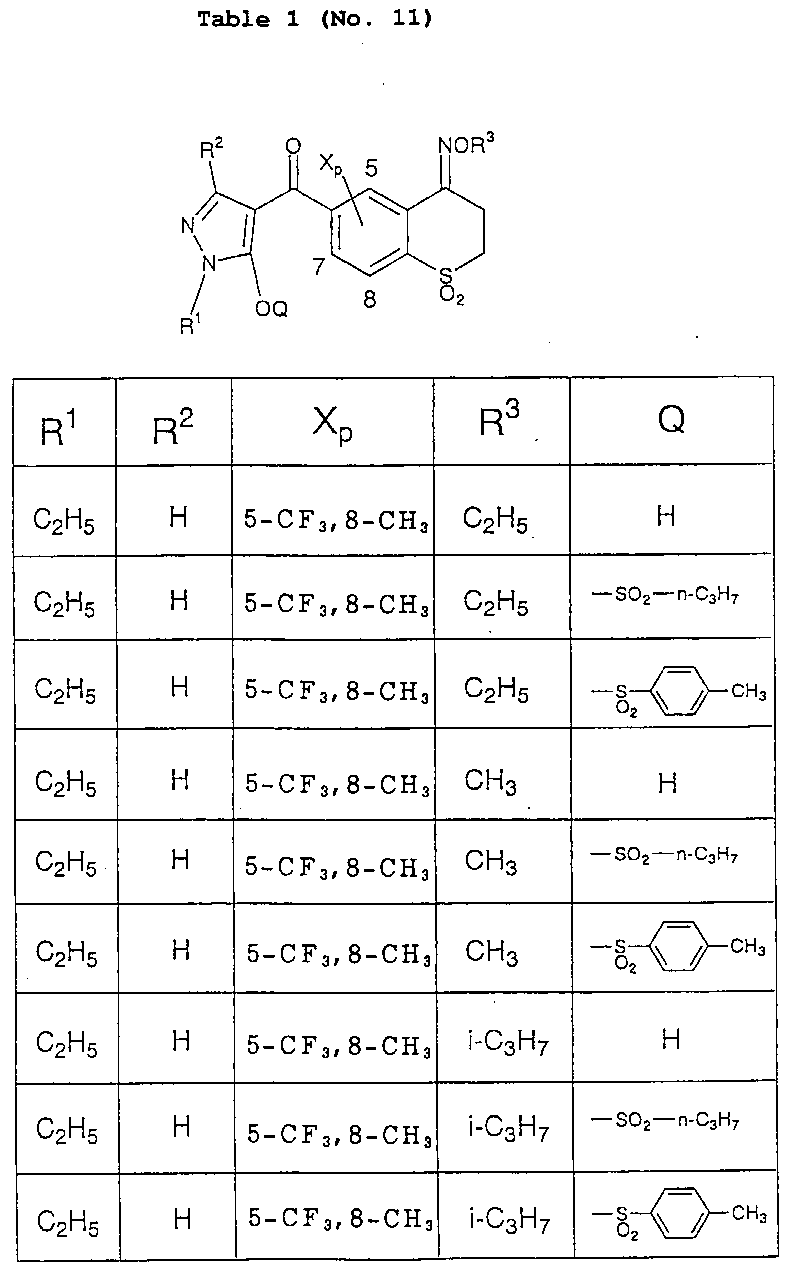

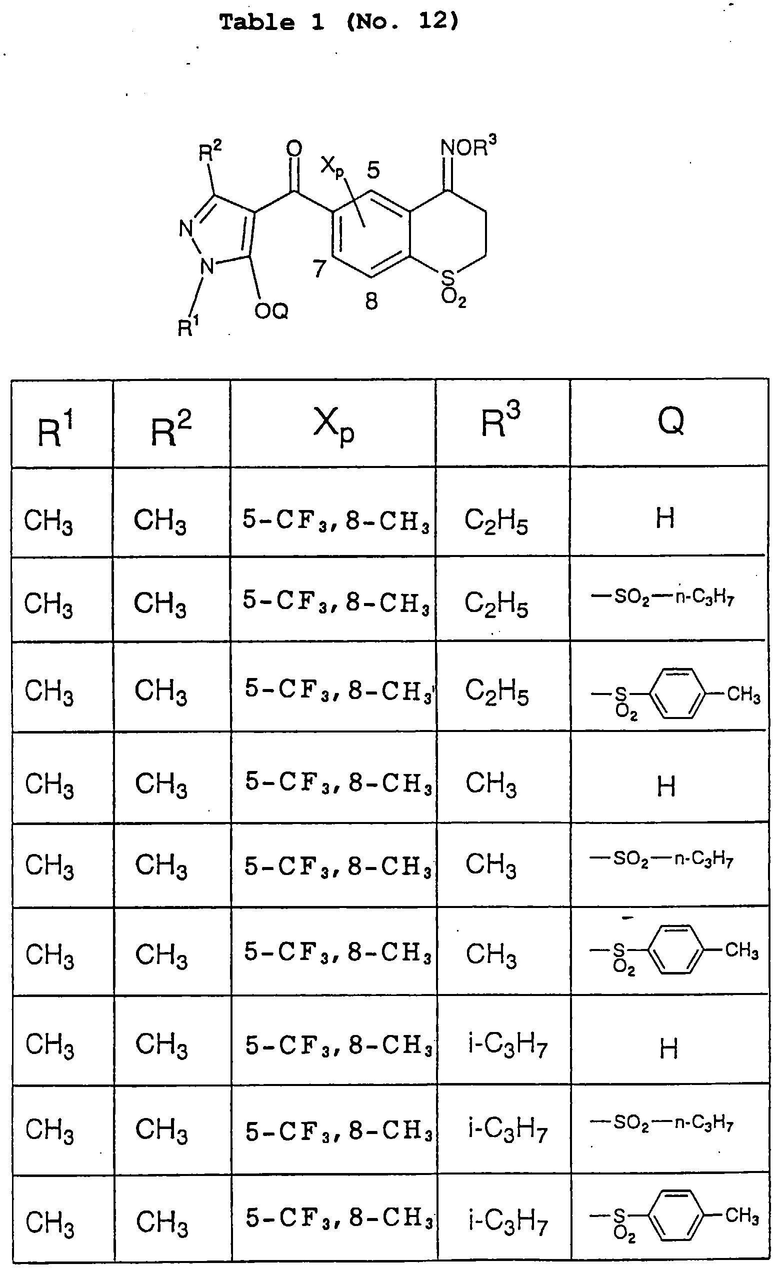

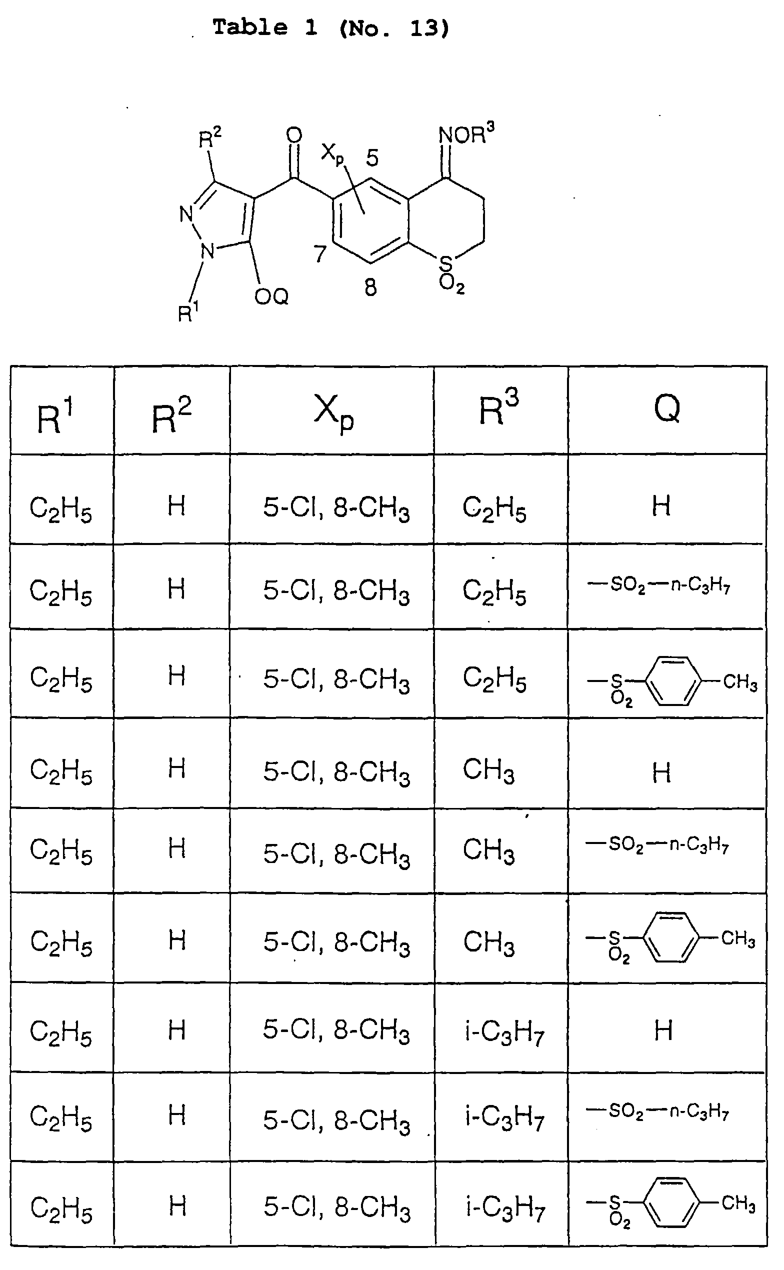

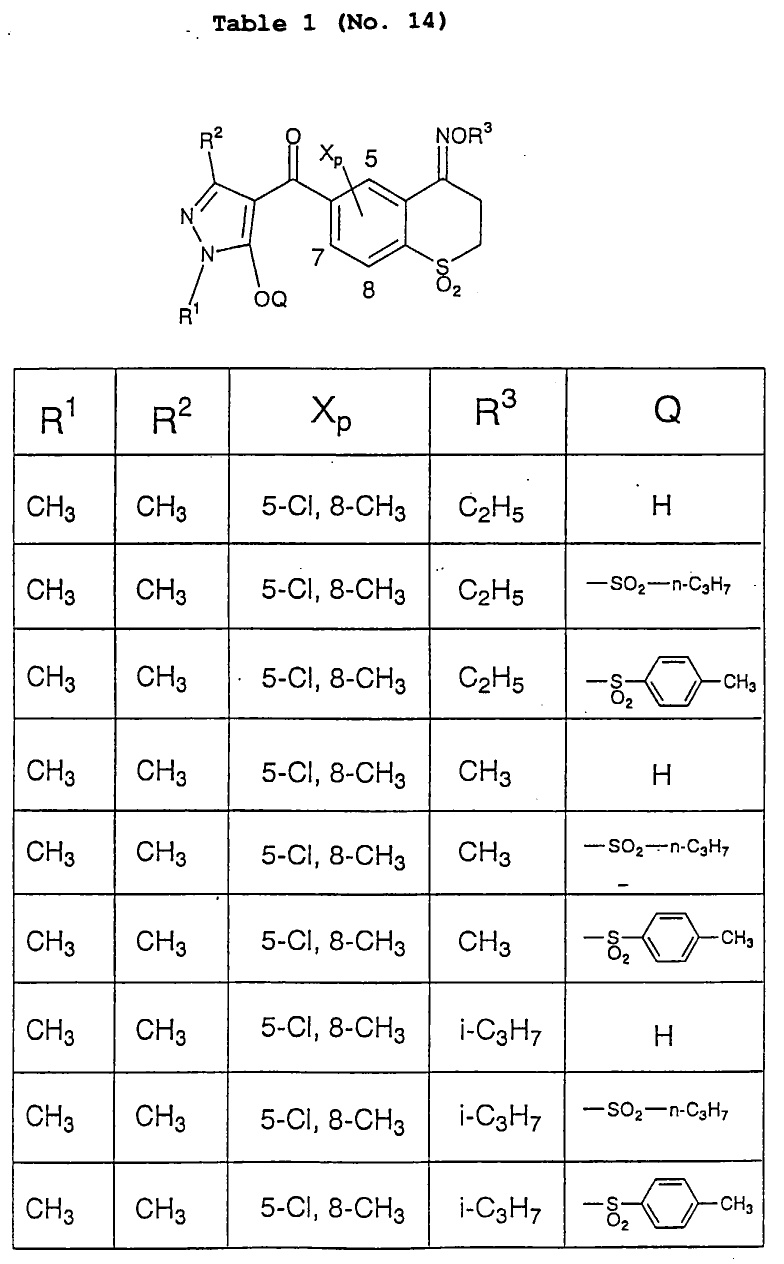

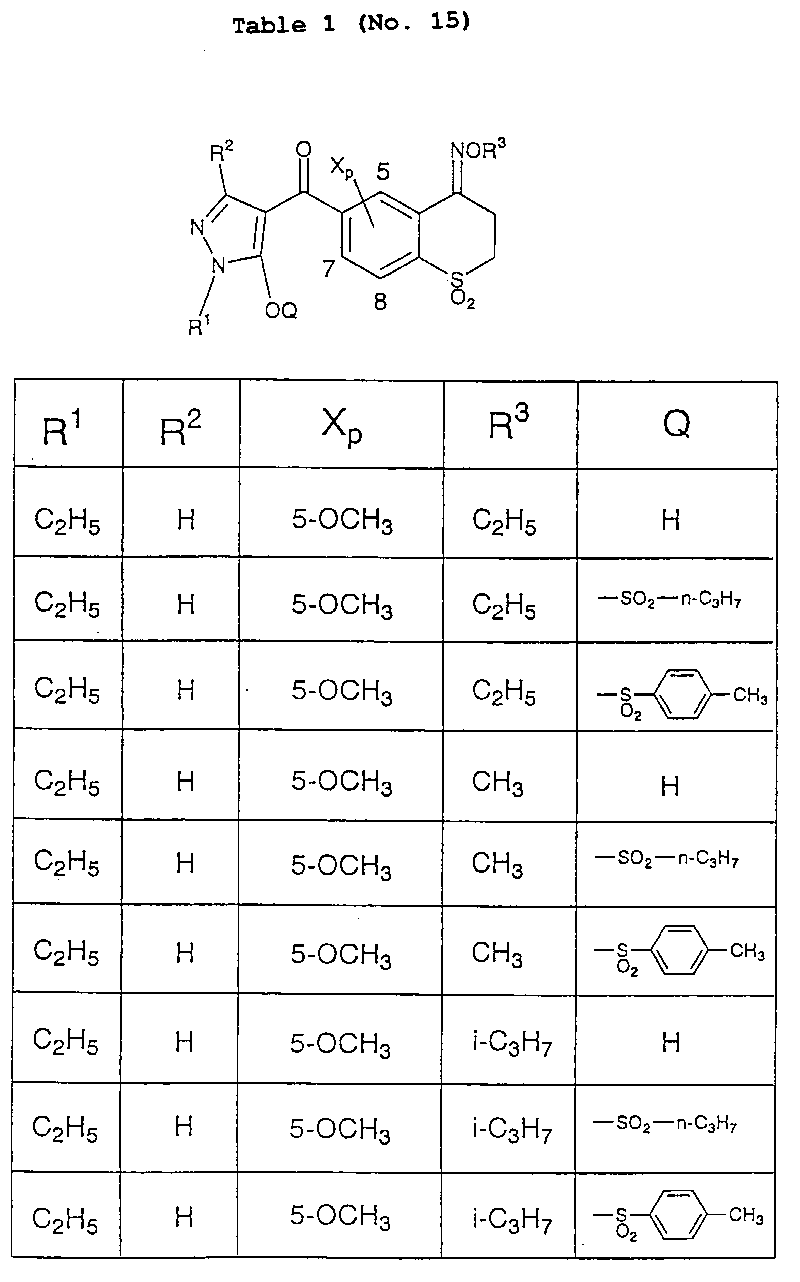

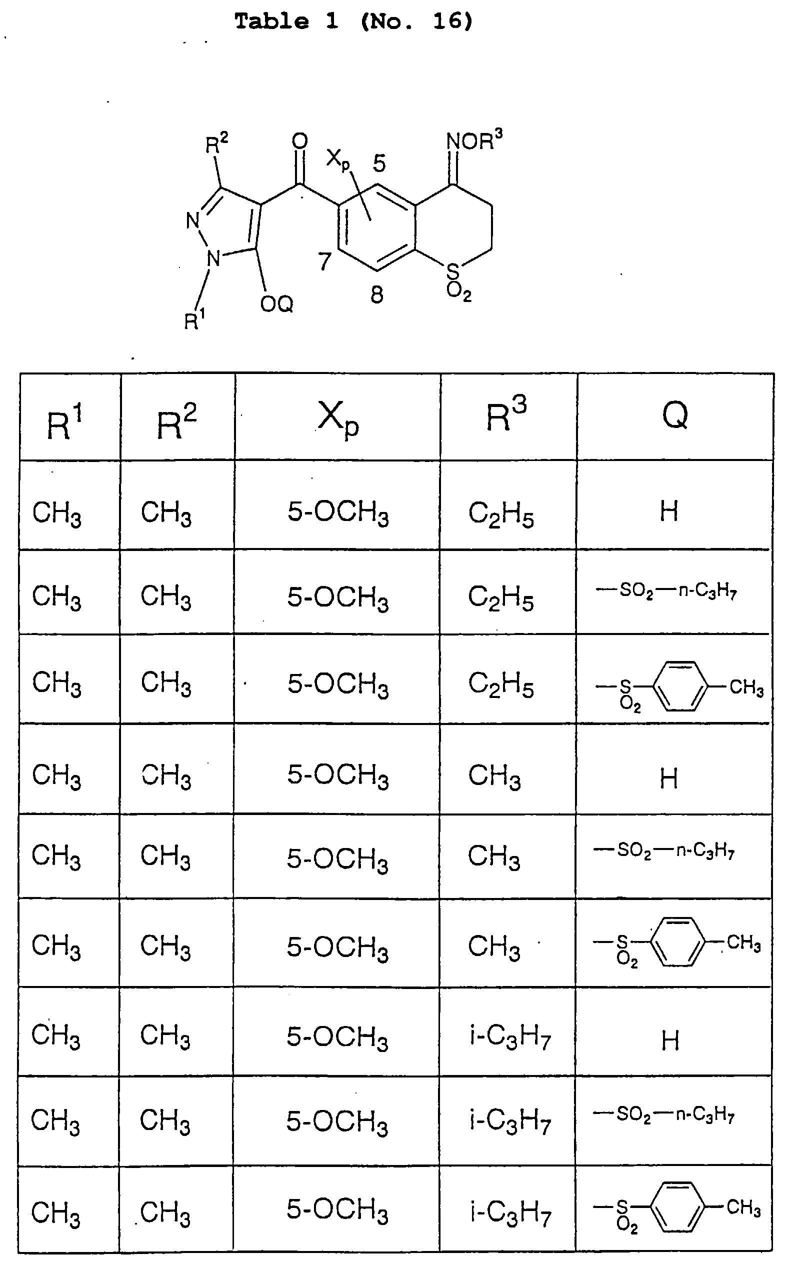

The pyrazole derivative of the formula (I) has geometric isomerism based on alkoxyimino group, shown by the following formulae (Ia) and (Ib), while the pyrazole derivative of the present invention includes all the isomers and mixtures of these.

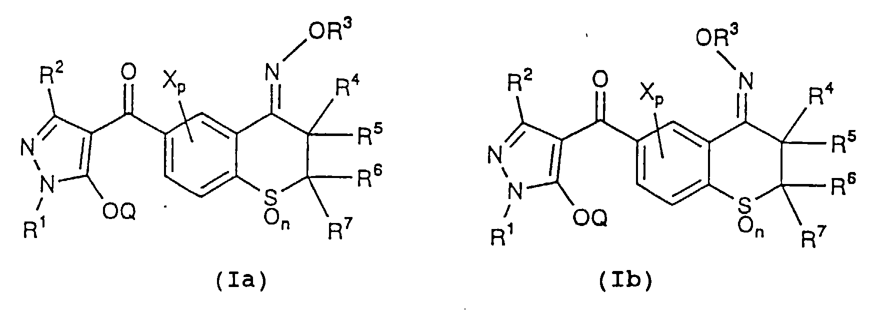

The pyrazole derivative of the formula (I) in which Q is a hydrogen atom, i.e., a compound of the formula (Ic), can have the following four structures due to tautomerism, and the pyrazole derivative of the present invention includes all of these compounds and mixtures of these.

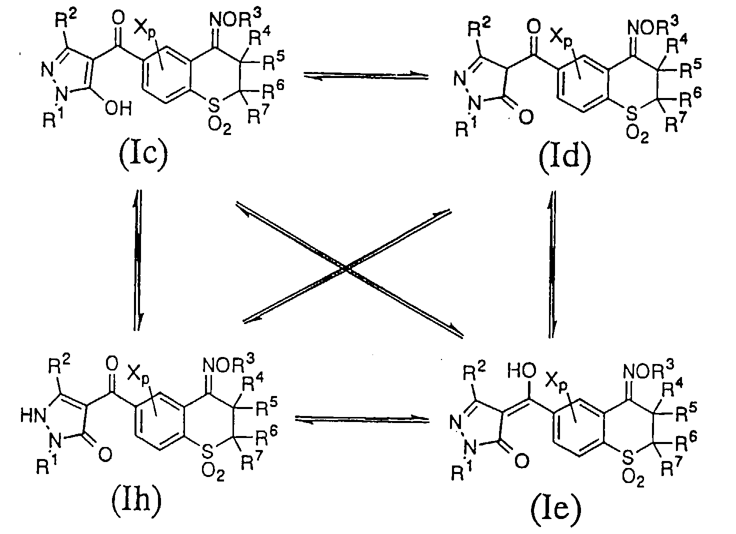

Further, some pyrazole derivatives of the formula (I) have asymmetric carbon, and various isomers are therefore present. The pyrazole derivative of the present invention includes all of these isomers and mixtures of these.

Further, the pyrazole derivative of the formula (Ic) is an acidic substance and can be easily converted to a salt by treating it with a base. This salt is also included in the pyrazole derivative of the present invention.

The above base can be selected from known bases without any limitation, and examples of the base include organic bases such as amines and anilines and inorganic bases such as sodium compounds and potassium compounds. Examples of the amines include monoalkylamine, dialkylamine and trialkylamine. Alkyl groups of the alkylamines are generally C₁∼C₄ alkyl groups . Examples of the anilines include aniline, monoalkylaniline and dialkylaniline. Alkyl groups of the alkylanilines are generally C₁∼C₄ alkyl groups. Examples of the sodium compounds include sodium hydroxide and sodium carbonate. Examples of the potassium compounds include potassium hydroxide and potassium carbonate.

The herbicide of the present invention contains the novel pyrazole derivative of the formula (I) and/or the salt thereof, provided by the present invention, as active ingredient. These compounds are used by mixing them with a liquid carrier such as a solvent or a solid carrier such as a mineral fine powder and preparing the resultant mixtures in the form of a wettable powder, an emulsifiable concentrate, a dust or granules. These compounds can be imparted with emulsifiability, dispersibility or spreadability by adding a surfactant when the above preparations are formed.

When the herbicide of the present invention is used in the form of a wettable powder, generally, 10 to 55% by weight of the pyrazole derivative and/or the salt thereof, provided by the present invention, 40 to 88 % by weight of a solid carrier and 2 to 5 % by weight of a surfactant are mixed to prepare a composition, and the composition can be used. When the herbicide of the present invention is used in the form of an emulsifiable concentrate, generally, the emulsifiable concentrate can be prepared by mixing 20 to 50 % by weight of the pyrazole derivative and/or the salt thereof, provided by the present invention, 35 to 75 % by weight of a solvent and 5 to 15 % by weight of a surfactant.

When the herbicide of the present invention is used in the form of a dust, generally, the dust can be prepared by mixing 1 to 15 % by weight of the pyrazole derivative and/or the salt thereof, provided by the present invention, 80 to 97 % by weight of a solid carrier and 2 to 5 % by weight of a surfactant. Further, when the herbicide of the present invention is used in the form of granules, the granules can be prepared by mixing 1 to 15 % by weight of the pyrazole derivative or the salt thereof, provided by the present invention, 80 to 97 % by weight of a sold carrier and 2 to 5 % by weight of a surfactant. The above solid carrier is selected from mineral powders. Examples of the mineral powders include oxides such as diatomaceous earth and slaked lime, phosphates such as apatite, sulfates such as gypsum and silicates such as talc, pyrophyllite, clay, kaolin, bentonite, acidic terra abla, white carbon, powdered quartz and powdered silica.

The solvent is selected from organic solvents. Specific examples of the solvent include aromatic hydrocarbons such as benzene, toluene and xylene, chlorinated hydrocarbons such as o-chlorotoluene, trichloroethane and trichloroethylene, alcohols such as cyclohexanol, amyl alcohol and ethylene glycol, ketones such as isophorone, cyclohexanone and cyclohexenyl-cyclohexanone, ethers such as butyl cellosolve, diethyl ether and methyl ethyl ether, esters such as isopropyl acetate, benzyl acetate and methyl phthalate, amides such as dimethylformamide, and mixtures of these.

The surfactant is selected from anionic surfactants, nonionic surfactants, cationic surfactants and amphoteric surfactants (amino acid and betaine).

The novel pyrazole derivative of the formula (I) provided by the present invention can be produced by the following method.

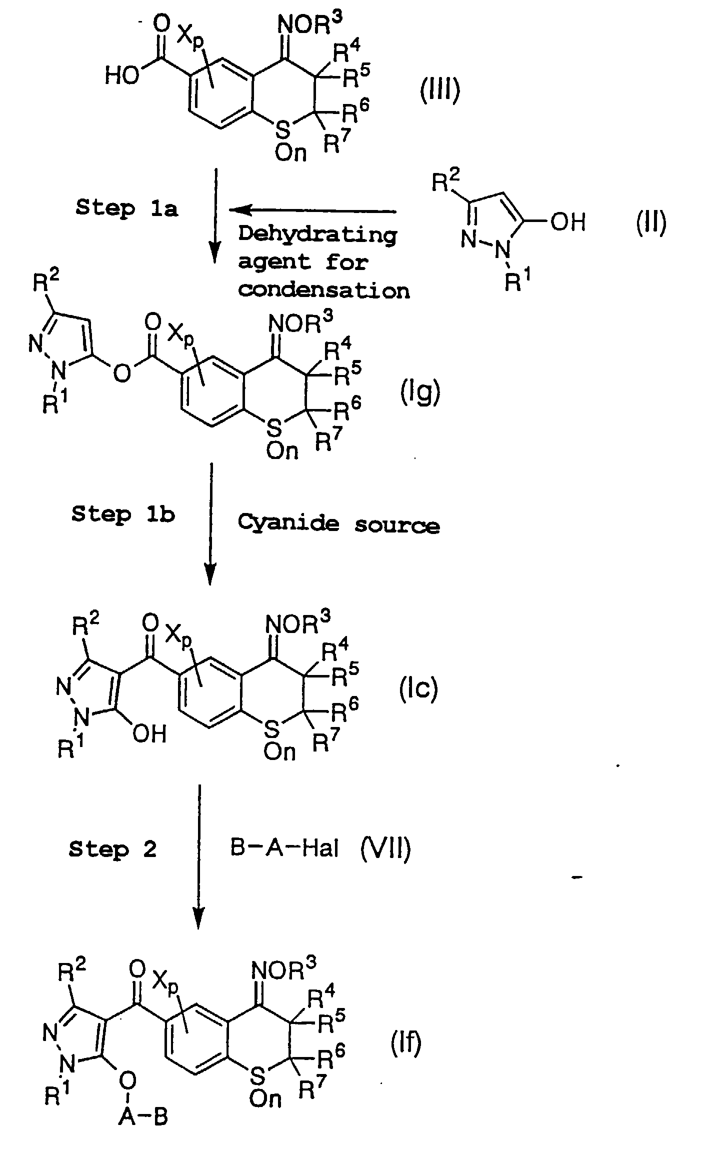

In the above reaction scheme, R¹, R², R³, R⁴, R⁵, R⁶, R⁷, X, p, A, B and n are as defined in the pyrazole derivative of the formula (I), and Hal is a halogen atom.

The pyrazole derivative of the formula (I) in which Q is a hydrogen atom (pyrazole derivative of the formula (Ic)) is produced by step 1a in which a carboxylic acid of the formula (III) and a pyrazole compound of the formula (II) are condensed to form an ester and step 1b in which the ester (Ig) formed by condensation is subject to rearrangement. Further, the pyrazole derivative of the formula (I) in which Q is a group of -A-B (pyrazole derivative of the formula (If)) is produced by step 2 in which the group of -A-B is further introduced into hydroxyl group of the pyrazole derivative obtained in step 1. Each step will be explained in detail hereinafter.

### Step 1a

The compound of the formula (III) and the compound of the formula (II) are allowed to react in an inert solvent in the presence of a dehydrating agent such as DCC (N,N'-dicyclohexylcarbodiimide), CDI (1,1'-carbonyldiimidazole) or EDC (1-(3-dimethylaminopropyl)3-ethylcarbodiimide), to prepare a pyrazole ester.

In the above reaction, the amount of the compound of the formula (II) per mole of the compound of the formula (III) is preferably 1.0 to 3 mol. The amount of the dehydrating agent per mole of the compound of the formula (III) is preferably 1.0 to 1.5 mol. The inert solvent is not specially limited so long as it is inert to the reaction. The inert solvent is preferably selected from secondary and tertiary alcohols such as t-butyl alcohol, t-amyl alcohol and 2-propanol, halogen-containing solvents such as dichloromethane, 1,2-dichloroethane, chloroform and chlorobenzene, and ether solvents such as diethyl ether, tetrahydrofuran, dioxane and 1,2-dimethoxyethane. The reaction temperature may be in the range of from -20°C to the boiling point of the solvent, while it is preferably around room temperature (10 ∼ 30°C).

Alternatively, the pyrazole ester (Ig) can be also prepared by reacting the compound of the formula (III) with a halogenating agent such as thionyl chloride, phosphorus oxychloride or phosphorus tribromide in an inert solvent to convert the compound of the formula (III) into a corresponding acid halide and reacting the acid halide with the compound of the formula (II) in an inert solvent in the presence of a base.

In the above reaction, the amount of the halogenating agent per mole of the compound of the formula (III) is preferably at least 1.0 mol. The inert solvent is not specially limited so long as it is inert to the reaction. For example, it is preferably selected from halogen-containing solvents such as dichloromethane, 1,2-dichloroethane, carbon tetrachloride and chlorobenzene and aromatic hydrocarbons such as benzene, toluene and xylene.

When the halogenating agent is a liquid, e.g., thionyl chloride, the halogenating agent may be used in an excess amount for use as a solvent. The reaction temperature may be in the range of room temperature to the boiling point of the solvent, while it is preferably 50 to 100°C.

The amount of the compound of the formula (II) per mole of the acid halide is preferably 1.0 to 3.0 mol. The base is not specially limited, while the base is selected from organic bases such as triethylamine and pyridine and inorganic bases such as sodium carbonate and potassium carbonate, and is used in an amount of 1.0 to 3.0 mol per mole of the acid halide. The solvent to be used in the above reaction is not specially limited so long as it is inert to the reaction, while it is preferably selected from halogen-containing solvents such as dichloromethane, 1,2-dichloroethane, chloroform and chlorobenzene. The reaction temperature may be in the range of from -20°C to the boiling point of the solvent, while it is preferably -20°C to 20°C.

### Step 1b

The pyrazole ester of the formula (Ig) is allowed to react in an inert solvent in the presence of a base, to prepare a pyrazole derivative of the formula (Ic). In this case, the reaction can be proceeded with under more moderate conditions in the co-presence of a so-called "cyanide source" in the reaction system. The "cyanide source" refers to a compound which generates a cyanide ion in the reaction system, and includes organic cyanohydrin compounds such as acetone cyanohydrin. Otherwise, cyanide ion can be generated in an organic solvent by the combined use of an inorganic cyanide ion compound such as sodium cyanide or potassium cyanide and a metal ion inclusion type phase transfer catalyst such as 18-crown-6 or benzo-18-crown-6. The "cyanide source" is not necessarily required for the reaction. When it is used, however, the amount of the cyanide source per mole of the pyrazole ester is 0.01 to 0.2 mol.

The base used in the above reaction is not specially limited. However, preferably, the base is selected from organic bases such as triethylamine and pyridine and inorganic bases such as sodium carbonate or potassium carbonate, and is used in an amount of 1.0 to 3.0 N per 1 N of the pyrazole ester. The inert solvent is not specially limited so long as it is inert to the reaction, while dioxane or acetonitrile is preferred. The reaction temperature is preferably around room temperature when the "cyanide source" is co-present, and it is preferably 50 to 130°C when the "cyanide source" is absent. The reaction temperature is particularly preferably around room temperature (10 - 25°C) in acetonitrile in the presence of triethylamine as a base when the "cyanide source" is used, and it is also particularly preferably the boiling point (101°C ) of dioxane as a solvent in the presence of potassium carbonate as a base when the "cyanide source" is not used.

### Step Iab

In steps 1a and 1b in the above reaction scheme, the pyrazole derivative of the formula (Ic) can be produced by one reaction without isolating the pyrazole ester (Ig) as an intermediate by using suitable reaction reagent and conditions. For example, DCC is used as a dehydrating agent in step 1a, and the compound of the formula (II) and the compound of the formula (III) may be allowed to react in an inert solvent in the presence of a base.

In the above reaction, the amount of the formula (II) per mole of the compound of the formula (III) is preferably 1.0 to 3.0 mol. The amount of DCC per mole of the compound of the formula (III) is preferably 1.0 to 1.5 mol. The base used together with DCC is not specially limited, while it is preferred to use potassium carbonate or sodium carbonate in an amount of 0.5 to 2.0 mol per mole of the compound of the formula (III). The inert solvent is not specially limited so long as it is inert to the reaction, while it is preferably selected from t-butyl alcohol, t-amyl alcohol and i-propyl alcohol. The reaction temperature may be in the range of from room temperature to the boiling point of the solvent, while it is preferably 50 to 100°C.

The pyrazole compound of the formula (II) used as a reaction reagent in the above reaction can be produced by one of the following method depending upon substituent thereof. In the following reaction schemes, R¹ and R² are as defined already.
(1) Method disclosed in East German Patent 83145 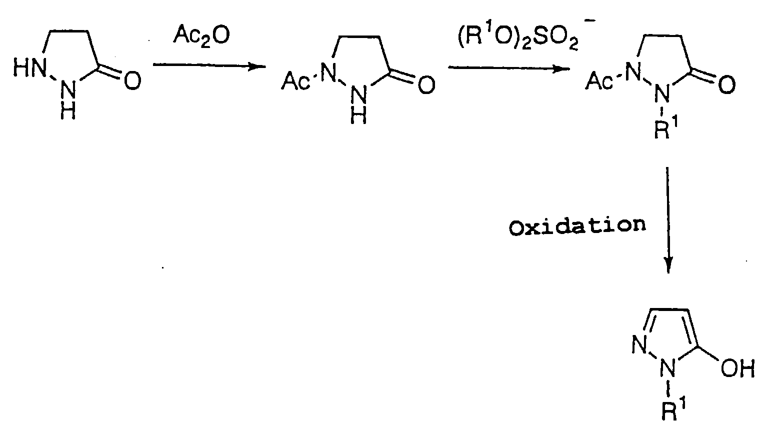
(2) Method disclosed in U. S. Patent 4,744,815 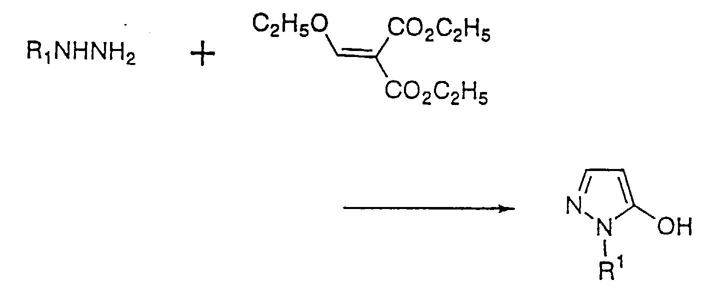
(3) Method disclosed in U. S. Patent 4,931,565 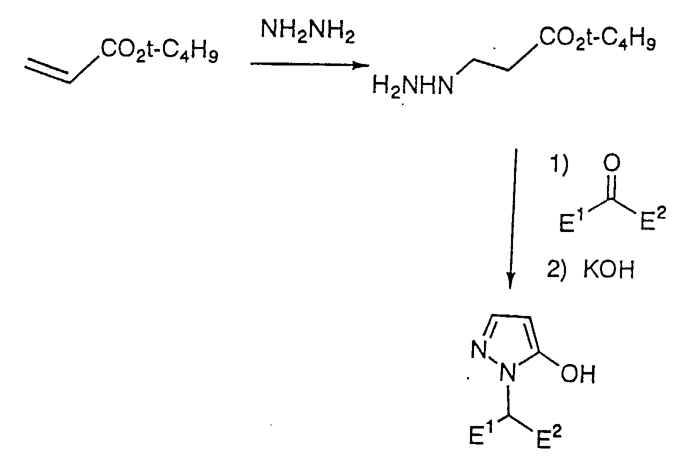
   Each of E¹ and E² is independently hydrogen, an optionally substituted alkyl group, an alkenyl group.
(4) Method disclosed in JP-A-3-44375 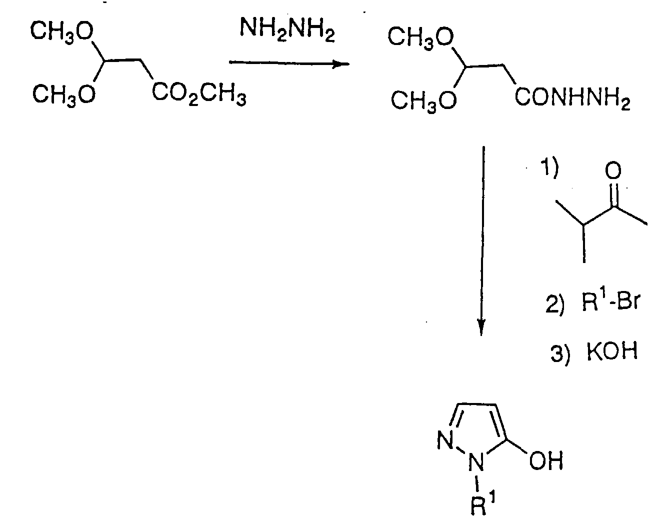
(5) Method disclosed in Ber. Vol. 43, page 2,106 (1910)

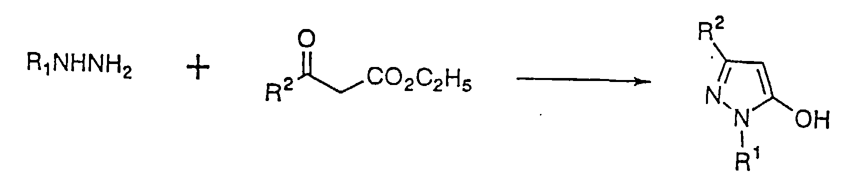

When 5-hydroxypyrazoles which are compounds of the formula (II) in which R² is a hydrogen atom are prepared, the above methods (1) to (4) are employed. When 5-hydroxypyrazoles which are compounds of the formula (II) in which R² is a C₁∼C₄ alkyl group, a C₁∼C₄ haloalkyl group or a C₂∼C₄ alkoxyalkyl group are prepared, the above method (5) is employed.

### Step 2

The compound (Ic) obtained in step 1 is reacted with B-A-Hal (VII) (A, B and Hal are as defined already) in an inert solvent in the presence of a base, to obtain the compound (If).

In this step, the amount of the compound (VII) per mole of the compound (Ic) is preferably 1 to 3 mol. Further, for trapping hydrogen halide by-produced in the reaction, it is preferred to use a base such as sodium carbonate, potassium carbonate, triethylamine or pyridine in at least an equimolar amount based on the starting material of the formula (Ic). The reaction temperature is preferably in the range of from room temperature to the boiling point of the solvent. The solvent used in the reaction is preferably selected from aromatic hydrocarbons such as benzene and toluene, ethers such as diethyl ether, ketones such as methyl ethyl ketone, and halogenated hydrocarbons such as methylene chloride and chloroform. Further, a two-phase solvent containing the above solvent and water may be used. In this case, a more preferred result can be obtained by adding a phase transfer catalyst such as crown ether or benzyltriethylammonium chloride to the reaction system.

The carboxylic acid of the formula (III) used as a starting material in the process for the production of the pyrazole derivative of the above formula (I), is a novel compound disclosed in no literature, and it can be prepared in one of the following schemes depending upon substituent thereof. 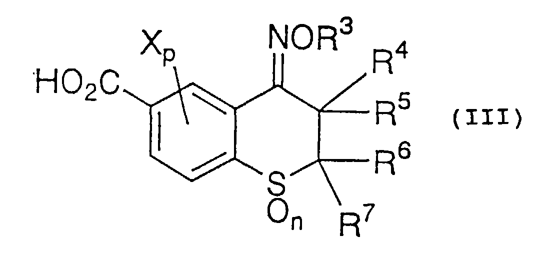 wherein X, p, R³, R⁴, R⁵, R⁶, R⁷ and n are as defined in the pyrazole derivative (I), provided that compounds of the formula (III) wherein p is 1, R³ is a C₁∼C₄ alkyl group, X is a C₁∼C₄ alkyl group and all of R⁴, R⁵, R⁶ and R⁷ are hydrogen atoms and compounds of the formula (III) wherein p is 2, R³ is a C₁∼C₄ alkyl group, two Xs are both C₁∼C₄ alkyl groups and all of R⁴, R⁵, R⁶ and R⁷ are hydrogen atoms are excluded.

In the schemes 1 to 5, R³, R⁴, R⁵, R⁶, R⁷, X and p are as defined in the pyrazole derivative of the formula (I), Hal is a halogen atom, q is 1 or 2, R in the scheme 4 is a C₁∼C₄ alkyl group, and X¹ in the scheme 5 is a halogen atom, a C₁∼C₄ haloalkyl group or a C₁∼C₄ alkoxy group, substituted on the 5-position on a thiochroman ring.

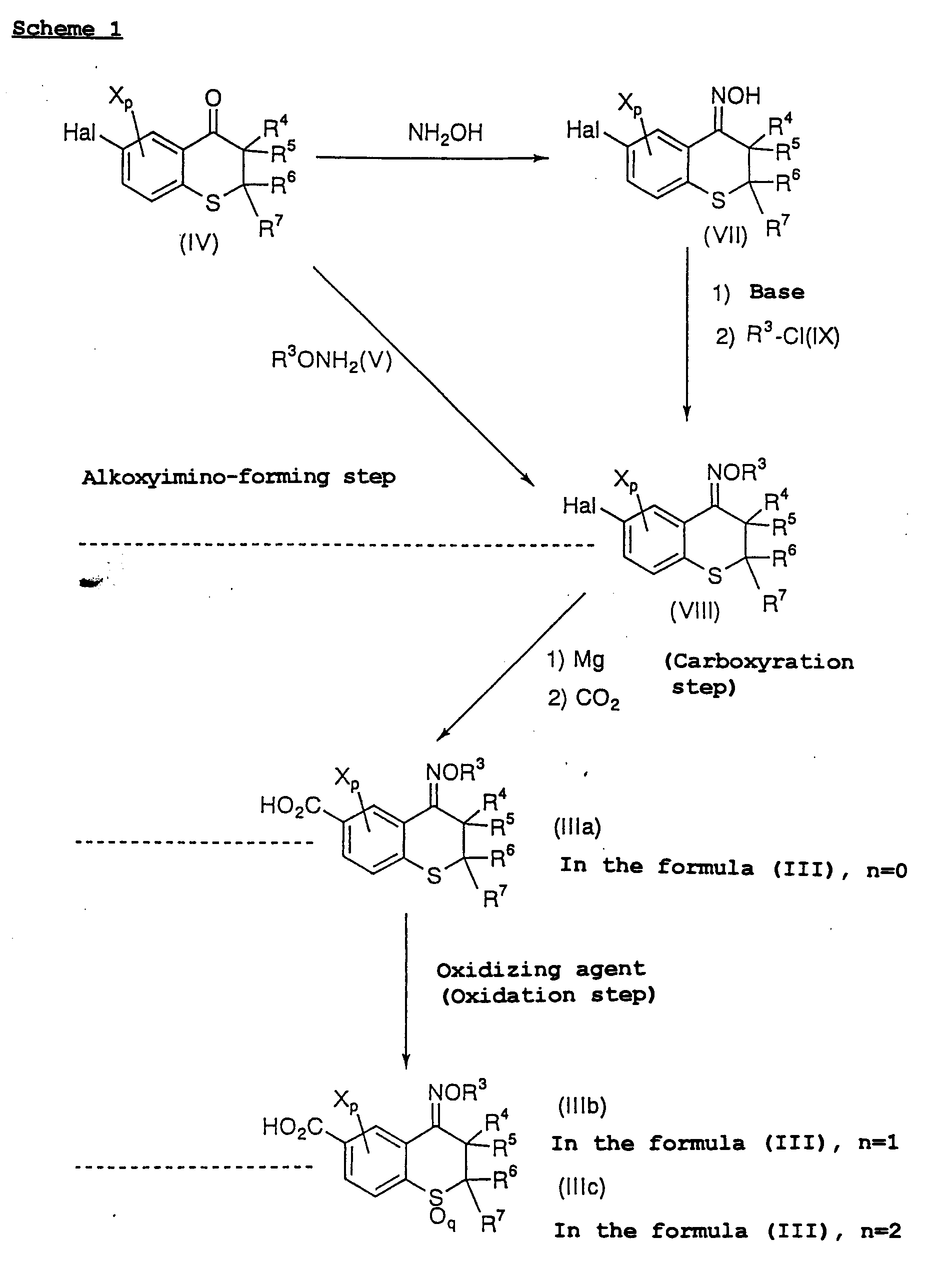

A carboxylic acid of the formula (III) in which the number of oxygen atom bonding to the sulfur atom is zero (i.e., n = 0, sulfide) (compound of the formula (IIIa)), is prepared by a step in which the carbonyl group of the thiochroman ring is converted to alkoxyimino and a carboxylation step in which the halogen atom is replaced with a carboxyl group. A compound of the formula (III) in which the number of oxygen atom(s) is 1 or 2 (i.e., n = 1 or n = 2, sulfoxide or sulfone) (compound of the formula (IIIb) or (IIIc)) is prepared by a step in which the carboxylic acid of the above formula (IIIa) is further oxidized. Each step will be explained hereinafter.

A halogenated thiochroman-4-one of the formula (IV) used as a starting material can be produced by various methods such as methods disclosed in JP-A-58-198483, International Laid-open Patent Publication WO88/06155 and Canadian Journal of Chemistry, vol. 51, page 839 (1973).

### Alkoxyimino-forming step

The synthesis of an oxime ether (VIII) by the conversion of a ketone (IV) to alkoxyimino is carried out by reacting a ketone (IV) with an alkoxyamine (V) in water or an organic solvent (e.g., ethanol, methanol or acetic acid) in the presence of an acid catalyst (e.g., hydrochloric acid) or a basic catalyst (e.g., pyridine, aniline, sodium hydroxide or sodium carbonate) at a temperature of 0°C to the reflux temperature of the solvent (water or organic solvent). In one embodiment, the synthesis is preferably carried out in ethanol in the presence of pyridine at the reflux temperature. In this reaction, the amount of the alkoxyamine (V) per mole of the ketone (IV) is preferably 1.0 to 5.0 mol, particularly preferably 1.0 to 2.0 mol.

The alkoxyamine or its mineral acid salt can be prepared according to a known method, e.g., the method described in "Organic Functional Group Preparations", page 377 and thereafter, S. R. Sandler and W. Caro, Academic Press, New York (1989).

In another method, the oxime ether (VIII) can be prepared by, first, reacting the ketone (IV) with a hydroxylamine to convert carbonyl group to hydroxyimino group, and then reacting it with R³-Cl (IX) in the presence of a base.

The synthesis of the oxime (VII) by converting the ketone (IV) to hydroxyimino can be carried out in the same manner as in the synthesis of the oxime ether (VIII) by the conversion of the ketone (IV) to the alkoxyimino, except that the alkoxyamine (V) is replaced with a hydroxylamine.

Then, the oxime ether (VIII) is obtained by reacting the oxime (VII) with a base (e.g., preferably sodium hydride, sodium methoxide or sodium ethoxide), and then reacting it with R³-Cl (IX), in an organic solvent (e.g., preferably, ether solvent such as diethyl ether, dimethoxyethane or tetrahydrofuran, or aprotic polar solvent such as dimethylformamide or dimethylacetamide).

The amount of the above base per mole of the oxime (VII) is preferably 1.0 to 1.5 mol, and the amount of R³-Cl (IX) per mole of the oxime (VII) is preferably 1.0 to 3.0 mol.

The reaction temperature is in the range of from 0°C to the reflux temperature of the solvent, and the reaction time is 30 minutes to 24 hours, preferably 1 to 3 hours.

### Carboxylation step

Then, the oxime ether (VIII) is reacted with magnesium (Mg) to form a Grignard reagent, and the Grignard reagent is reacted with carbon dioxide (CO₂) to obtain a sulfide (IIIa) (in the formula (III) , n = 0) which is included in the carboxylic acid of the formula (III). It is preferred to use an ether such as diethyl ether or tetrahydrofuran as a solvent. The reaction temperature is -78 to 50°C, particularly preferably 0 to 50°C.

The amount of the magnesium (Mg) for obtaining the Grignard reagent, per mole of the oxime ether (VIII), is preferably 1.0 to 5.0 mol. The Grignard reaction is preferably carried out in the co-presence of an alkyl iodide such as methyl iodide or an alkyl bromide such as ethyl bromide, since the reaction proceeds smoothly. The amount of the alkyl halide in this case is preferably 0.1 to 3.0 mol per mole of the oxime ether (VIII).

The reaction between the Grignard reagent and carbon dioxide (CO₂) is carried out by introducing carbon dioxide gas into the Grignard reagent in the same manner as in solvent from a carbon dioxide container, or by introducing carbon dioxide gas generated from dry ice (solid carbonate) into the Grignard reagent. Alternatively, dry ice may be directly added to the Grignard reagent.

### Oxidation step

A sulfoxide (IIIb) or a sulfone (IIIc) is obtained by reacting the sulfide (IIIa) obtained in the above carboxylation step with an oxidizing agent (e.g., hydrogen peroxide, peracetic acid or sodium metaperiodate) in a solvent (e.g., acetic acid, water or methanol). When the amount of the oxidizing agent is 1 equivalent based on the sulfide (IIIa), the sulfoxide (IIIb) is obtained. When the amount of the oxidizing agent is 2 equivalents based on the sulfide (IIIa), the sulfone (IIIc) is obtained.

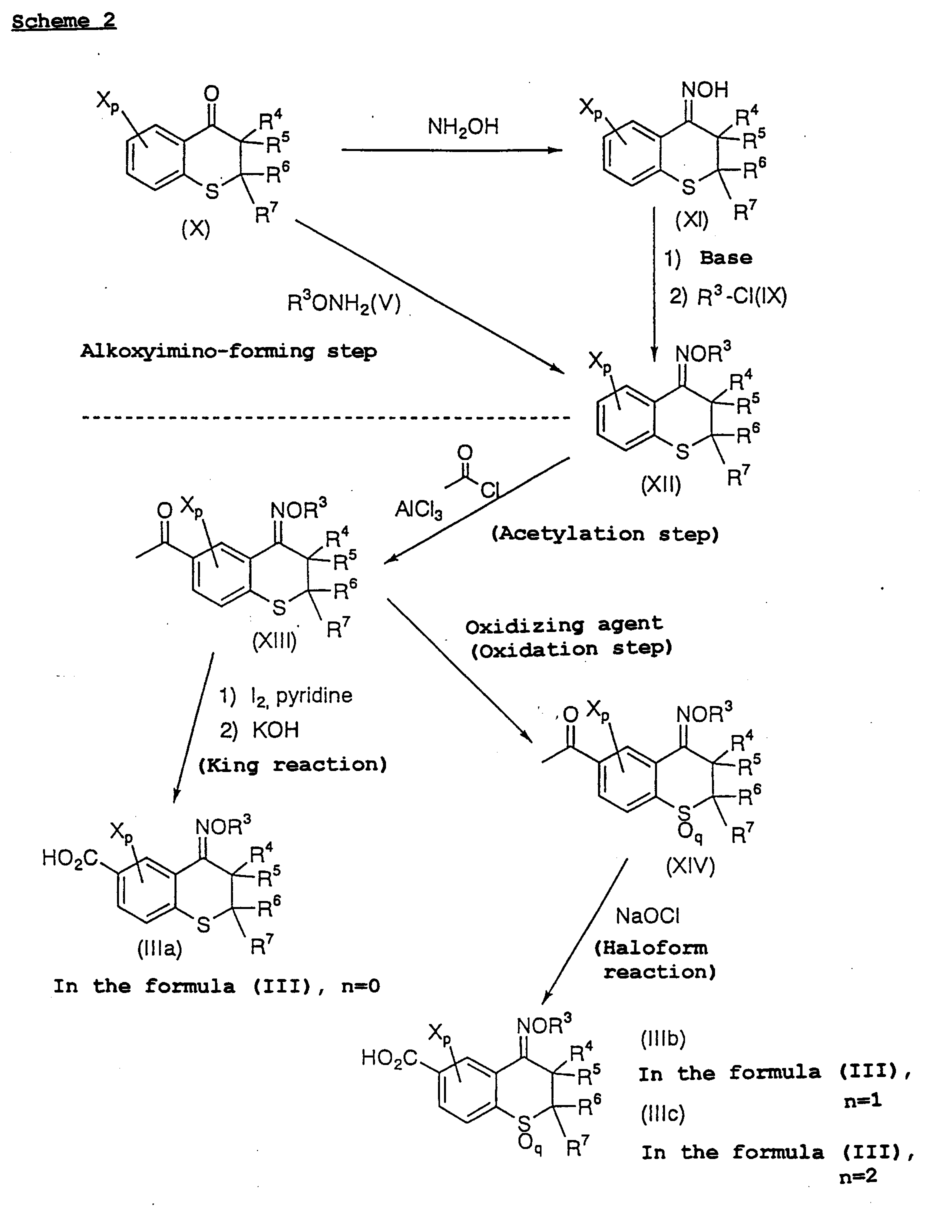

The step of forming alkoxyimino and the oxidation step can be carried out basically in the same manner as in the scheme 1.

### Acetylation step

The reaction in the acetylation step is a generally known Friedel-Crafts reaction. The reaction conditions, etc., are described in detail, for example, in "Shin-Jikken Kagaku Koza 14, Syntheses and Reactions of Organic Compounds II, page 799 (Maruzen)", according to which the reaction can be carried out.

### King reaction

The King reaction is described in detail, for example, in "Journal of American chemical Society, Vol. 66 (1944), page 1,612" and "the same Journal, Vol. 73 (1951), page 3,803", according to which the King reaction can be carried out.

### Haloform reaction

The haloform reaction is described in detail, for example, in "Shin-Jikken Kagaku Koza 15, Oxidation and Reduction [I-1], page 377 (Maruzen)", according to which the haloform reaction can be carried out.

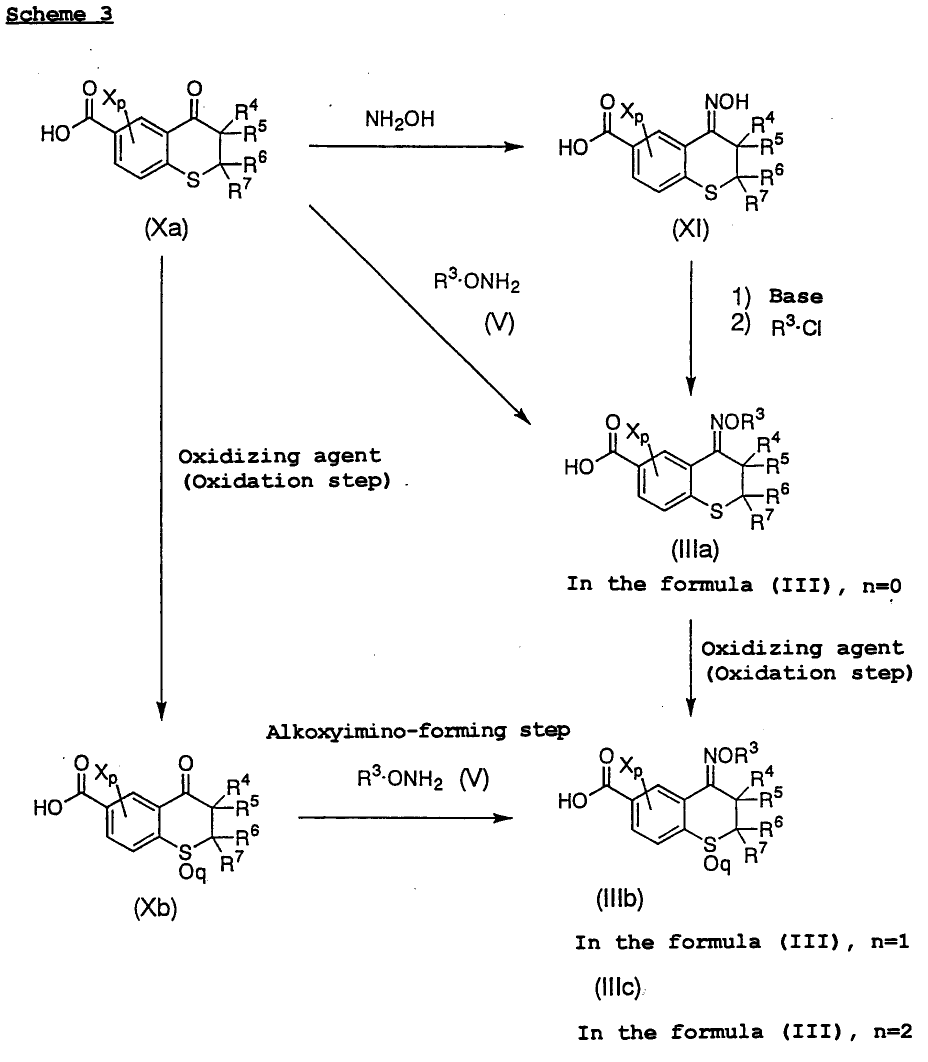

The alkoxyimino-forming step and the oxidation steps can be carried out basically in the same manner as in the scheme 1.

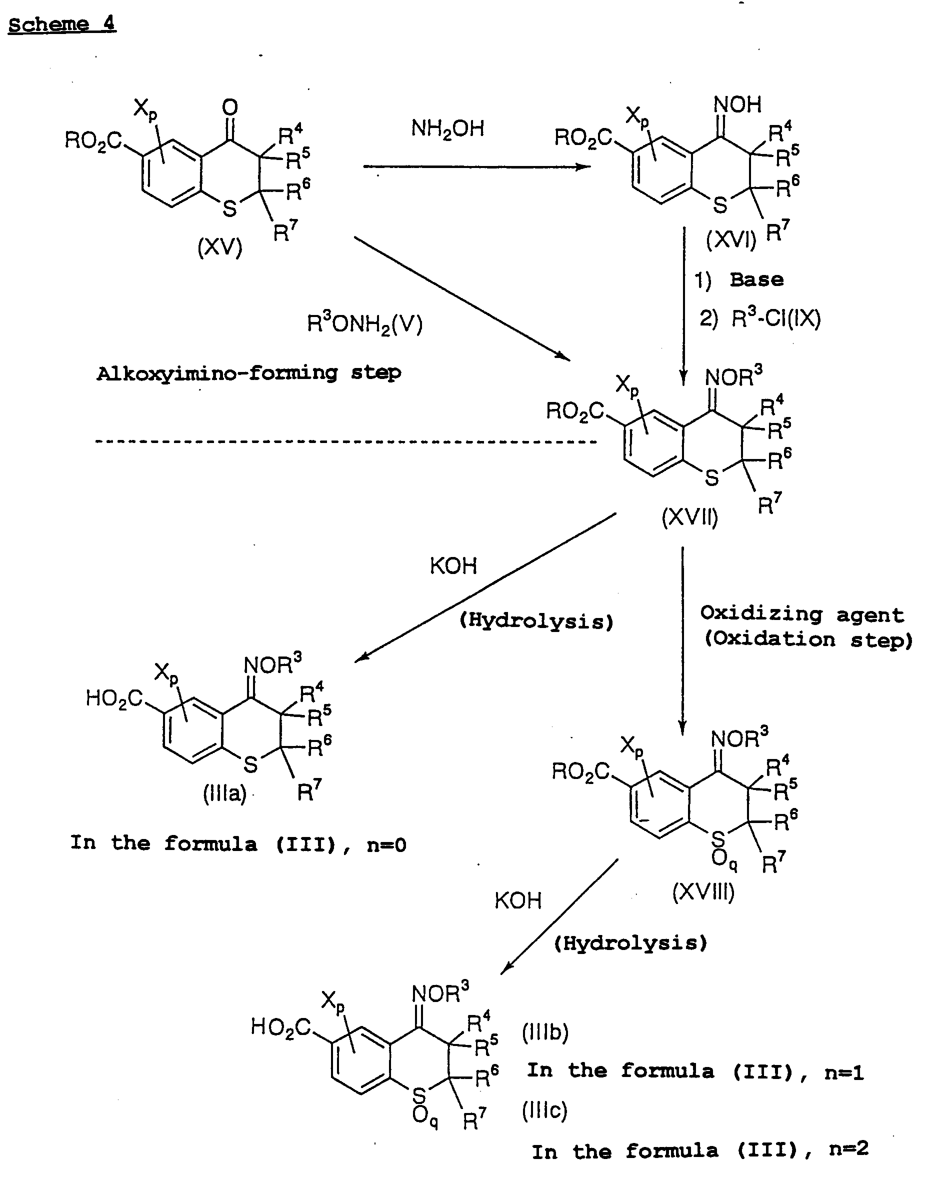

The alkoxyimino-forming step and the oxidation step can be carried out basically in the same manner as in the scheme 1.

An ester (XVII) or (XVIII) can be hydrolyzed by dissolving the ester (XVII) or (XVIII) in an alcohol solvent such as methanol, ethanol or isopropanol and reacting KOH in an amount of 1.0 to 10.0 mol, preferably 1.0 to 3.0 mol, per mole of the ester (XVII) or (XVIII). The reaction generally smoothly proceeds at room temperature. The reaction time is 1 to 8 hours, preferably 1 to 3 hours.

KOH can be rapidly dissolved in the co-presence of water in the reaction system, and the reaction can be further smoothly proceeded with by heating.

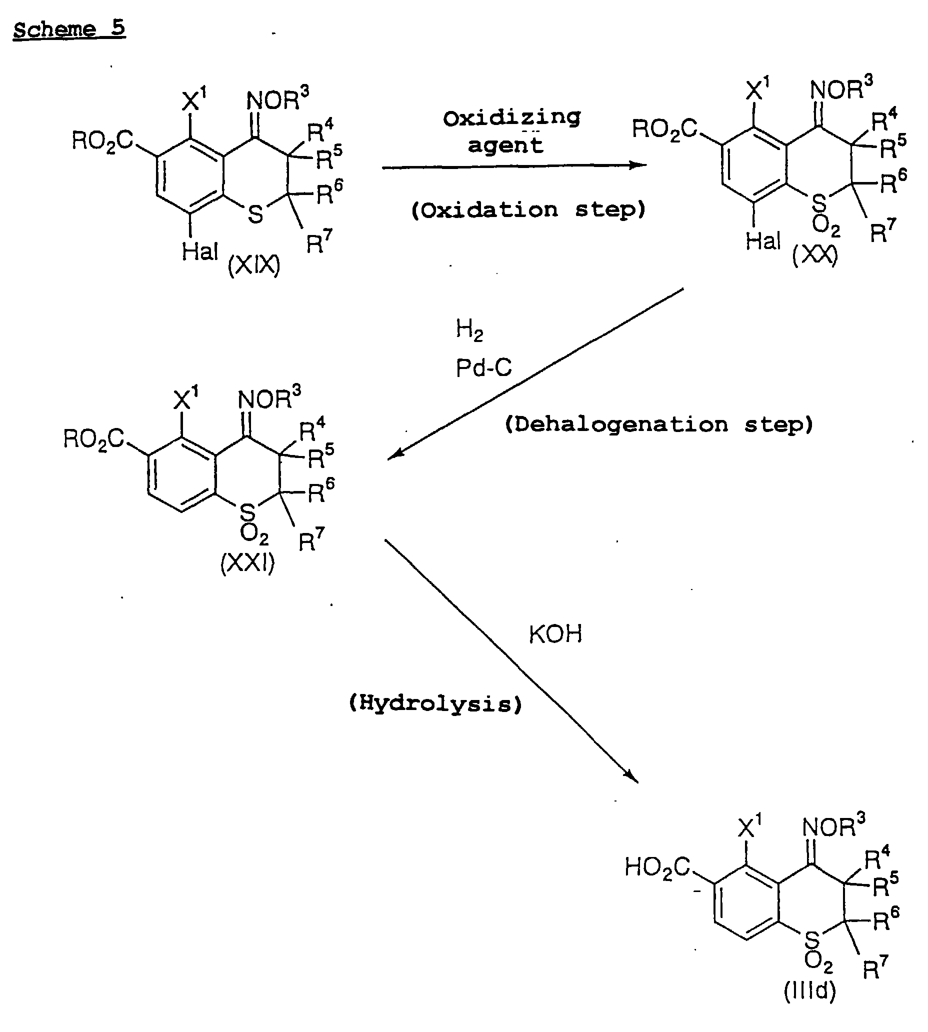

A carboxylic acid of the formula (IIId) represents a compound of the formula (III) in which p is 1, X is a halogen atom, a C₁∼C₄ haloalkyl group or a C₁∼C₄ alkoxy group, bonding to the 5-position on the thiochroman ring, and n is 2.

The oxidation step and the hydrolysis can be carried out basically in the same manner as in the scheme 1 or 4, respectively.

### Dehalogenation step

The dehalogenation step is a step in which an ester (XX) is dissolved in an alcohol solvent such as methanol or ethanol or an ether solvent such as dimethoxyethane, 1,4-dioxane or tetrahydrofuran and dehalogenated with hydrogen in the presence of a catalyst such as palladium-carbon (Pd-C).

For trapping formed hydrogen halide, it is preferred to use a base such as triethylamine or pyridine in at least one equivalent (mole) per mole of the ester (XX).

The catalyst as 5 % palladium-carbon is used in an amount of 50 to 500 mg per gram of the ester (XX). The hydrogen pressure is preferably in the range of from atmospheric pressure to 100 kg/cm², the reaction temperature is preferably in the range of room temperature to 120°C, and the reaction time is preferably 30 minutes to 8 hours. The amount of the co-present base is properly 1 to 1.5 equivalents (mol) per mole of the ester (XX).

The carboxylic acid of the formula (III) has geometric isomers of the following formulae (IIIf) and (IIIg) based on alkoxyimino group, and the carboxylic acid of the present invention includes these isomers and a mixture of these.

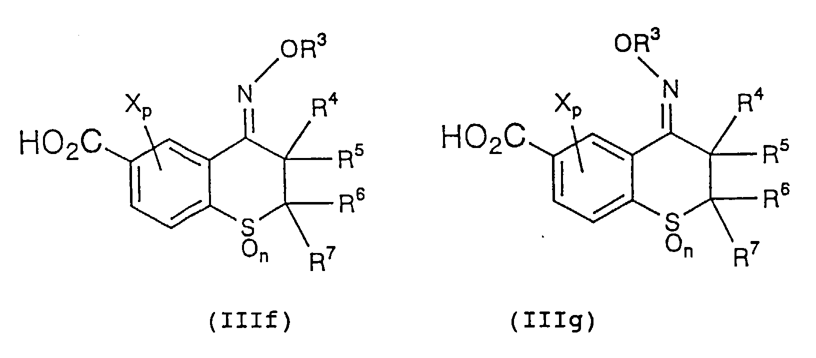

Some carboxylic acids of the formula (III) have asymmetric carbon, and various isomers are present. The carboxylic acid of the present invention includes all of the isomers and mixtures of the isomers.

Further, the carboxylic acid of the formula (III) is an acidic substance and can be easily converted to a salt by treating it with a base, and the salt is also included in the carboxylic acid of the present invention.

The above base can be selected from known bases without any limitation, and examples of the base include organic bases such as amines and anilines and inorganic bases such as sodium compounds and potassium compounds. Examples of the amines include monoalkylamine, dialkylamine and trialkylamine. Alkyl groups of the alkylamines are generally C₁∼C₄ alkyl groups . Examples of the anilines include aniline, monoalkylaniline and dialkylaniline. Alkyl groups of the alkylanilines are generally C₁∼C₄ alkyl groups. Examples of the sodium compounds include sodium hydroxide and sodium carbonate. Examples of the potassium compounds include potassium hydroxide and potassium carbonate.

A 4-oxothiochroman-6-carboxylic acid (Xa) or its ester (XV), used as a starting material in the above schemes 3 and 4, can be synthesized by various methods, e.g., by the following scheme 6 or 7.

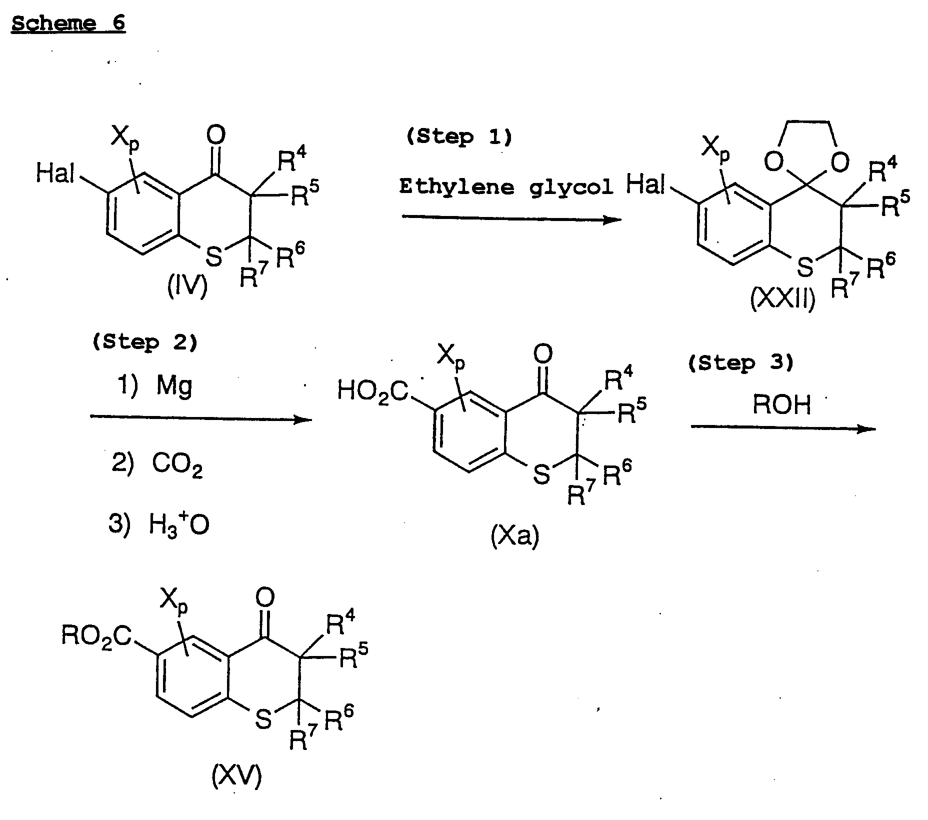

### (Step 1)

An acetal (XXII) is obtained by reacting the ketone (IV) used as a starting material in the scheme 1 with 1 to 10 equivalents (mole), per equivalent mole of the ketone (IV), of ethylene glycol in an aromatic hydrocarbon solvent such as benzene, xylene or toluene or a halogen-containing solvent such as dichloroethane or tetrachloroethane in the presence of an acid catalyst such as sulfuric acid or p-toluenesulfonic acid. The amount of the acid catalyst based on the ketone (IV) is generally 1 to 10 % by weight.

The reaction temperature is in the range of from 80°C to the reflux temperature of the solvent, and the reaction is preferably carried out by refluxing the solvent with removing water formed by azeotropically boiling. The reaction time is several hours to several tens hours.

### (Step 2)

The step 2 is the same carboxylation step as that explained concerning the scheme 1, and it can be carried out in the same manner.

### (Step 3)

The step 3 is a step in which 4-oxothiochroman-6-carboxylic acid (Xa) is reacted with an alcohol (ROH) to obtain a 4-oxothiochroman-6-carboxylic acid ester (XV).

The amount of the alcohol (ROH) per mole of the 4-oxothiochroman-6-carboxylic acid (Xa) is approximately 1 to 5 equivalents (mole). As a reaction solvent, the alcohol (ROH) may be used, or the solvent may be selected from the aromatic hydrocarbon solvents or halogen-containing solvents described concerning the step 1.

It is preferred to use the same acid catalyst as that described concerning the step 1 in an amount of 1 to 20 % by weight based on the 4-oxothiochroman-6-carboxylic acid (Xa), and it is preferred to reflux the solvent with removing formed water. The reaction time is several hours to tens hours.

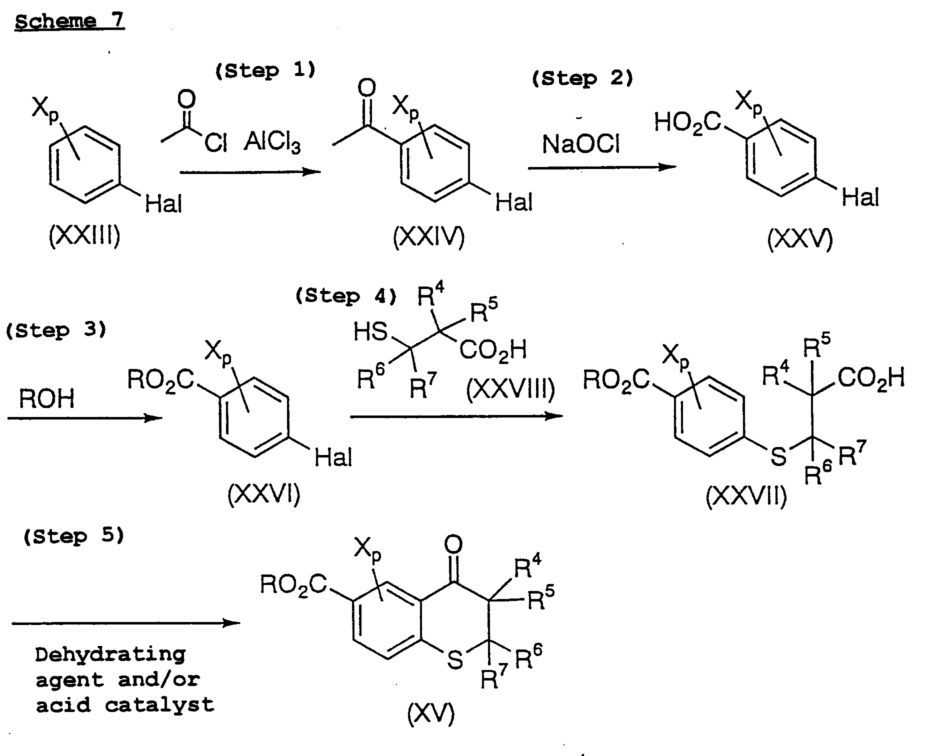

### (Step 1)

The step 1 is an acetylation step according to a Friedel-Crafts reaction explained concerning the scheme 2, and it can be carried out in the same manner as in the scheme 2.

### (Step 2)

The step 2 is the same haloform reaction as that explained concerning the scheme 2, and it can be carried out in the same manner as in the scheme 2.

### (Step 3)

The step 3 is the same esterification as that explained concerning the scheme 6, and it can be carried out in the same manner as in the step 3 in the scheme 6.

### (Step 4)

The step 4 is a step in which an ester (XXVI) is reacted with mercaptopropionic acid (XXVIII) to obtain a sulfide (XXVII).

The amount of the mercaptopropionic acid (XXVIII) per mole of the ester (XXVI) is 1.1 to 1.5 equivalents (mole). For trapping formed hydrogen halide, a base such as potassium carbonate or sodium carbonate is co-present. The amount of the base per equivalent weight (mole) of the ester (XXVI) is 1 to 1.5 equivalent s (mole). As a reaction solvent, preferred are aprotic polar solvents such as dimethylformamide (DMF) and dimethylacetamide (DMA). The reaction temperature may be in the range of from room temperature to the reflux temperature of the solvent, while it is preferably 80 to 120°C. The reaction is completed approximately in 1 to 24 hours depending upon substituent, and it is generally completed in 2 to 6 hours.

### (Step 5)

The step 5 is a step in which the sulfide (XXVII) is reacted with a dehydrating agent such as sulfuric acid or polyphosphoric acid to obtain 4-oxothiochroman-6-carboxylic acid ester (XV).

It is sufficient to use the dehydrating agent in an amount of at least 1 equivalent (mole) per mole of the sulfide (XXVII). A large amount of the dehydrating agent may be used to use it as a solvent. The reaction temperature is approximately room temperature to 120°C, preferably 50 to 80°C. The reaction time is approximately 10 minutes to 3 hours.

The following Referential Preparation Example 1 shows the preparation of 8-fluoro-6-ethoxycarbonyl-5-methylthiochroman-4-one (5) corresponding to the 4-oxothiochroman-6-carboxylic acid ester (XV) obtained in the above scheme 7.

### Referential Preparation Example 1

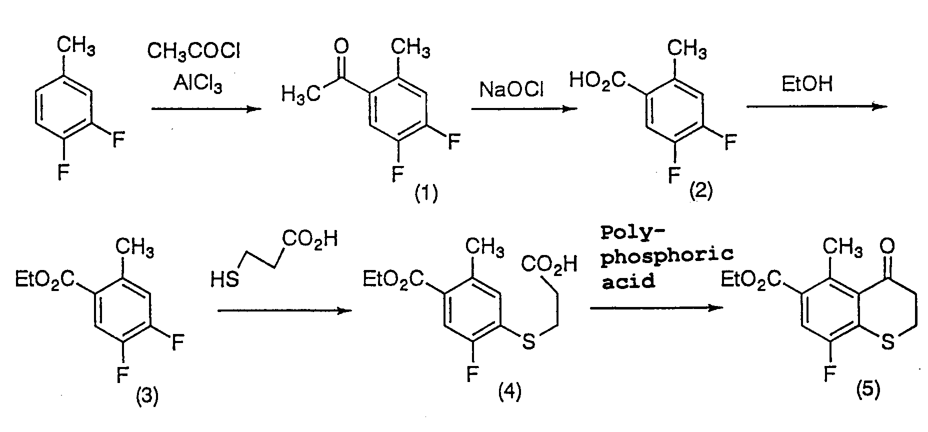

### Synthesis of 3,4-difluoro-6-methylacetophenone (1)

5.5ml (2.0eq., 77mmol) of acetyl chloride was added to a solution of 10.0 g (1.9 eq., 75 mmol) of aluminum chloride in 20 ml of 1,2-dichloroethane, and then 5.0 g (39 mmol) of 3,4-difluorotoluene was dropwise added with cooling with ice. After the completion of the addition, the mixture was stirred at room temperature for 5 hours. The reaction mixture was cooled and then gradually added to 100 ml of ice water to separate the reaction mixture to two layers. An organic layer was concentrated. An aqueous layer was extracted with methylene chloride, and the extract was added to the organic layer. The resultant mixture was washed with 5 % hydrochloric acid once, with a sodium hydrogencarbonate aqueous solution twice and with a saturated sodium chloride aqueous solution once, and then dried over anhydrous sodium sulfate. The solvent was distilled off under reduced pressure to give 6.77 g (yield 100 %) of a crude product of 3,4-difluoro-6-methylacetophenone.
NMR (ppm, solvent: deutero chloroform, internal standard: tetramethylsilane): 2.49(3H,s), 2.55(3H,s), 7.04(1H,dd, J = 7.7, 11.1), 7.55(1H,dd, J = 8.2)

### Synthesis of 3,4-difluoro-6-methylbenzoic acid (2)

130 ml of a solution of 6.77 g (39 mmol) of the above-obtained 3,4-difluoro-6-methylacetophenone in dioxane was cooled to 0°C with ice, and 130 ml (0.11 mmol) of a 6.3 % sodium hypochlorite aqueous solution was dropwise added at 5°C or lower. Then, the mixture was stirred in ice water bath for 2 hours. Then, a solution of 1.0 g (7.9 mmol) of sodium sulfite in 5 ml of water was added. The reaction mixture was washed with methylene chloride twice, and then 20 ml of concentrated hydrochloric acid was added with cooling with ice. The reaction mixture was extracted with ethyl acetate three times, and an organic layer was dried over anhydrous sodium sulfate. The solvent was distilled off under reduced pressure to give 5.41 g (yield 79 %) of a crude product of 3,4-difluoro-6-methylbenzoic acid.
NMR (ppm, solvent: deutero chloroform, internal standard: tetramethylsilane):2.60(3H,s), 7.07(1H,dd, J = 7.7, 10.8), 7.89(1H,dd, J = 8.4)

### Synthesis of ethyl 3,4-difluoro-6-methylbenzoate (3)

5.41 Grams (31 mmol) of the above-obtained 3,4-difluoro-6-methylbeozoic acid was dissolved in 40 ml of ethanol, 6 ml of concentrated sulfuric acid was added, and the mixture was refluxed under heat for 7 hours. Ice water was added to the reaction mixture, and the mixture was extracted with ethyl acetate twice. The resultant organic layer was consecutively washed with a sodium hydrogencarbonate aqueous solution and with a saturated sodium chloride aqueous solution, and dried over anhydrous sodium sulfate. The solvent was distilled off under reduced pressure to give 6.29 g (yield 100 %) of a crude product of ethyl 3,4-difluoro-6-methylbenzoate.
NMR (ppm, solvent: deutero chloroform, internal standard: tetramethylsilane):1.39(3H,t, J = 7.0), 2.57(3H,s), 4.35(2H,q), 7.03(1H,dd, J = 7.7, 11.1), 7.77(1H,dd, J = 8.4)

### Synthesis of 3-(2-fluoro-4-ethoxycarbonyl-5-methylphenylthio)propionic acid (4)

4.0 Grams (1.2 eq., 38 mmol) of 3-mercaptopropionic acid was added to a solution of 6.29 g (31 mmol) of the above-obtained ethyl 3,4-difluoro-6-methylbenzoate and 20 ml of a solution of 6.0 g (1.4 eq., 43 mmol) of potassium carbonate in DMF, and then the mixture was stirred under heat at 80 ∼ 90°C for 6 hours. The reaction mixture was cooled, then ice water was added, and the mixture was washed with methylene chloride twice. Concentrated hydrochloric acid was added to an aqueous layer, and the aqueous layer was extracted with ethyl acetate three times. Then, an organic layer was dried over anhydrous sodium sulfate. The solvent was distilled off under reduced pressure to give 6.36 g (yield 70 %) of a crude product of 3-(2-fluoro-4-ethoxycarbonyl-5-methylphenylthio)-propionic acid.
NMR (ppm, solvent: deutero chloroform, internal standard: tetramethylsilane):1.38(3H,t, J = 7.3), 2.56(3H,s), 2.71(2H,t, J = 6.3), 3.22(2H,t), 7.18(1H,d,J = 7.3), 7.62(1H,d, J = 10.4)

### Synthesis of 8-fluoro-6-ethoxycarbonyl-5-methylthiochroman-4-one (5)

15 Grams of polyphosphoric acid was added to 2.0 g (7.0 mmol) of the above-obtained 3-(2-fluoro-4-ethoxycarbonyl-5-methylphenylthio)propionic acid, and the mixture was stirred under heat at 50 ∼ 60°C for 30 minutes. The reaction mixture was allowed to cool to room temperature and then was gradually added to ice, and the mixture was extracted with ethyl acetate three times. The resultant organic layer was washed with a sodium hydrogencarbonate aqueous solution twice, with water twice and with a saturated sodium chloride aqueous solution once, and dried over anhydrous sodium sulfate. The solvent was distilled off under reduced pressure to give 1.69 g (yield 90 %) of a crude product of 8-fluoro-6-ethoxycarbonyl-5-methylthiochroman-4-one.
NMR (ppm, solvent: deutero chloroform, internal standard:tetramethylsilane):1.39(3H,t, J = 7.1), 2.66(3H,s), 2.9 - 3.1(2H,m), 3.2-3.4(2H,m), 4.36(2H,q), 7.51(1H,d, J = 9.9)

The following Referential Preparation Examples 2 and 3 show the preparation of 2-fluoroethoxyamine hydrochloride and propargyloxyamine hydrochloride, respectively.

### Referential Preparation Example 2

### (1) Synthesis of N-(2-fluoroethoxy)phthalimide

1.7 Grams (10 mmol) of N-hydroxyphthalimide and 1.3 g (10 mmol) of 2-fluoro-1-bromoethane were dissolved in 10 ml of dimethylformamide, further, 2 g (14 mmol) of potassium carbonate was added, and the mixture was stirred at 60°C for 7 hours. The reaction mixture was diluted with 100 ml of water, and a formed solid was collected by filtration, washed with water and dried under reduced pressure to give 1.3 g (yield 60 %) of N-(2-fluoroethoxy)phthalimide.
NMR (ppm, solvent: deutero chloroform, internal standard: tetramethylsilane): 4.3-5.1(4H,m), 7.82(4H,m)

### (2) Synthesis of 2-fluoroethoxyamine hydrochloride

5 Grams (24 mmol) of N-(2-fluoroethoxy)phthalimide was dissolved in 30 ml of chloroform, and further, the resultant solution was dissolved in 30 ml of ethanol. To this solution was added 2.3 ml (48 mmol) of hydrazine hydrate, and the mixture was stirred at 70°C for 1 hour. The reaction mixture was diluted with 150 ml of chloroform, a formed solid was removed by filtration, and the filtrate was washed with 20 ml of water. The resultant organic layer was dried over anhydrous sodium sulfate and acidified with concentrated hydrochloric acid, and the solvent was distilled off. The resultant residue was dried under reduced pressure to give 1.9 g (yield 69 %) of 2-fluoroethoxyamine hydrochloride in the form of a white plate-like crystal.
NMR (ppm, solvent: deutero chloroform + deutero methanol, internal standard: tetramethylsilane): 4.1-4.9(4H,m)

### Referential Preparation Example 3

### (1) Synthesis of N-(propargyloxylphthalimide

5 Grams (31 mmol) of N-hydroxyphthalimide and 3.7 g (31 mmol) of propargyl bromide were dissolved in 40 ml of dimethylformamide, further, 4.3 g (31 mmol) of potassium carbonate was added, and the mixture was stirred at 70°C for 4 hours. The reaction mixture was diluted with 150 ml of water, and a formed solid was collected by filtration, washed with water and dried under reduced pressure to give 5.4 g (yield 69 %) of N-(propargyloxy)phthalimide.
NMR (ppm, solvent: deutero chloroform, internal standard: tetramethylsilane): 2.61(1H,t, J=2), 4.89(2H,d, J=2), 7.6-7.9(4H,m)

### (2) Synthesis of propargyloxyamine hydrochloride

5.4 Grams (27 mmol) of N-(propargyloxy)phthalimide was dissolved in 30 ml of chloroform, and further, the resultant solution was dissolved in 30 ml of ethanol. To this was added 2.6 ml (54 mmol) of hydrazine hydrate, and the mixture was stirred at 70°C for 1 hour. The reaction mixture was diluted with 100 ml of chloroform, a formed solid was removed by filtration, and the filtrate was washed with 50 ml of water. The resultant organic layer was dried over anhydrous sodium sulfate and acidified with concentrated hydrochloric acid, and the solvent was distilled off. The resultant residue was dried under reduced pressure to give 2.5 g (yield 86 %) of propargyloxyamine hydrochloride in the form of a yellowish acicular crystal.
NMR (ppm, solvent: deutero chloroform + deutero methanol, internal standard: tetramethylsilane): 2.95(1H,t, J = 2), 4.80(2H,d, J = 2)

### EXAMPLES

The present invention will be specifically explained with reference to Preparation Examples and Herbicide Examples hereinafter, while the present invention shall not be limited thereto.

### Carboxylic Acid (III) Preparation Example 1

### Synthesis of 8-chloro-4-methoxyimino-5-methylthiochroman6-carboxylic acid (carboxylic acid of the formula (IIIa); in the formula (III), n = 0) (Scheme 1)

### (1) Alkoxyimino-forming step

A 100-ml eggplant type flask was charged with 10.0 g (34 mmol) of 6-bromo-8-chloro-5-methylthiochroman-4-one corresponding to the compound of the formula (IV) in the scheme 1, 5.0 g (1.8 eq., 60 mmol) of O-methylhydroxylamine hydrochloride corresponding to the compound of the formula (V), 25 ml of ethanol and 13 ml of pyridine, and the mixture was refluxed under heat for 2 hours. After allowed to cool, the reaction mixture was added to 100 ml of 5 % hydrochloric acid. A precipitated solid was recovered by filtration to give 10.4 g (yield 86 %) of 6-bromo-8-chloro-4-methoxyimino-5-methylthiochroman.

### (2) Carboxylation step

2.7 Grams (110 mmol) of magnesium was suspended in 80 ml of dry tetrahydrofuran (THF) in a 200-ml three-necked flask, and to this suspension was added 10.4 g (32 mmol) of the 6-bromo-8-chloro-4-methoxyimino-5-methylthiochroman corresponding to the compound of the formula (VIII) in the scheme 1, obtained in the above (1). Then, 7.0 g (2.0 eq., 64 mmol) of ethyl bromide was added, and the mixture was refluxed under heat for 1.5 hours. After allowed to cool, the reaction mixture was cooled to 10°C with an ice bath, and carbon dioxide gas was bubbled for 30 minutes. The tetrahydrofuran was removed under reduced pressure, and the reaction mixture was recovered by means of ethyl acetate and washed with 100 ml of 5 % hydrochloric acid three times. Then, the reaction mixture was extracted with 100 ml of a 5 % potassium carbonate aqueous solution twice, and the resultant extracts were combined and adjusted to a pH of 1 with 10 % hydrochloric acid. A formed solid was recovered by filtration to give 5.7 g (yield 63 %) of 8-chloro-4-methoxyimino-5-methylthiochroman-6-carboxylic acid as the captioned end product corresponding to the compound of the formula (IIIa).

### Carboxylic Acid (III) Preparation Example 2

### Preparation of 8-chloro-4-methoxyimino-5-methylthiochroman-6-carboxylic acid-1,1-dioxide (carboxylic acid of the formula (IIIc); in the formula (III) , n = 2) (Scheme 1)

A 50-ml eggplant type flask was charged with 3.0 g (10.5 mmol) of the 8-chloro-4-methoxyimino-5-methylthiochroman-6-carboxylic acid obtained in the above Carboxylic Acid Preparation Example 1, corresponding to the compound of the formula (IIIa), 5 ml of acetic acid and 3.0 g (2.5 eq., 26mmol) of a 30 % hydrogen peroxide aqueous solution, and the mixture was allowed to react at 80°C for 2 hours. After allowed to cool, the reaction mixture was poured into 50 ml of water, and a formed solid was recovered by filtration to give 3.1 g (yield 93 %) of 8-chloro-4-methoxyimino-5-methylthiochroman-6-carboxylic acid-1,1-dioxide as the captioned end product corresponding to the compound of the formula (IIIc).
NMR (ppm, solvent: deutero acetone, internal standard: tetramethylsilane): 2.63(3H,s), 3.25 ∼ 3.71(4H,m), 4.02(3H,s), 7.87(H,s)

### Carboxylic Acid (III) Preparation Example 3

### Synthesis of 8-fluoro-4-methoxyimino-5-methylthiochroman-6-carboxylic acid-1,1-dioxide (carboxylic acid of the formula (IIIc); in the formula (III), n = 2) (Scheme 4)

### (1) Alkoxyimino-forming step

1.69 Grams (6.3 mmol) of 8-fluoro-6-ethoxycarbonyl-5-methylthiochroman-4-one corresponding to the compound of the formula (XV) in the scheme 4 was dissolved in 6.0 ml of ethanol. 0.63 Grams (1.2 eq., 7.5 mmol) of O-methylhydroxylamine hydrochloride corresponding to the compound of the formula (V) and 2.0 ml of pyridine were added, and the mixture was stirred under heat at 90°C for 8 hours. After the completion of the reaction, 30 ml of water was added to the reaction mixture, and the reaction mixture was extracted with ethyl acetate three times. The resultant organic layer was washed with 5 % hydrochloric acid and with a saturated sodium chloride aqueous solution, and dried over anhydrous sodium sulfate. The solvent was distilled off to give 1.40 g (yield 74 %) of 8-fluoro-6-ethoxycarbonyl-5-methyl-4-methoxyiminothiochroman corresponding to the compound of the formula (XVII).
NMR (ppm, solvent: CDCl₃, internal standard: tetramethylsilane): 1.38(3H,s, J = 7.3), 2.65(3H,s), 2.8-3.0(2H,m), 3.05-3.15(2H,m), 4.00(3H,s), 4.35(2H,q), 7.44(1H,d, J = 9.7)

### (2) Oxidation step

1.4 Grams (4.7 mmol) of the 8-fluoro-6-ethoxycarbonyl-5-methyl-4-methoxyiminothiochroman obtained in the above (1), corresponding to the compound of the formula (XVII), was dissolved in 2.0 ml of acetic acid, and 3.0 ml (5.5 eq., 26 mmol) of a 30 % hydrogen peroxide aqueous solution was added. The mixture was stirred at 80°C for 2.5 hours. After the completion of the reaction, 10 ml of water and 1 ml of an aqueous solution of 0.2 g (2 mmol) of sodium hydrogensulfite were added, and the mixture was extracted with ethyl acetate three times. The resultant organic layer was washed with a saturated sodium chloride aqueous solution and dried over anhydrous sodium sulfate, and the solvent was distilled off to give 1.50 g (yield 97 %) of 8-fluoro-6-ethoxycarbonyl-5-methyl-4-methoxyiminothiochroman-1,1-dioxide corresponding to the compound of the formula (XVIII).
NMR (ppm, solvent: CDCl₃, internal standard: tetramethylsilane): 1.40(3H,s, J = 7.3), 2.62(3H,s), 3.3-3.5(4H,m), 4.07(3H,s), 4.40(2H,q), 7.51(1H,d, J = 10.2)

### (3) Hydrolysis step

1.4 Grams (4.3 mmol) of the 8-fluoro-6-ethoxycarbonyl-5-methyl-4-methoxyiminothiochroman-1,1-dioxide otained in the above (2), corresponding to the compound of the formula (XVIII), 0.40 g of potassium hydroxide, 15 ml of methanol and 5 ml of water were stirred under heat at 80°C for 1 hour. After the completion of the reaction, 50 ml of water was added to the reaction mixture to separate it to two layers. The resultant aqueous layer was washed with ethyl acetate, then neutralized with concentrated hydrochloric acid and extracted with ethyl acetate. The resultant organic layer was washed with a saturated sodium chloride aqueous solution and then dried over anhydrous sodium sulfate, and the solvent was distilled off to give 1.11 g (yield 87 %) of the intended 8-fluoro-4-methoxyimino-5-methylthiochroman-6-carboxylic acid-1,1-dioxide corresponding to the compound of the formula (IIIc).
NMR (ppm, solvent: CDCl₃, internal standard: tetramethylsilane): 2.70(3H,s), 3.4-3.5(4H,m), 4.08(3H,s), 7.70(1H,d, J = 10.2)

### Carboxylic Acid (III) Preparation Example 4

### Synthesis of 5,8-dichloro-4-methoxyiminothiochroman-6-carboxylic acid (carboxylic acid of the formula (IIIa); in the formula (III), n = 0) (Scheme 1)

### (1) Alkoxyimino-forming step

13.0 Grams (41.7 mmol) of 6-bromo-5,8-dichlorothiochroman-4-one was dissolved in 100.0 ml of ethanol. 7.0 Grams (2.0 eq., 83.3 mmol) of O-methylhydroxylamine hydrochloride and 6.8 ml of pyridine were added, and the mixture was stirred at 90°C for 3 hours. After the completion of the reaction, 30 ml of water was added to the reaction mixture, and the reaction mixture was extracted with ethyl acetate twice. The resultant organic layer was washed with 5 % hydrochloric acid and with a saturated sodium chloride aqueous solution, and dried over anhydrous sodium sulfate, and the solvent was distilled off to give 14.2 g (yield 100 %) of 6-bromo-5,8-dichloro-4-methoxyiminothiochroman.
NMR (ppm, solvent: deutero chloroform, internal standard: tetramethylsilane): 2.82-3.25(4H,m), 4.02(3H,s), 7.60(1H,s)

### (2) Carboxylation step

6.0 Grams (249.5 mmol) of magnesium was suspended in 500 ml of diethyl ether, and to this suspension was added 17.0 g (49.9 mmol) of the 6-bromo-5,8-dichloro-4-methoxyiminothiochroman obtained by the method in the above (1). Then, 13.0 ml (174.7 mmol) of ethyl bromide was added, and the mixture was refluxed under heat for 4 hours. Thereafter, the reaction system was cooled to 20°C, and carbon dioxide gas introduced for 30 minutes for carrying out a reaction. After the completion of the reaction, water and hydrochloric acid were added, excessive magnesium was removed by filtration, and the diethyl ether was distilled off under reduced pressure. Ethyl acetate was added to a residue to recover the reaction mixture, and an organic layer was isolated. The resultant organic layer was washed with 1 % hydrochloric acid, a saturated sodium hydrogencarbonate aqueous solution was added, and the intended product was extracted from an organic layer. The so-obtained aqueous layer was adjusted to a pH of 1 with 5 % hydrochloric acid, and a formed solid was recovered by filtration. The so-obtained solid was washed with water and dried in a desciccator to give 6.0 g (yield 40 %) of 5,8-dichloro-4-methoxyiminothiochroman-6-carboxylic acid corresponding to the compound of the formula (IIIa).
NMR (ppm, solvent: deutero acetone, internal standard: tetramethylsilane): 3.16(4H,s), 4.00(3H,s), 5.85(1H,bs), 7.75 (1H,s)

### Carboxylic Acid (III) Preparation Example 5

### Synthesis of 5,8-dichloro-4-methoxyiminothiochroman-6-carboxylic acid-1,1-dioxide (carboxylic acid of the formula (IIIc); in the formula (III) , n = 2) (Scheme 1)

0.13 Gram (0.42 mmol) of the 5,8-dichloro-4-methoxyiminothiochroman-6-carboxylic acid obtained in Carboxylic Acid (III) Preparation Example 4 was dissolved in 1.0 ml of acetic acid, 0.12 ml (2.5 eq., 1.1 mmol) of a 30 % hydrogen peroxide aqueous solution was added, and the mixture was stirred under heat at 80°C for 2 hours. After the completion of the reaction, 10 ml of water and 1 ml of an aqueous solution of 0.2 g (2 mmol) of sodium hydrogensulfite were added. Then, the mixture was extracted with ethyl acetate twice. The resultant organic layer was washed with a saturated sodium chloride aqueous solution and dried over anhydrous sodium sulfate, and the solvent was distilled off to give 0.13 g (yield 91 %) of 5,8-dichloro-4-methoxyiminothiochroman-6-carboxylic acid-1,1-dioxide corresponding to the compound of the formula (IIIc).
NMR (ppm, solvent: deutero acetone, internal standard: tetramethylsilane): 3.15-4.00(4H,s), 4.09(3H,s), 4.85(1H,bs), 7.88(1H,s)

### Carboxylic Acid (III) Preparation Example 6

### Synthesis of 5-chloro-4-methoxyiminothiochroman-6-carboxylic acid-1,1-dioxide (carboxylic acid of the formula (IIIc); in the formula (III), n = 2) (Scheme 5)

### (1) Oxidation step

2.1 Grams (6.3 mmol) of 5,8-dichloro-6-ethoxycarbonyl-4-methoxyiminothiochroman was dissolved in 3.0 ml of acetic acid, 1.6 ml (2.5 eq., 15.8 mmol) of a 30 % hydrogen peroxide aqueous solution was added, and the mixture was stirred under heat at 90°C for 2.0 hours. After the completion of the reaction, 10 ml of water and 1 ml of an aqueous solution of 0.2 g (2 mmol) of sodium hydrogensulfite were added. Then, the mixture was extracted with ethyl acetate twice. The resultant organic layer was washed with a saturated sodium chloride aqueous solution and dried over anhydrous sodium sulfate, and the solvent was distilled off to give 2.1 g (yield 91 %) of 5,8-dichloro-6-ethoxycarbonyl-4-methoxyiminothiochroman-1,1-dioxide.
NMR (ppm, solvent: deutero chloroform, internal standard: tetramethylsilane): 1.40(3H,t), 3.15-3.65(4H,m), 4.09(3H,s), 4.43(2H,q), 7.67(1H,s)

### (2) Dehalogenation step

2.1 Grams (5.7 mmol) of the 5,8-dichloro-6-ethoxycarbonyl-4-methoxyiminothiochroman-1,1-dioxide obtained in the above (1) was dissolved in 10 ml of tetrahydrofuran, and the mixture was placed in an autoclave. Then, 0.51 ml (1.1 eq., 6.3 mmol) of pyridine and 530 mg of 5 % palladium-carbon were added, and the mixture was allowed to react under the atmosphere of pressurized hydrogen gas (40 kgf/cm²) at 60°C for 2.5 hours. After the completion of the reaction, the catalyst was removed by filtration, and the reaction mixture was concentrated under reduced pressure. The remaining oil was dissolved in ethyl acetate, washed with 5 % hydrochloric acid and with a saturated sodium chloride aqueous solution and dried over anhydrous sodium sulfate, and the solvent was distilled off. The reaction mixture was recrystallized from a mixed solvent of ethyl acetate and hexane, and precipitated solid was recovered by filtration, and dried with a desciccator to give 0.75 g (yield 40 %) of 5-chloro-6-ethoxycarbonyl-4-methoxyiminothiochroman-1,1- dioxide.
NMR (ppm, solvent: deutero chloroform, internal standard: tetramethylsilane): 1.41(3H,t), 3.34(4H,t), 4.11(3H,s), 4.44(2H,q), 7.68(1H,d), 7.95(1H,d)

### (3) Hydrolysis step

0.75 Gram (2.3 mmol) of the 5-chloro-6-ethoxycarbonyl-4-methoxyiminothiochroman-1,1-dioxide obtained in the above (2), 0.18 g of potassium hydroxide, 10 ml of ethanol and 2 ml of water were stirred under heat at 70°C for 0.5 hour. After the completion of the reaction, the solvent was distilled off, 5 ml of water was added, and then, the mixture was adjusted to a pH of 1 with 5 % hydrochloric acid. Then, the mixture was extracted with ethyl acetate twice. The resultant organic layer was washed with a saturated sodium chloride aqueous solution and dried over anhydrous sodium sulfate, and the solvent was distilled off to give 0.72 g (yield 100 %) of 5-chloro-4-methoxyiminothiochroman-6-carboxylic acid-1,1-dioxide corresponding to the compound of the formula (IIIc).
NMR (ppm, solvent: deutero acetone, internal standard: tetramethylsilane): 3.2-3.75(4H,m), 4.09(3H,s), 7.89(1H,d), 8.00(1H,d), 10.10(1H,bs)

### Carboxylic Acid (III) Preparation Example 7

### Synthesis of 4-propargyloxyimino-5-methylthiochroman-6-carboxylic acid-1,1-dioxide (carboxylic acid of the formula (IIIc); in the formula (III), n = 2) (Scheme 3)

### (1) Alkoxyimino-forming step

1.4 Grams (5.5 mmol) of 5-methylthiochroman-4-one-6-carboxylic acid-1,1-dioxide and 1.2 g (11 mmol) of propargyloxyamine hydrochloride were suspended in 15 ml of ethanol, 1.3 ml (16 mmol) of pyridine was added, and the mixture was refluxed for 9 hours. The reaction mixture was diluted with 30 ml of water, acidified by adding 5 % hydrochloric acid and extracted with 150 ml of ethyl acetate. The resultant organic layer was washed with 50 ml of water and dried over anhydrous sodium sulfate, and the solvent was distilled off. The resultant residue was dried under reduced pressure to give 1.7 g (yield 100 %) of 4-propargyloxyimino-5-methylthiochroman-6- carboxylic acid-1,1-dioxide in the form of a yellowish glass-like solid.
NMR (ppm, solvent: deutero acetone, internal standard: tetramethylsilane): 2.71(3H,s), 3.07(1H,t, J = 2), 3.4-3.5(4H,m), 4.90(2H,d, J = 2), 7.85(1H,d, J = 7), 8.05(1H,d, J = 7)

### Carboxylic Acid (III) Preparation Example 8

### Synthesis of 4-allyloxyimino-5-methylthiochroman-6-carboxylic acid-1,1-dioxide (carboxylic acid of the formula (IIIc); in the formula (III), n = 2) (Scheme 3)

### (1) Alkoxyimino-forming step

1.4 Grams (5.5 mmol) of 5-methylthiochroman-4-one-6-carboxylic acid-1,1-dioxide and 1.2 g (11 mmol) of allyloxyamine hydrochloride were suspended in 15 ml of ethanol, and the mixture was refluxed for 3 hours. The reaction mixture was diluted with 150 ml of ethyl acetate. An organic layer was washed with 60 ml of 1 % hydrochloric acid and 40 ml of water, and dried over anhydrous sodium sulfate, and the solvent was distilled off. The resultant residue was dissolved in 6 ml of ethanol, a solution of 0.37 g of potassium hydroxide in 2 ml of water was added, and the mixture was allowed to stand at room temperature overnight. The solvent was distilled off from the reaction mixture, and the residue was dissolved in 100 ml of water. The mixture was adjusted to a pH of 10 by adding 10 ml of a 5 % potassium carbonate aqueous solution. The resultant solution was washed with 20 ml of ethyl acetate, and then the resultant aqueous layer was acidified with 5 % hydrochloric acid and extracted with 150 ml of ethyl acetate. The resultant organic layer was washed with 50 ml of water and dried over anhydrous sodium sulfate, and the solvent was distilled off. The resultant residue was dried under reduced pressure to give 1.4 g (yield 82 %) of 4-allyloxyimino-5-methylthiochroman-6-carboxylic acid-1,1-dioxide in the form of a yellowish glass-like solid.
NMR (ppm, solvent: deutero acetone, internal standard: tetramethylsilane): 2.67(3H,s), 3.44(2H,d, J = 3), 3.49(2H,d, J = 3), 4.77(2H,d, J = 5), 5.2-5.5(2H,m), 5.9-6.3(1H,m), 7.85(1H,d, J = 8), 7.98(1H,d, J = 8)

### Carboxylic Acid (III) Preparation Example 9

### Synthesis of 4-(2-fluoroethyl)oxyimino-5-methylthiochroman-6-carboxylic acid-1,1-dioxide (carboxylic acid of the formula (IIIc); in the formula (III), n = 2) (Scheme 3)

### (1) Alkoxyimino-forming step

1.0 Gram (3.9 mmol) of 5-methylthiochroman-4-one-6-carboxylic acid-1,1-dioxide and 0.7 g (6.1 mmol) of 2-fluoroethyloxyamine hydrochloride were suspended in 20 ml of t-amyl alcohol, and the suspension was refluxed for 2 hours. The reaction mixture was diluted with 150 ml of ethyl acetate. An organic layer was washed with 60 ml of 1 % hydrochloric acid and 50 ml of water and then dried over anhydrous sodium sulfate, and the solvent was distilled off. The resultant residue was dried under reduced pressure to give 1.2 g (yield 100 %) of 4-(2-fluoroethyl)oxyimino-5-methyl-thiochroman-6-carboxylic acid-1,1-dioxide in the form of a yellowish glass-like solid.
NMR (ppm, solvent: deutero acetone, internal standard: tetramethylsilane): 2.68(3H,s), 3.47(4H,m), 4.2-5.1(4H,m), 7.86 (1H,d, J = 8), 8.00(1H,d, J = 8)

Table 2 shows the structures of the carboxylic acids obtained in the above Carboxylic Acid (III) Preparation Examples 1 to 9 and the numbers of the schemes employed.

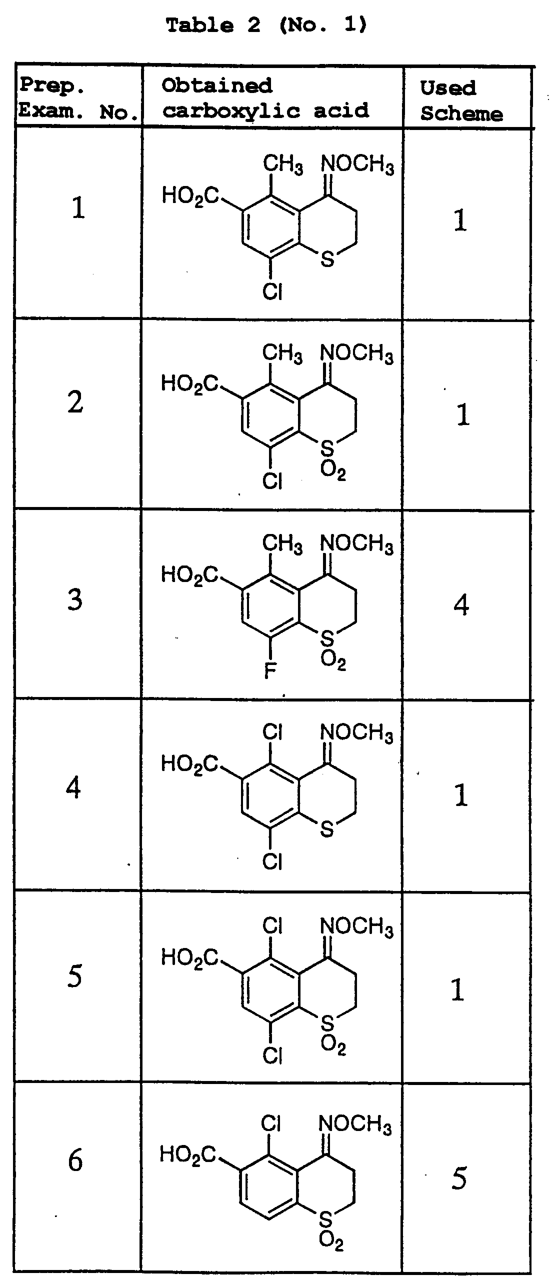

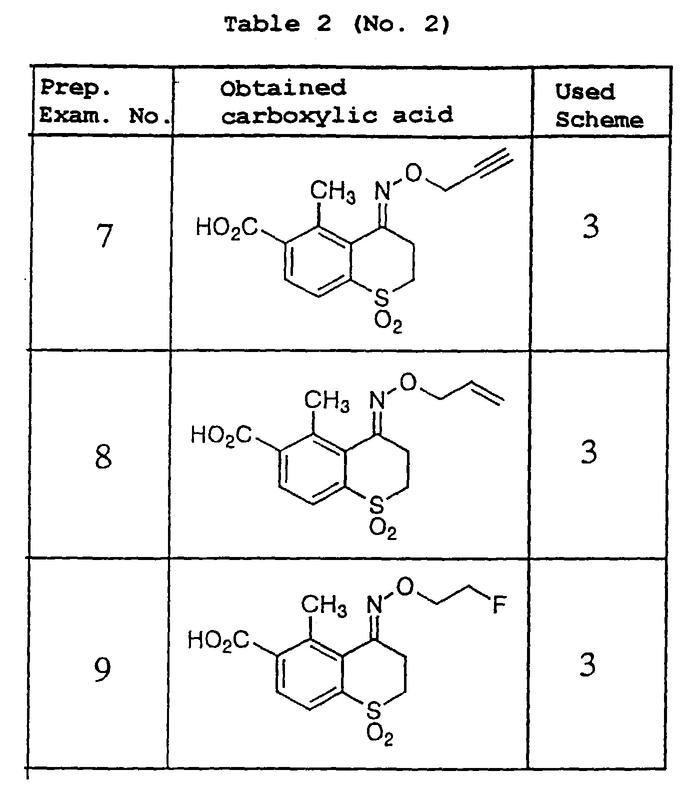

### Pyrazole Derivative (I) Preparation Example 1

### Synthesis of 8-chloro-4-methoxyimino-5-methyl-6-(1-ethyl-5-hydroxypyrazol-4-yl)carbonylthiochroman (compound of the formula (Ic)

A 100-ml eggplant type flask was charged with 1.7 g (5.4 mmol) of the 8-chloro-4-methoxyimino-5-methylthiochroman-6-carboxylic acid (corresponding to the compound of the formula (III)) obtained in Carboxylic Acid (III) Preparation Example 1, 1.3 g (1.2 eq., 6.3 mmol) of dicyclohexylcarbodiimide (DCC) as a condensing agent, 0.7 g (1.15 eq., 6.2 mmol) of 1-ethyl-5-hydroxypyrazole (corresponding to the compound of the formula (II) and 10 ml of t-amyl alcohol, and the mixture was allowed to react at 60°C for 30 minutes. Then, 0.6 g of potassium carbonate was added, and the mixture was allowed to react at 90°C for 8 hours. After the reaction mixture was allowed to cool, the t-amyl alcohol was removed under reduced pressure, water was added to the reaction mixture, the reaction mixture was dissolved in the water, and an insoluble was removed by filtration. An aqueous layer was washed with ethyl acetate, and then adjusted to a pH of 1 with 10 % hydrochloric acid. A precipitated solid was recovered by filtration to give 1.3 g (yield 57 %) of the captioned end product corresponding to the compound of the formula (Ic). Table 3 shows the structure of the starting material used in this Preparation Example and the structure and yield of the compound obtained in this Preparation Example. Table 4 shows physical property values of the obtained compound.

### Pyrazole Derivative (I) Preparation Examples 2 - 4

Compound Nos. 2 ∼ 4 corresponding to the compound of the formula (Ic) were obtained in the same manner as in Pyrazole Derivative (I) Preparation Example 1 except that the 8-chloro-4-methoxyimino-5-methylthiochroman-6-carboxylic acid corresponding to the compound of the formula (III)), used as a starting material in Pyrazole Derivative (I) Preparation Example 1, was replaced by starting materials (corresponding to the compound of the formula (III)) shown in Table 3. Table 3 shows the structures of the starting materials and the structures and yields of the obtained compounds. Table 4 shows physical property values of the obtained compounds.

### Pyrazole Derivative (I) Preparation Example 5

### Synthesis of 8-chloro-4-methoxyimino-5-methyl-6-(1-ethyl-5-p-toluenesulfonyloxypyrazol-4-yl)carbonylthiochroman (compound of the formula (If)

A 100-ml eggplant type flask was charged with 0.7 g (1.8 mmol) of the 8-chloro-4-methoxyimino-5-methylthiochroman-6-(1-ethyl-5-hydroxypyrazol-4-yl)carbonylthiochroman (corresponding to the compound of the formula (Ic)) obtained in Pyrazole Derivative (I) Preparation Example 1, 10 ml of methylene chloride was added, and the 8-chloro-4-methoxyimino-5-methylthiochroman-6-(1-ethyl-5-hydroxypyrazol-4-yl)carbonylthiochroman was dissolved therein. Further, 0.28 g (1.1 eq., 2.0 mmol) of potassium carbonate and 10 ml of water were added. Then, a solution of 0.38 g (1.1 eq., 2.0 mmol) of p-toluenesulfonyl chloride (corresponding to the compound of the formula (VII)) as a reaction reagent in a small amount of methylene chloride was added, and 10 mg of benzyltriethylammonium chloride as a phase transfer catalyst was added. The mixture was allowed to react at room temperature for 24 hours, a methylene chloride layer was separated, the methylene chloride was removed under reduced pressure, and the resultant crude product was purified by silica gel column chromatography (developer solvent = ethyl acetate:n-hexane = 1:3 mixed solvent) to give 0.87 g (yield 89 %) of the captioned end product corresponding to the compound of the formula (If). Table 3 shows the structure of the starting material and the reaction reagent and the structure and yield of the obtained compound. Table 4 shows physical property values of the obtained compound.

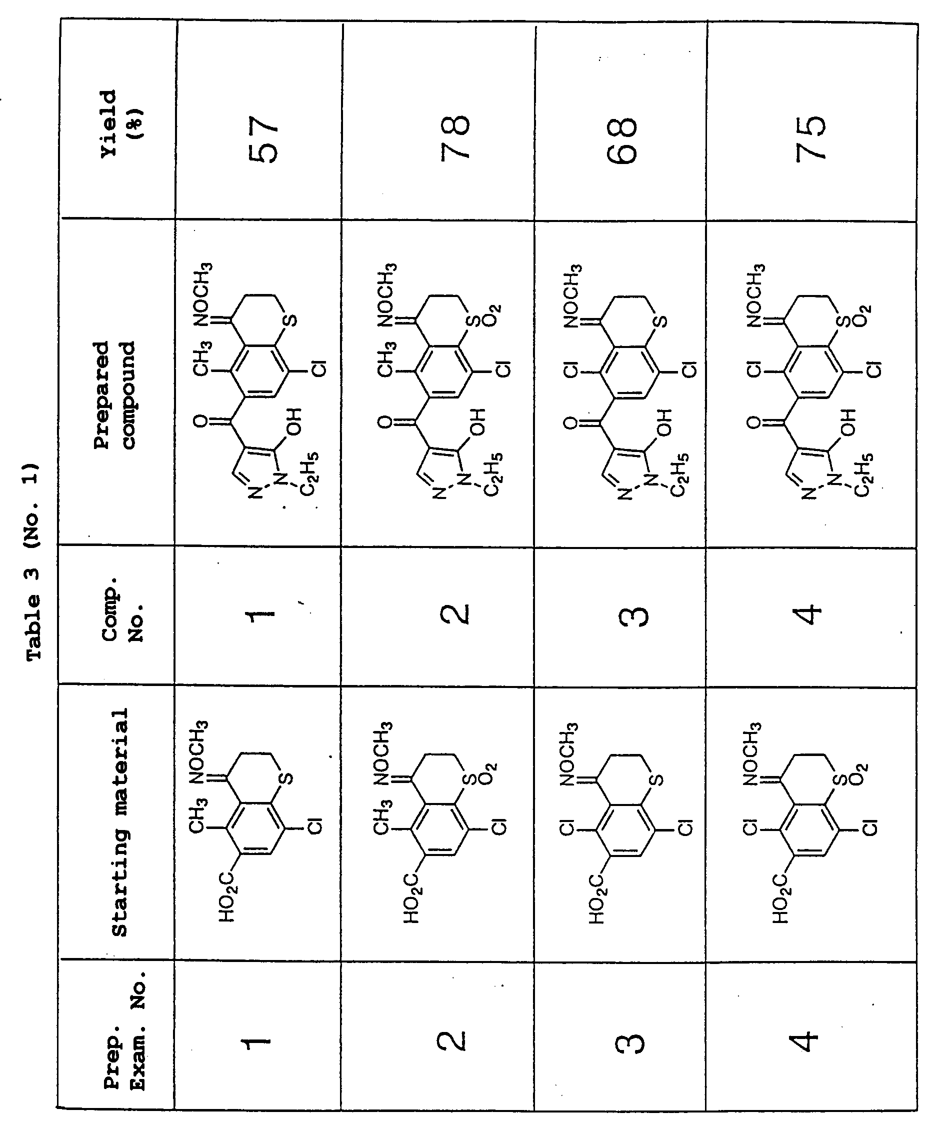

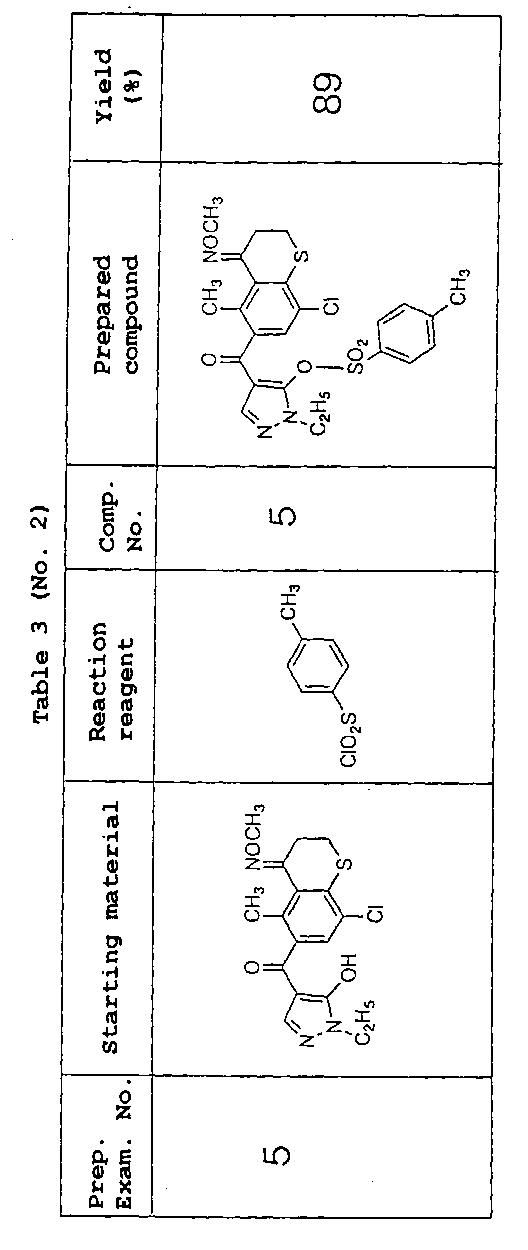

**Table 4**

| Comp. No. | NMR (ppm) Int. Standard: Tetramethylsilane Solvent: Deutero chloroform | IR(cm⁻¹) KBr tablet | Melting point (°C) |
|---|---|---|---|
| 1 | 1.45(3H, t) 2.52(3H, s) 2.85∼3.25(4H, m) 3.99(3H, s) 4.07(2H, q) 6.5(H, broad)7.38(H, s) 7.41(H, s) | 3000 | 121.9∼124.2 |
| | | 2960 | |
| | | 1630 | |
| 2 | 1.46(3H, t) 2.50(3H, s) 3.25∼3.55(4H, m) 4.05(3H, s) 4.09(2H, q) 7.34(H, s) 7.48(H, s) 8.2(H, broad) | 3000 | 111.1∼116.8 |
| | | 2950 | |
| | | 1640 | |
| | | 1320 | |
| | | 1130 | |
| 3 | 1.45(3H, t) 2.90∼3.25(4H, m) 4.03(3H, s) 4.08(2H, q) 5.4(H, broad) 7.35(H, s) 7.44(H, s) | | Glass-like substance |
| | | ---- | |
| 4 | 1.47(3H, t) 3.3∼3.55(4H, m) 4.10(3H, s) 4.12(2H, q) 4.2(H, broad) 7.20(H, s) 7.82(H, s) | | Glass-like substance |
| | | ---- | |
| 5 | 1.51(3H, t) 2.40(3H, s) 2.44(3H, s) 2.85∼3.20(4H, m) 3.98(3H, s) 4.21(2H, q) 7.06(H, s) 7.36(2H, d) 7.55(H, s) 7.87(2H, d) | 3000 | Oily substance |
| | | 2960 | |
| | | 1670 | |
| | | 1390 | |
| | | 1190 | |

### Pyrazole Derivative (I) Preparation Example 6

### Synthesis of 8-fluoro-4-methoxyimino-5-methyl-6-(1-ethyl-5-hydroxypyrazol-4-yl)carbonylthiochroman-1,1-dioxide (compound of the formula (Ic))

1.1 Grams (3.9 mmol) of the 8-fluoro-4-methoxyimino-5-methylthiochroman-6-carboxylic acid-1,1-dioxide corresponding to the compound of the formula (IIIc), obtained in Carboxylic Acid (III) Preparation Example 3, was dissolved in 12 ml of t-amyl alcohol, and 0.48 g (1.1 eq., 4.3 mmol) of 1-ethyl-5-hydroxypyrazole and 0.96 g (1.2 eq., 4.7 mmol) of cyclohexylcarbodiimide were added. The mixture was stirred at room temperature for 2 hours. Then, the mixture was stirred at 40°C for 30 minutes, and 0.41 g (3.0 mmol) of potassium carbonate was added. Then, the reaction mixture was temperature-increased to 80°C, and the reaction mixture was stirred for 8 hours. After the completion of the reaction, 50 ml of water was added to the reaction mixture, and the resultant aqueous layer was washed with methylene chloride, neutralized with 5 % hydrochloric acid and extracted with ethyl acetate. The resultant organic layer was washed with a saturated sodium chloride aqueous solution and dried over anhydrous sodium sulfate, and the solvent was distilled off to give 1.32 g (yield 86 %) the captioned end product corresponding to the compound of the formula (Ic). Table 5 shows the structure of the starting material used in this Preparation Example and the structure and yield of the compound obtained in this Preparation Example. Table 6 shows physical property values of the obtained compound.

### Pyrazole Derivative (I) Preparation Example 7

### Synthesis of 8-fluoro-4-methoxyimino-5-methyl-6-(1-ethyl-5-p-toluenesulfonyloxypyrazol-4-yl)carbonylthiochroman1,1-dioxide (compound of the formula (If))

The captioned end product was obtained at an yield of 60 % in the same manner as in Pyrazole Derivative (I) Preparation Example 5 except that the starting material was replaced with the compound 6 obtained in Pyrazole Derivative (I) Preparation Example 6. Table 5 shows the structures of the starting material and the reaction reagent and the structure and yield of the obtained compound. Table 6 shows the physical property values of the obtained compound.

### Pyrazole Derivative (I) Preparation Example 8

### Synthesis of 8-fluoro-4-methoxyimino-5-methyl-6-(1-ethyl-5-n-propanesulfonyloxypyrazol-4-yl)carbonylthiochroman1,1-dioxide (compound of the formula (If))

The captioned end product was obtained at an yield of 83 % in the same manner as in Pyrazole Derivative (I) Preparation Example 5 except that the starting material was replaced with the compound obtained in Pyrazole Derivative (I) Preparation Example 6 and that the reaction reagent was replaced with n-propanesulfonyl chloride. Table 5 shows the structures of the starting material and the reaction reagent and the structure and yield of the obtained compound. Table 6 shows the physical property values of the obtained compound.

### Pyrazole Derivative (I) Preparation Example 9

### Synthesis of 8-fluoro-4-methoxyimino-5-methyl-6-(1,3-dimethyl-5-hydroxypyrazol-4-yl)carbonylthiochroman-1,1-dioxide (compound of the formula (Ic))

The captioned end product was obtained at an yield of 65 % in the same manner as in Pyrazole Derivative (I) Preparation Example 6 except that the 1-ethyl-5-hydroxypyrazole used in Pyrazole Derivative (I) Preparation Example 6 was replaced with 1,3-dimethyl-5-hydroxypyrazole. Table 5 shows the structure of the starting material and the structure and yield of the obtained compound. Table 6 shows the physical property values of the obtained compound.

### Pyrazole Derivative (I) Preparation Example 10

### Synthesis of 8-fluoro-4-methoxyimino-5-methyl-6-(1,3-dimethyl-5-p-toluenesulfonyloxypyrazol-4-yl)carbonylthiochroman-1,1-dioxide (compound of the formula (If))

The captioned end product was obtained at an yield of 68 % in the same manner as in Pyrazole Derivative (I) Preparation Example 5 except that the starting material was replaced with the compound 9 obtained in Pyrazole Derivative (I) Preparation Example 9. Table 5 shows the structures of the starting material and the reaction reagent and the structure and yield of the obtained compound. Table 6 shows the physical property values of the obtained compound.

### Pyrazole Derivative (I) Preparation Example 11

### Synthesis of 8-fluoro-4-methoxyimino-5-methyl-6-(1,3-dimethyl-5-n-propanesulfonyloxypyrazol-4-yl)carbonylthiochroman-1,1-dioxide (compound of the formula (If))

The captioned end product was obtained at an yield of 68 % in the same manner as in Pyrazole Derivative (I) Preparation Example 5 except that the starting material was replaced with the compound 9 obtained in Pyrazole Derivative (I) Preparation Example 9 and that the reaction reagent was replaced with n-propanesulfonyl chloride. Table 5 shows the structures of the starting material and the reaction reagent and the structure and yield of the obtained compound. Table 6 shows the physical property values of the obtained compound.

### Pyrazole Derivative (I) Preparation Example 12

### Synthesis of 8-fluoro-4-methoxyimino-5-methyl-6-(1,3-dimethyl-5-cyclohexanecarbonyloxypyrazol-4-yl)carbonylthiochroman-1,1-dioxide (compound of the formula (If))

0.47 Gram (1.2 mmol) of the compound 9 obtained in Pyrazole Derivative (I) Preparation Example 9, as a starting material, was dissolved in 6 ml of dichloroethane. To this solution were added 0.21 g (1.5 mmol) of cyclohexanecarbonyl chloride and 0.29 ml (3.6 mmol) of pyridine, and the mixture was allowed to react at room temperature for 7 hours. After the completion of the reaction, 30 ml of water was added, and the mixture was extracted with methylene chloride three times. The resultant organic layer was washed with 5 % hydrochloric acid and with a saturated sodium chloride aqueous solution and dried over anhydrous sodium sulfate, and the solvent was distilled off under reduced pressure. The resultant oil was purified by silica gel column chromatography to give the captioned end product at an yield of 53 %. Table 5 shows the structures of the starting material and the reaction reagent and the structure and yield of the obtained compound. Table 6 shows the physical property values of the obtained compound.

### Pyrazole Derivative (I) Preparation Example 13

### Synthesis of 5-chloro-4-methoxyimino-6-(1-ethyl-5-hydroxypyrazol-4-yl)carbonylthiochroman-1,1-dioxide (compound of the formula (Ic))

The captioned end product was obtained at an yield of 83 % in the same manner as in Pyrazole Derivative (I) Preparation Example 1 except that the starting material was replaced with the 5-chloro-4-methoxyiminothiochroman-6-carboxylic acid-1,1-dioxide obtained in Carboxylic acid (III) Preparation Example 6. Table 5 shows the structure of the starting material and the structure and yield of the obtained compound. Table 6 shows the physical property values of the obtained compound.

### Pyrazole Derivative (I) Preparation Example 14

### Synthesis of 5-chloro-4-methoxyimino-6-(1-ethyl-5-n-propanesulfonyloxypyrazol-4-yl)carbonylthiochroman-1,1-dioxide (compound of the formula (If))

The captioned end product was obtained at an yield of 85 % in the same manner as in Pyrazole Derivative (I) Preparation Example 5 except that the starting material was replaced with the compound 13 obtained in Pyrazole Derivative (I) Preparation Example 13 and that the reaction reagent was replaced with n-propanesulfonyl chloride. Table 5 shows the structures of the starting material and the reaction reagent and the structure and yield of the obtained compound. Table 6 shows the physical property values of the obtained compound.

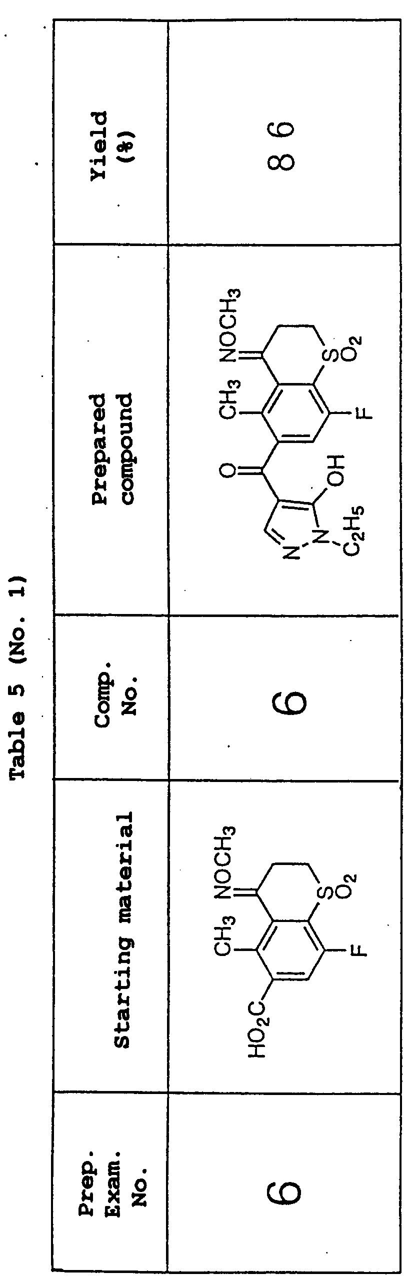

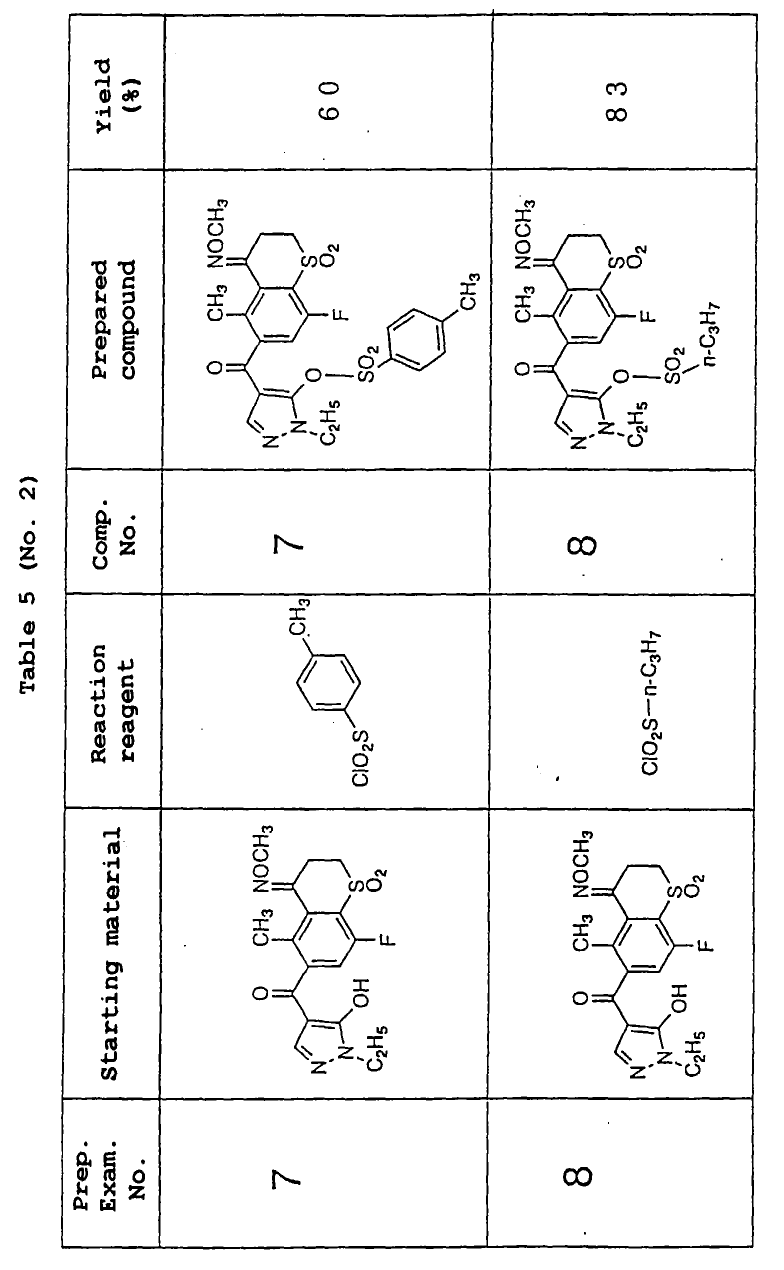

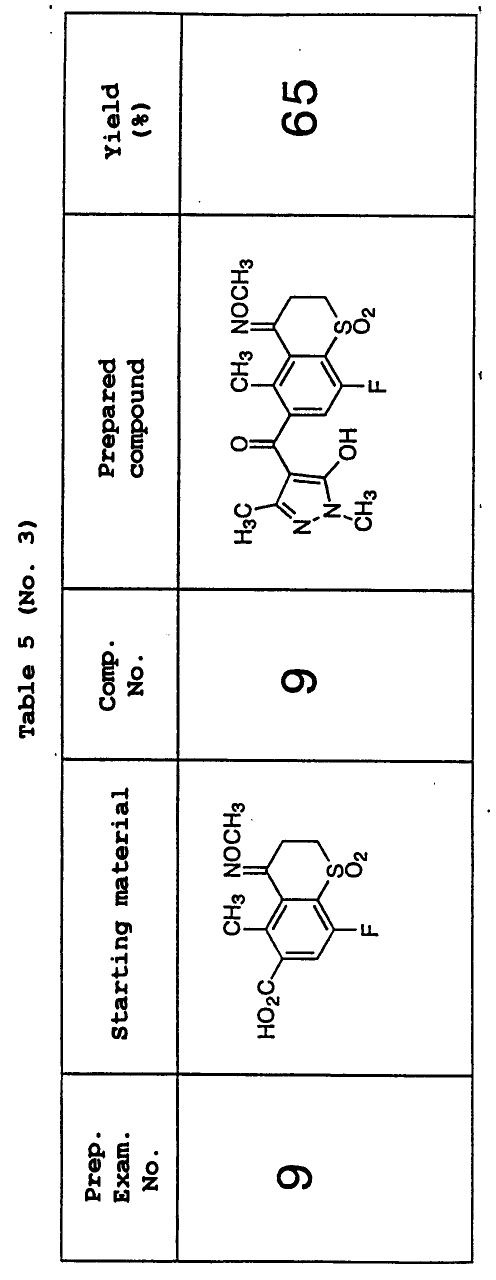

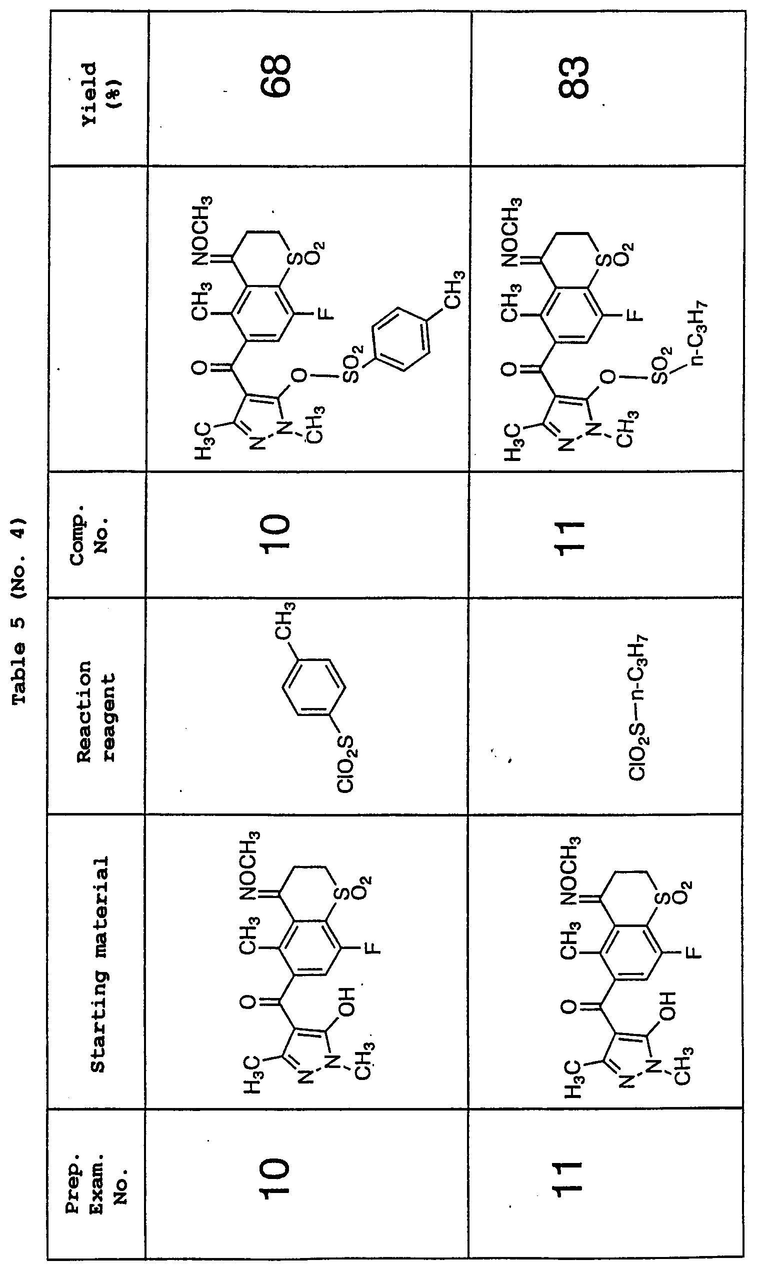

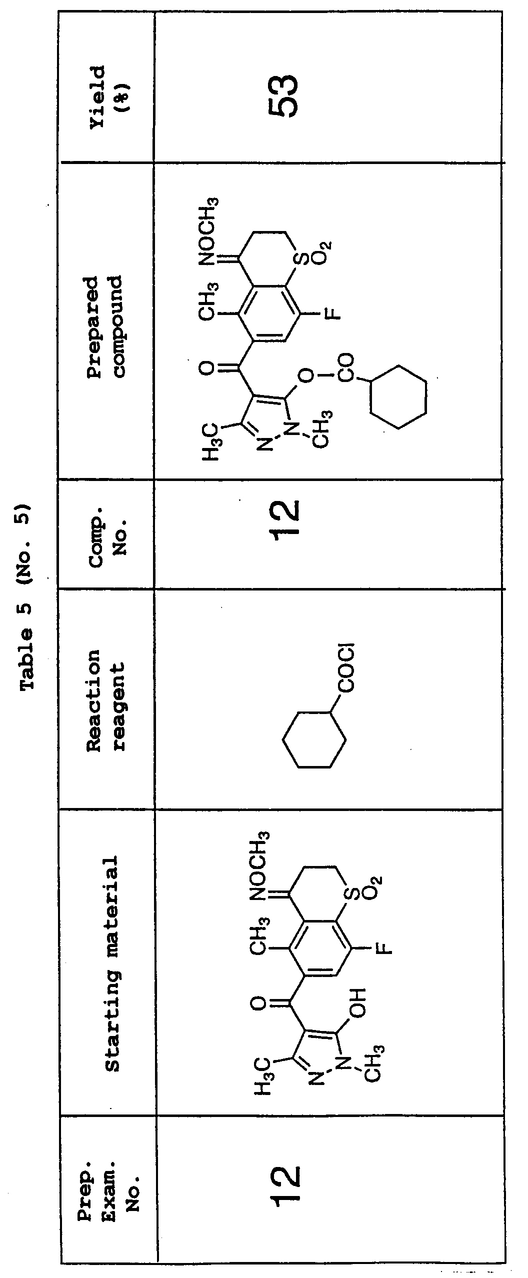

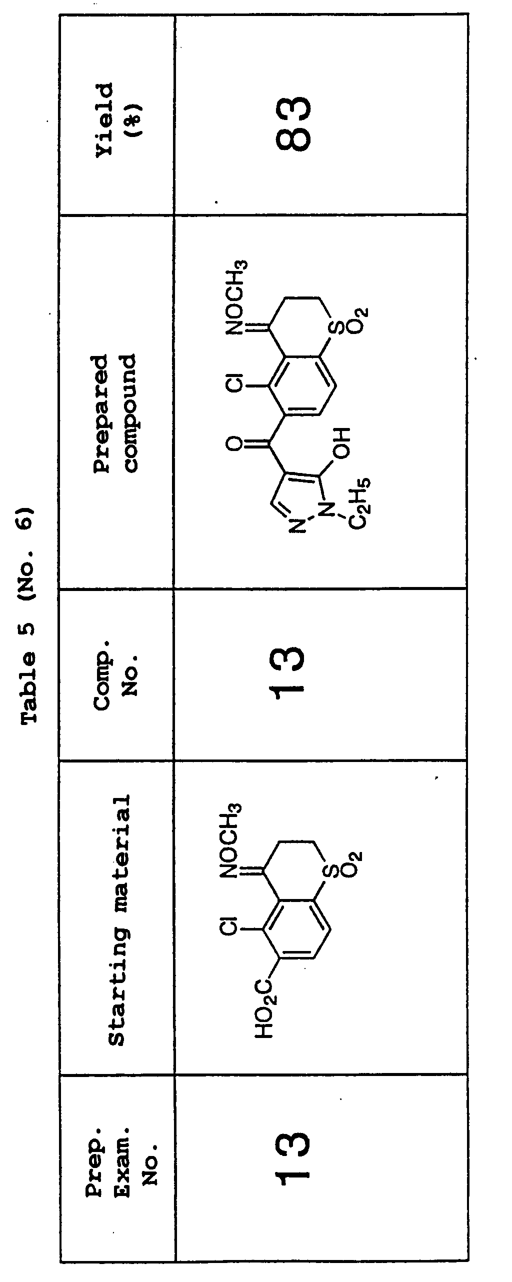

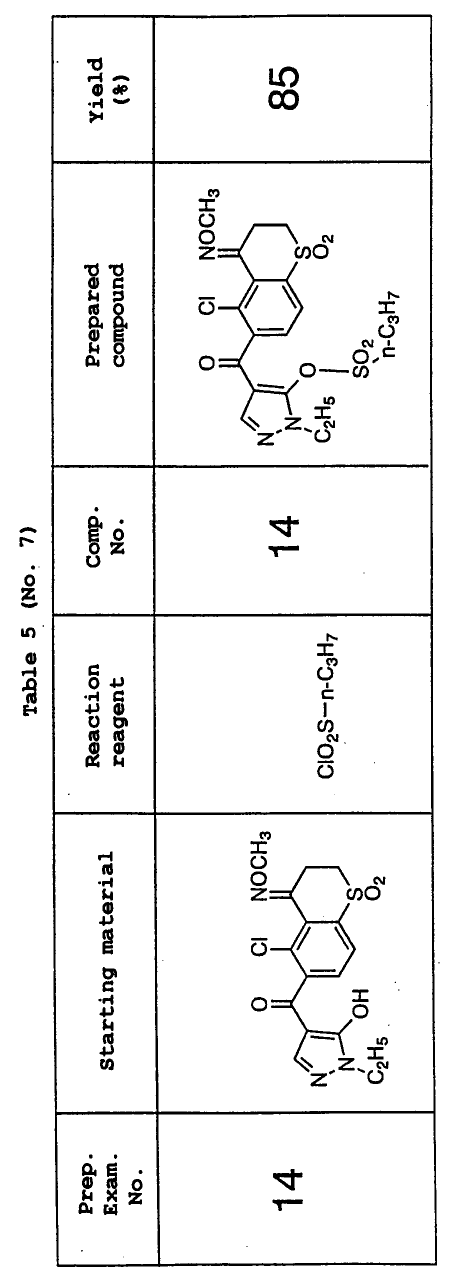

**Table 6 (No. 1)**

| Comp. No. | NMR(ppm) Int. standard: tetramethylsilane Solvent: deutero acetone | IR(cm⁻¹) KBr tablet | Melting point (°C) |
|---|---|---|---|
| 6 | 1.39(3H,t,J=7.3),2.44(3H,d),3.3∼3.7(4H,m), 4.05(3H,s),4.06(2H,q),6.90(1H, broad), 7.41(1H,d,J=9.9),7.44(1H,s) | --- | Glass-like substance |

**Table 6 (No. 2)**

| Comp. No. | NMR (ppm) Int. standard: Tetramethylsilane Solvent: Deutero chloroform | IR(cm⁻¹) KBr tablet | Melting point (°C) |
|---|---|---|---|
| 7 | 1.48(3H,t,J=7.3),2.41(3H,d),2.48(3H,s), 3.35∼3.39(4H,m), 4.05(3H,s),4.15(2H,q),6.97(1H,d,J=9.7), 7.41(2H,d,J=8.4),7.89(2H,d) | 3000 | Glass-like substance |
| | | 2960 | |
| | | 1680 | |
| | | 1385 | |
| | | 1320 | |
| | | 1205 | |
| | | 1135 | |
| 8 | 1.19(3H,t,J=7.6),1.52(3H,t,J=7.3), 2.12(2H,m),2.44(3H,d) 3.3∼3.5(4H,m),3.37(3H,t) 4.05(3H,s),4.22(2H,q),7.16(1H,d,J=9.3), 7.45(1H,s) | 3000 | Glass-like substance |
| | | 2960 | |
| | | 1680 | |
| | | 1395 | |
| | | 1190 | |
| 9 | 2.10(3H,s),2.44(3H,s) 3.3∼3.5(4H,m),3.72(3H,s), 4.06(3H,s)6.5(1H, broad) 7.10(1H,d,J=10.0) | 3000 | Glass-like substance |
| | | 1650 | |
| | | 1340 | |
| | | 1150 | |
| 10 | 2.30(3H,s),2.47(6H,s) 3.38(2H, broad),3.58(3H,s), 4.05(3H,s),6.95(1H,d,J=9.7) 7.38(2H,d,J=8.4),7.64(2H,d) | 2990 | Glass-like substance |
| | | 2900 | |
| | | 1680 | |
| | | 1405 | |
| | | 1340 | |
| | | 1200 | |
| | | 1150 | |
| 11 | 1.11(3H,t,),1.8∼2.2(2H,m),2.08(3H,s), 2.44(3H,s),3.2∼3.4(6H,m),3.81(3H,s) 4.05(3H,s),6.5(1H, broad), 7.12(1H,d,J=9.7) | 3000 | Glass-like substance |
| | | 2960 | |
| | | 1660 | |
| | | 1390 | |
| | | 1190 | |

**Table 6 (No. 3)**

| Comp. No. | NMR(ppm) Int. standard: tetramethylsilane Solvent: deutero chloroform | IR(cm⁻¹) KBr tablet | Melting point (°C) |
|---|---|---|---|
| 12 | 1.1∼2.0(13H,m),2.34(3H,s)2.39(3H,s) 3.3∼3.4(4H,m),3.57(3H,s),4.05(3H,s) 7.04(1H,d,J=9.5) | 2960 | Glass-like substance |
| | | 2850 | |
| | | 1790 | |
| | | 1670 | |
| | | 1350 | |
| | | 1140 | |

**Table 6 (No. 4)**

| Comp. No . | NMR(ppm) Int. standard: tetramethylsilane Solvent: deutero acetone | IR(cm⁻¹) KBr tablet | Melting point (°C) |
|---|---|---|---|
| 13 | 1.39(3H,t),3.25∼3.90(4H,m), 4.06(2H,q),4.06(3H,s)7.36(1H,s) 7.70(1H,d)8.00(1H,d) 9.16(1H, broad) | 1650 | Glass-like substance |
| | | 1550 | |
| | | 1350 | |
| | | 1190 | |
| | | 1060 | |
| 14 | 1.17(3H,t),1.51(3H,t),1.80∼2.30(2H,m) 3.37(4H,s),3.50∼3.95(2H,m),4.09(3H,s) 4.22(2H,q),7.47(1H,s),7.50(1H,d) 8.00(1H,d) | 3000,2950 | Glass-like substance |
| | | 1740,1680 | |
| | | 1550,1390 | |
| | | 1330,1180 | |
| | | 1060 | |

### Pyrazole Derivative (I) Preparation Example 15

### Synthesis of 4-propargyloxyimino-5-methyl-6-(1-ethyl-5-propanesulfonyloxypyrazol-4-yl)carbonylthiochroman-1,1-dioxide (compound of the formula (Ic))

### (1) Esterification step (step 1a)

1.8 Grams (5.9 mmol) of the 4-propargyloxyimino-5-methylthiochroman-6-carboxylic acid-1,1-dioxide obtained in Carboxylic Acid (III) Preparation Example 7 was suspended in 20 ml of 1,2-dichloroethane, 1 ml (14 mmol) of thionyl chloride was added, and the mixture was stirred at 70°C for 3 hours. The solvent was distilled off from the reaction mixture to obtain a corresponding acid chloride. Separately, 0.9 g (8 mmol) of 1-ethyl-5-hydroxypyrazole and 0.8 g (8 mmol) of triethylamine were dissolved in 20 ml of tetrahydrofuran and cooled in ice bath. To this solution was gradually added 10 ml of the above-prepared tetrahydrofuran solution of acid chloride, and the mixture was allowed to stand at room temperature overnight. The reaction mixture was diluted with 150 ml of ethyl acetate. The resultant organic layer was washed with 100 ml of water three times and with 100 ml of a saturated sodium hydrogencarbonate aqueous solution once, and dried over anhydrous sodium sulfate, and the solvent was distilled off. The resultant dark brown oily substance was purified by silica gel column chromatography (silica gel/ethyl acetate: n-hexane = 1:1) to give 0.5 g (yield 21 %) of 4-propargyloxyimino-5-methyl-6-(1-ethylpyrazol-5-yloxycarbonyl)thiochroman-1,1-dioxide in the form of a yellow paste.
NMR (ppm, solvent: deutero chloroform, internal standard: tetramethylsilane): 1.45(3H,t, J = 7), 2.53(1H,t, J = 2), 2.82(3H,s), 3.41(4H,s), 4.10(2H,q, J = 7), 4.85(2H,d, J =2), 6.29(1H,d, J = 2), 7.50(1H,d, J = 2), 8.04(2H,s)

### (2) Rearrangement step and step of introducing group of -A-B (step 1b and step 2)

0.5 Gram (1.2 mmol) of the 4-propargyloxyimino-5-methyl-6-(1-ethylpyrazol-5-yloxycarbonyl)thiochroman 1,1-dioxide obtained in the above esterification step and 0.15 g (1.5 mmol) of triethylamine were dissolved in 6 ml of acetonitrile, and to this solution was added four drops of acetonecyanhydrin. The mixture was allowed to stand at room temperature overnight. The reaction mixture was distilled to remove the solvent, and the resultant red oily substance was dissolved in 20 ml of water. The mixture was acidified by adding 5 % hydrochloric acid and extracted with 100 ml of dichloromethane. The resultant organic layer was dried over anhydrous sodium sulfate, and the solvent was distilled off to give a red glass-like solid. The solid was dissolved in 3 ml of dichloromethane. 0.02 Gram of benzyltriethylammonium chloride, a solution of 0.2 g (1.4 mmol) of a potassium carbonate in 3 ml of water and 0.2 g (1.4 mmol) of propanesulfonyl chloride were added, and the mixture was stirred at room temperature for 8 hours and allowed to stand overnight. The reaction mixture was diluted with 100 ml of ethyl acetate. The resultant organic layer was washed with 50 ml of a saturated sodium hydrogencarbonate aqueous solution and with 50 ml of water, and dried over anhydrous sodium sulfate, and the solvent was distilled off to give a brown oily substance. The oily substance was purified by silica gel column chromatography (silica gel/ethyl acetate:n-hexane = 1:1) to give 0.47 g (yield 77 %) of 4-propargyloxyimino-5-methyl-6-(1-ethyl-5-propanesulfonyloxypyrzol-4-ylcarbonyl)thiochroman-1,1-dioxide in the form of a yellowish glass-like solid.

Table 7 shows the structure of the starting material used in this Preparation Example and the structure and yield of the compound obtained in this Preparation Example. Table 8 shows the physical property values of the obtained compound.

### Pyrazole Derivative (I) Preparation Example 16

### Synthesis of 4-allyloxyimino-5-methyl-6-(1-ethyl-5-propanesulfonyloxypyrazol-4-ylcarbonyl)thiochroman-1,1-dioxide (compound of the formula (If))

### (1) Esterification step and rearrangement step (step 1ab)

1.5 Grams (yield 83 %) of 4-allyloxyimino-5-methyl-6-(1-ethyl-5-hydroxypyrazol-4-ylcarbonyl)-thiochroman-1,1-dioxide was obtained in the same manner as in Pyrazole Derivative (I) Preparation Example 1 except that the starting material was replaced with the compound 8 obtained in Carboxylic Acid (III) Preparation Example 8.
NMR (ppm, solvent: deutero chloroform, internal standard: tetramethylsilane): 1.47(3H,t, J = 7), 2.56(3H,s), 3.39(4H,s), 4.08(2H,q, J = 7), 4.75(2H,d, J = 6), 5.2-5.4(2H,m), 5.8-6.2(1H,m), 7.31(1H,s), 7.52(1H,d, J = 8), 7.97(1H,d, J = 8)

### (2) Step of introducing group of -A-B (step 2)

1.1 Grams (yield 58 %) of the captioned compound was obtained in the same manner as in Pyrazole Derivative (I) Preparation Example 5 except that the starting material was replaced with the 4-allyloxyimino-5-methyl-6-(1-ethyl-5-hydroxypyrazol-4-ylcarbonyl)thiochroman-1,1-dioxide obtained in the above (1) and that n-propanesulfonyl chloride was used as a reaction reagent.

Table 7 shows the structures of the starting material and the reaction reagent and the structure and yield of the obtained compound. Table 8 shows the physical property values of the obtained compound.

### Pyrazole Derivative (I) Preparation Example 17

### Synthesis of 4-(2-fluoroethyl)oxyimino-5-methyl-6-(1-ethyl-5-propanesulfonyloxypyrazol-4-ylcarbonyl)-thiochroman-1,1-dioxide (compound of the formula (If)

### (1) Esterification step and rearrangement step

1.3 Grams (yield 81 %) of 4-(2-fluoroethyl)oxyimino-5-methyl-6-(1-ethyl-5-hydroxypyrazol-4-ylcarbonyl)-thiochroman-1,1-dioxide was obtained in the same manner as in Pyrazole Derivative (I) Preparation Example 1 except that the starting material was replaced with the compound 9 obtained in Carboxylic Acid (III) Preparation Example 9.
NMR (ppm, solvent: deutero chloroform, internal standard: tetramethylsilane): 1.46(3H,t, J = 7), 2.57(3H,s), 3.39(4H,t, J = 4), 4.08(2H,q, J = 7), 4.2-5.0(4H,m), 7.31(1H,s), 7.54(1H,d, J = 8), 7.98(1H,d, J = 8)

### (2) Step of introducing group of -A-B (Step 2)

0.8 Gram (yield 49 %) of the captioned end product was obtained in the same manner as in Pyrazole Derivative (I) Preparation Example 5 except that the starting material was replaced with the 4-(2-fluoroethyl)oxyimino-5-methyl-6-(1-ethyl-5-hydroxypyrazol-4-ylcarbonyl) thiochroman-1,1-dioxide and that n-propanesulfonyl chloride was used as a reaction reagent.

Table 7 shows the structures of the starting material and the reaction reagent and the structure and yield of the obtained compound. Table 8 shows the physical property values of the obtained compound.

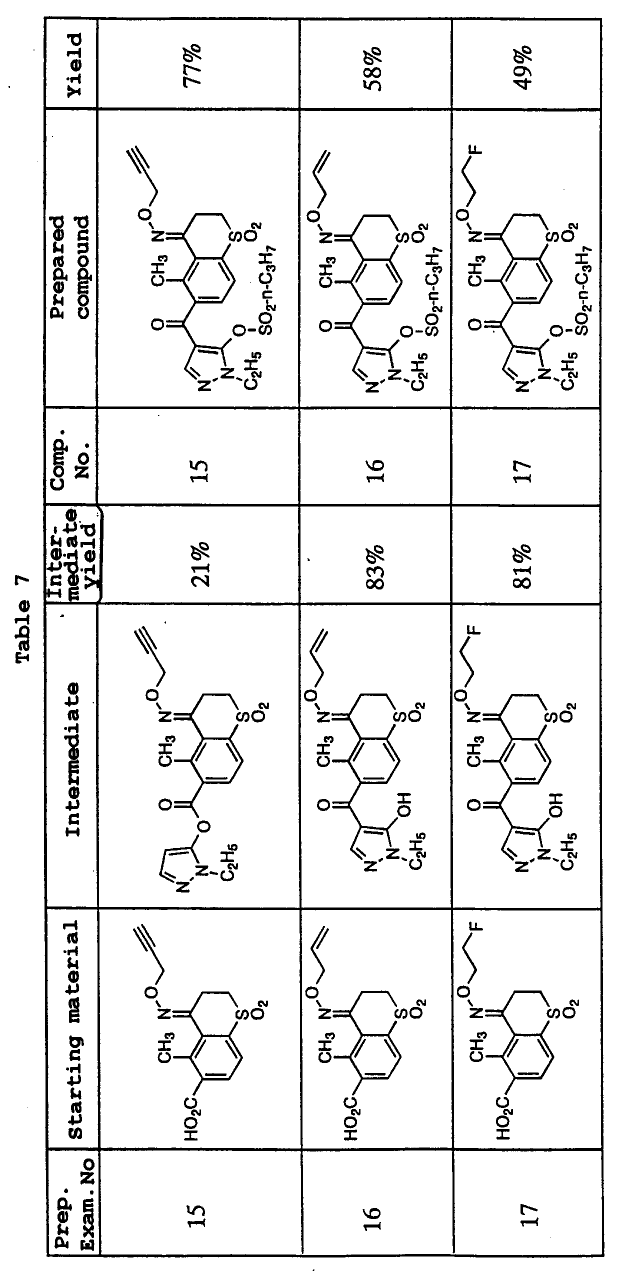

**Table 8**

| Comp. No. | NMR (ppm) Int. standard: tetramethylsilane Solvent: deutero chloroform | IR(cm⁻¹) KBr tablet | Melting point (°C) |
|---|---|---|---|
| 15 | 1.84(3H, t, J=7), 1.52(3H, t, J=7), 1.9-2.3(2H, m), 2.49(1H, t, J=2), 2.54(3H, s), 3.39(4H, s), 3.78(2H, q, J=7), 4.23(2H, q, J=7), 4.83(2H, d, J=2) 7.44(1H, s), 7.48(1H, d, J=7), 7.97(1H, d, J=7) | 3250, 2950 | Glass-like solid |
| | | 2900, 1640 | |
| | | 1520, 1500 | |
| | | 1150,1100 | |
| | | 1000, 800 | |
| 16 | 1.18(3H, t, J=7), 1.52(3H, t, J=7), 1.9-2.3(2H, m), 2.50(3H, s), 3.38(4H, t, J=4), 3.73(2H, t, J=8), 4.23(2H, q, J=7), 4.74(2H, d, J=6), 5.2-5.4(2H, m), 5.8-6.2(1H, m), 7.44(1H, s), 7.46(1H, d, J=8), 7.96(1H, d, J=8) | 3000, 2960 | 94-99 |
| | | 1670, 1560 | |
| | | 1390, 1320 | |
| | | 1300, 1180 | |
| 17 | 1.18(3H, t, J=7), 1.52(3H, t, J=7), 1.9-2.3(2H, m), 2.50(3H, s), 3.37(2H, d, J=4), 3.43(2H, d, J=4), 3.74(2H, t, J=7), 4.23(2H, q, J=7), 4.3-4.5(2H, m), 4.79(2H, dt, J=30,4), 7.43(1H, s), 7.48(1H, d, J=8), 7.97(1H, d, J=8) | 3000, 2950 | 143-149 |
| | | 1680, 1550 | |
| | | 1390, 1330 | |
| | | 1180, 900 | |
| | | 840 | |

### Herbicide Examples

### (1) Preparation of herbicides

97 Parts by weight of talc (trade name: Zeaklite) as a carrier, 1.5 parts by weight of alkylarylsulfonic acid (trade name: Neoplex, supplied by Kao-Atlas K.K.) as a surfactant and 1.5 parts by weight of a nonionic and anionic surfactant (trade name: Sorpol 800A, supplied by Toho Chemical Co., Ltd.) were uniformly pulverized and mixed to prepare a carrier for a wettable powder.

90 Parts by weight of the above carrier and 10 parts by weight of one of the compounds obtained in the above Preparation Examples (or 10 parts by weight of the following compound (A) for Comparative Example) were uniformly pulverized and mixed to obtain herbicides.

The compound (A) used as a comparative chemical is disclosed in International Laid-open Patent Publication No. WO94/01431 and has the following structure.

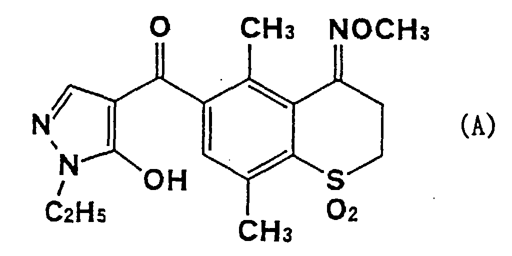

The compound of the present invention can be used for any one of soil treatment, soil-mixing treatment and foliar treatment as a herbicide for upland soil. Examples of the cropland weeds which the compound of the present invention can control include broad-leaved weeds such as
solanaceous weeds typified by black nightshade (Solanum nigrum) and jimsonweed (Datura stramonium),
malvaceous weeds typified by velvetleaf (Abutilon theophrasti) and pricky sida (Side spinosa),
convolvulaceous weeds typified by morning-glories (Ipomoea spps.) such as tall morning-glory (Ipomoea purpurea) and bindweeds (Calystegia spps.),
amaranthaceous weeds typified by livid amaranth (amaranthus lividus),
composite weeds typified by cocklebur (Xanthium strumarium), common ragweed (Ambrosia artemisiaefolia), sunflower (Helianthus annus), hairy galinsoga (Galinsoga ciliat), Canada thistle (Cirsium arvense), groundsel (Sencio vulgaris) and annual fleabane (Erigeron annus),
cruciferous weeds typified by yellow cress(Rorippa indica), wild mustard (Sinapis arvensis) and shepherdspurse (Capsella bursapastris),
polygonaceous weeds typified by smartweed (Polygonumblumei) and black bindweed (Polygonumconvolvulus),
portulacaceous weeds typified by common purslane (Portulaca oleracea),
chenopodiaceous weeds typified by common lambsquaters (white-goose-foot or green pigweed)(Chenopodium album), fig-leaved goosefoot (Chenopodium ficifolium) and kochia (Kochia scoparia)
caryophyllaceous weeds typified by common chickweed (Stellaria media),
scrophulariaceous weeds typified by birdseye (Veronica persica),
commelinaceous weeds typified by Asiatic dayflower (Commelina communis),
labiate weeds typified by henbit (Lamium amplexicaule) and purple deadnettle (Lamium purpureum),
euphorbiaceous weeds typified by milk purslane (Euphorbia supina) and spotted spurge (Euphorbia maculate),
rubiaceous weeds typified by bedstraw (Galium spurium), cleavers (Galium aparine) and madder (Rubia akane),
violaceous weeds typified by violet (Viola arvensis), and
leguminous weeds typified by hemp sesbania (Sesbania exaltata) and sicklepod (Cassia obtusifolia);
graminaceous weeds typified by shattercane (Sorgham bicolor), fall panicum (Panicum dichotomiflorum), Johnsongrass (Sorghum halepense), barnyardgrass (Echinochloa crus-galli), Henry crabgrass (Digitaria adscendens), wildoat (Avena fatua), goosegrass (Eleusine indica), green foxtail (Setaria viridis) and blackgrass (Alopecurus aegualis); and
cyperaceous weeds typified by purple nutsedge (Cyperus rotundus, Cyperus esculentus).

The compound of the present invention can be used for any one of soil treatment and foliar treatment under submergence as a herbicide for paddy land. Examples of paddy weeds include
alismataceous weeds typified by oriental waterplantain (Alisma canaliculatum), arrowhead (Sagittaria trifolia) and Sagittaria pygmaea,
cyperaceous weeds typified by umbrella plant (Cyperus difformis), Cyperus serotinus, bulrush (Scirpus juncoides) and water chestnut (Eleochadaris kuroguwai),
scrothulariaceous weeds typified by common falsepimpernel (Lindemia pyxidaria),
potenderiaceous weeds typified by monochoria (Monochoria Vaginalis),
potamogetonaceous weeds typified by largeleaf pondweed (Potamogeton distinctus),
lythraceous weeds typified by toothcup (Rotala indica), and
graminaceous weeds typified by barnyardgrass (Echinochloa crus-galli).

Further, the compound of the present invention can be applied to non-agricultural fields such as sports grounds, vacant land, railroad sides, etc., in addition to upland field, paddy land and orchards.

The usefulness of the compound of the present invention will be specifically explained hereinafter on the basis of the following tests.

### (2) Biological test (Foliar treatment test)

Seeds of predetermined weeds selected from large crabgrass, barnyardgrass, green foxtail, cocklebur, velvetleaf, slender amaranth, Jimsonweed, common chickweed, giant foxtail and wildoat and seeds of corn, wheat and barley were sown in 1/5,000-are Wagner pots filled with upland soil, and covered with upland soil. Then, the seeds were grown in a greenhouse. When these plants were at their three- to four-leaved stage, a predetermined amount of the herbicide obtained in the above (1) was suspended in water and uniformly sprayed to their leaves and stalks at an amount of 2,000 liters/hectare Thereafter, the plants were grown in the greenhouse, and 30 days after the treatment, the herbicide was evaluated for herbicidal efficacy and phytotoxicity to crops. Tables 9 and 10 show the results.

The herbicidal efficacy and the phytotoxicity to the crops are shown as follows.

| (Ratings) | |
|---|---|
| Herbicidal efficacy | Ratio of remaining plant weight to non-treated (%) |
| 0 | 81 - 100 |
| 1 | 61 - 80 |
| 2 | 41 - 60 |
| 3 | 21 - 40 |
| 4 | 1 - 20 |
| 5 | 0 |

| Phytotoxicity to crops | Ratio of remaining plant weight to non-treated (%) |
|---|---|
| - | 100 |
| ± | 95 - 99 |
| + | 90 - 94 |
| ++ | 80 - 89 |
| +++ | 0 - 79 |

The ratio of remaining plant weight to non-treated was determined as a ratio of remaining plant weight to non-treated = (remaining plant weight in treated plot/remaining plant weight in non-treated plot) x 100.

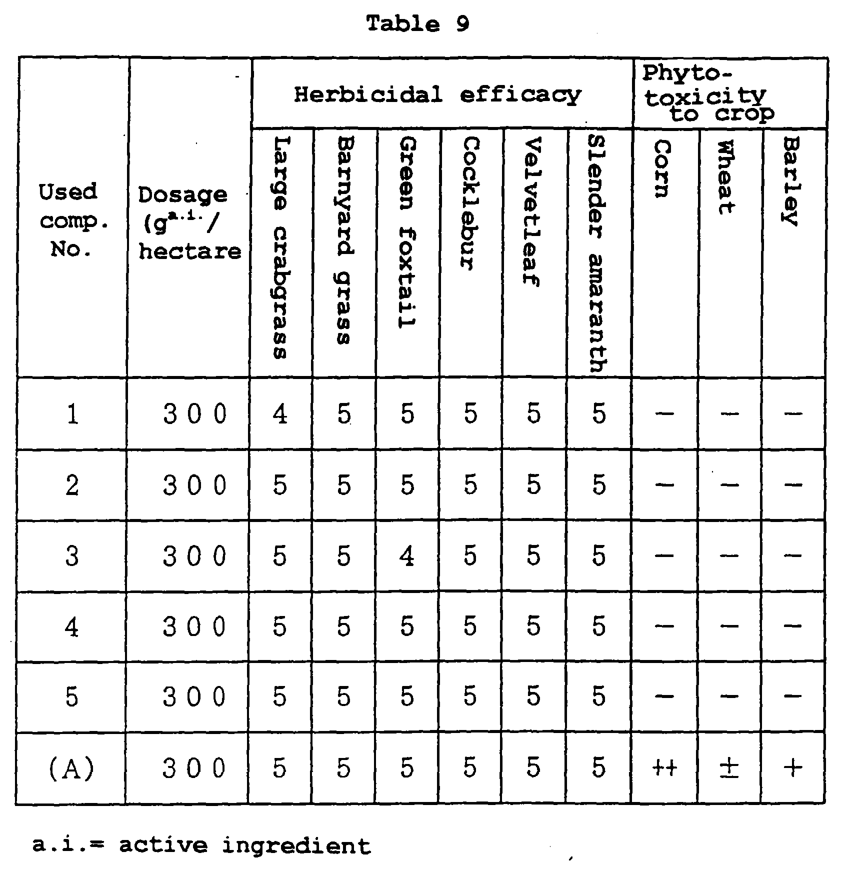

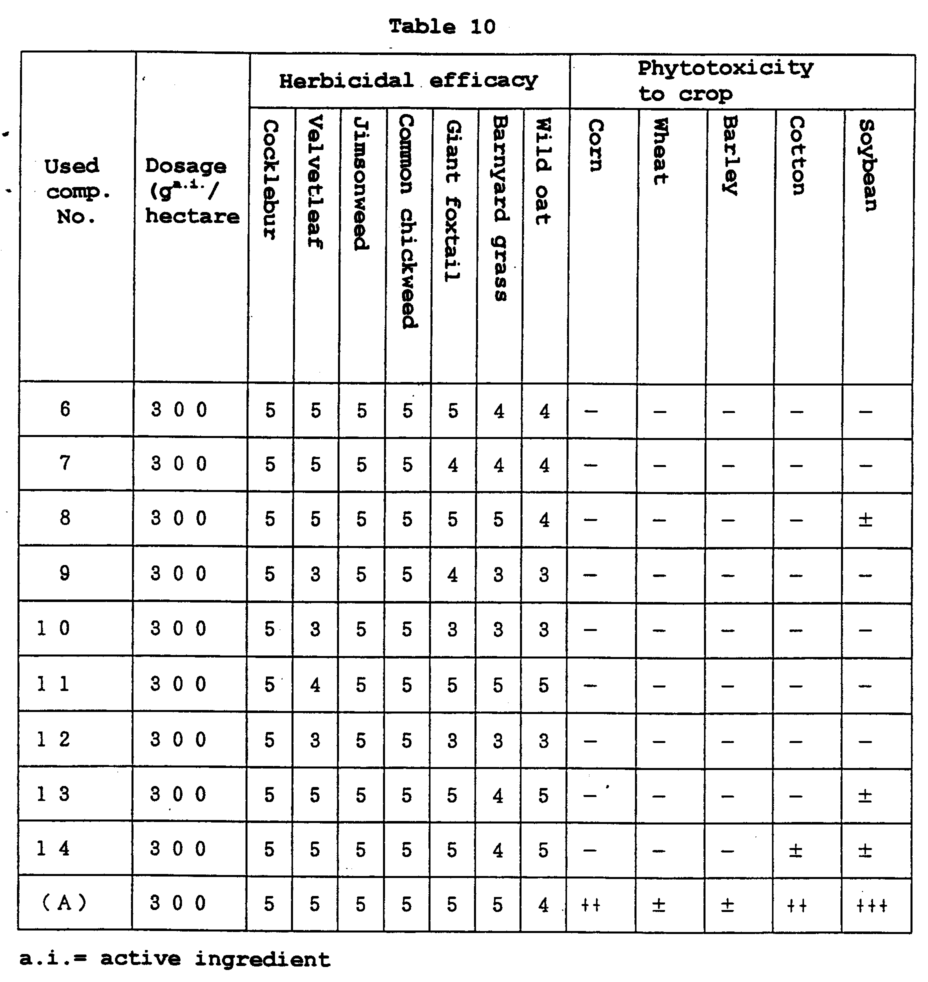

The results in Tables 9 and 10 show the following. In the foliar treatment at a three and four-leaved stage, the comparative herbicide causes phytotoxicity on crops, while all the herbicides of the present invention are free from phytotoxicity to the crops and have high safety to the crops. Further, the herbicides of the present invention exhibit excellent herbicidal efficacy to various weeds and show excellent selectivity between crops and weeds.

### (3) Biological test (Soil treatment test)

Seeds of weeds such as cocklebur, velvetleaf, Jimsonweed, common chickweed, giant foxtail, barnyardgrass and wildoat and seeds of corn, wheat, barley and cotton were sown in 1/5,000-are Wagner pots filled with upland soil, and covered with upland soil. Then, a predetermined amount of the herbicide prepared in the above (1) was suspended in water and uniformly sprayed onto the soil surface at an amount of 2,000 liters/hectare. Thereafter, the seeds were grown in a greenhouse, and 20 days after the treatment, the herbicide was determined for herbicidal efficacy and phytotoxicity to crops. Table 11 shows the results.

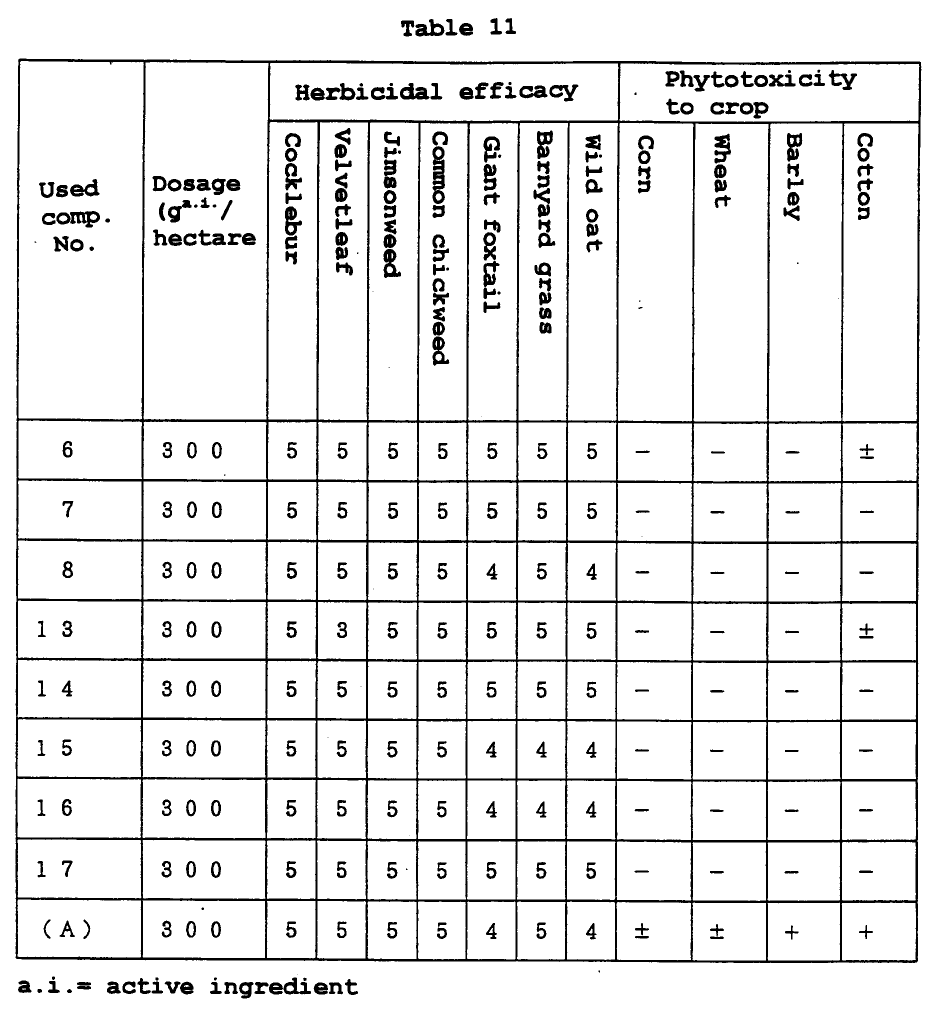

The results in Table 11 show the following. In the soil treatment test, the comparative herbicide causes phytotoxicity on crops, while all the herbicides of the present invention are free from phytotoxicity to the crops and have high safety to the crops. Further, the herbicides of the present invention exhibit excellent herbicidal efficacy to various weeds and show excellent selectivity between crops and weeds.

As explained above, according to the present invention, there are provided novel pyrazole derivatives which remarkably excellent selectivity between crops and weeds in foliar treatment and soil treatment, and herbicides containing them as active ingredients.

## Claims

1. A pyrazole derivative of the formula (I), 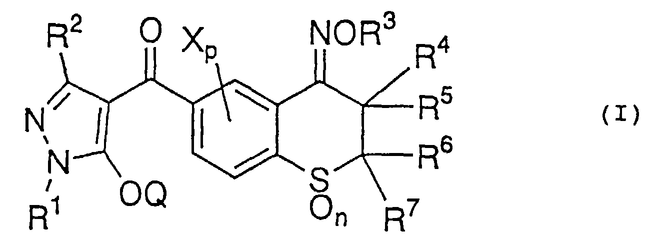 wherein:
R¹ is a C₁∼C₄ alkyl group, a C₂∼C₄ alkenyl group or a C₂∼C₄ haloalkenyl group;
R² is a hydrogen atom, a C₁∼C₄ alkyl group, a C₁∼C₄ haloalkyl group or a C₂∼C₄ alkoxyalkyl group;
X is a C₁∼C₄ alkyl group, a C₁∼C₄ haloalkyl group, a C₂∼C₄ alkoxyalkyl group, a halogen atom, a C₁∼C₄ alkoxy group or a C₁∼C₄ haloalkoxy group;
p is an integer of 0, 1 or 2;
R³ is a C₁∼C₄ alkyl group, a C₁∼C₄ haloalkyl group, a C₃-C₆ cycloalkyl group, a C₃∼C₆ alkenylalkyl group, a C₃∼C₆ alkynylalkyl group, a C₃∼C₆ haloalkenylalkyl group or a C₂∼C₄ alkoxyalkyl group;
each of R⁴, R⁵, R⁶ and R⁷ is independently a hydrogen atom, a C₁∼C₄ alkyl group, a C₁∼C₄ haloalkyl group or a C₂∼C₄ alkoxyalkyl group;
n is an integer of 0, 1 or 2; and
Q is a hydrogen atom or a group of -A-B,
in which A is 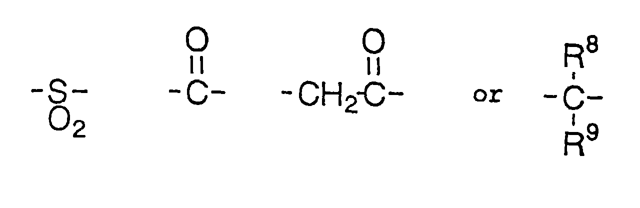
in which each of R⁸ and R⁹ is independently a hydrogen atom or a C₁∼C₄ alkyl group; and
B is a C₁∼C₁₂ alkyl group, a C₃∼C₁₀ cycloalkyl group or a group of 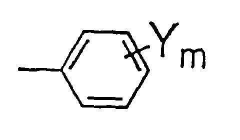
in which Y is a C₁∼C₄ alkyl group, a C₁∼C₄ alkoxy group, a C₁∼C₄ haloalkyl group, a nitro group or a halogen atom; and
m is an integer of 0, 1 or 2;
provided that compounds of the formula (I) wherein p is 1, R³ is a C₁∼C₄ alkyl group, X is a C₁∼C₄ alkyl group and all of R⁴, R⁵, R⁶ and R⁷ are hydrogen atoms and compounds of the formula (I) wherein p is 2, R³ is a C₁∼C₄ alkyl group, two Xs are both C₁∼C₄ alkyl groups and all of R⁴, R⁵, R⁶ and R⁷ are hydrogen atoms are excluded.

2. The pyrazole derivative of claim 1, wherein R¹ is a C₁∼C₄ alkyl group.

3. The pyrazole derivative of claim 2, wherein R¹ is methyl or ethyl.

4. The pyrazole derivative of claim 1, wherein R² is a C₁∼C₄ alkyl group.

5. The pyrazole derivative of claim 1, wherein R³ is a C₁∼C₄ alkyl group, a C₁∼C₄ haloalkyl group, a C₃∼C₆ alkenylalkyl group or a C₃∼C₆ alkynylalkyl group.

6. The pyrazole derivative of claim 5, wherein R³ is methyl, -CH₂CH₂F, allyl or propargyl.

7. The pyrazole derivative of claim 1, wherein each of R⁴, R⁵, R⁶ and R⁷ is independently a hydrogen atom or a C₁∼C₄ alkyl group.

8. The pyrazole derivative of claim 1, wherein p is 1 and X is a C₁∼C₄ alkyl group, a C₁∼C₄ haloalkyl group, a halogen atom or a C₁∼C₄ alkoxy group substituted on the 5-position on the thiochroman ring.

9. The pyrazole derivative of claim 8, wherein X is methyl, trifluoromethyl, a chlorine atom or methoxy.

10. The pyrazole derivative of claim 1, wherein p is 2 and one X is a C₁∼C₄ alkyl group, a halogen atom or a C₁∼C₄ haloalkyl group substituted on the 5-position on the thiochroman ring, and the other X is a halogen atom, a C₁∼C₄ alkoxy group or a C₁∼C₄ alkyl group substituted on the 8-position on the thiochroman ring.

11. The pyrazole derivative of claim 10, wherein one X is methyl, a chlorine atom, trifluoromethyl or methoxy substituted on the 5-position on the thiochroman ring and the other X is a chlorine atom, a fluorine atom, methoxy or methyl substituted on the 8-position on the thiochroman ring.

12. The pyrazole derivative of claim 1, wherein n is 0 or 2.

13. The pyrazole derivative of claim 1, wherein Q is a hydrogen atom.

14. The pyrazole derivative of claim 1, wherein Q is a group of -A-B in which A is

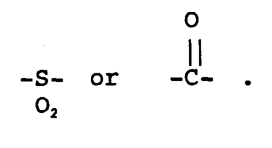

15. The pyrazole derivative of claim 1, wherein q is a group of -A-B in which B is selected from the group consisting of a C₁∼C₄ alkyl group, a C₃∼C₇ Cycloalkyl group or a group of 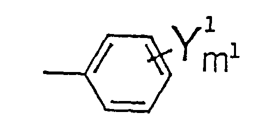 in which Y¹ is a C₁∼C₄ alkyl group or a halogen atom and m¹ is an integer of 0, 1 or 2.

16. The pyrazole derivative of claim 15, wherein Q is a group of -A-B in which B is selected from the group consisting of propyl, cyclohexyl and toluyl.

17. The pyrazole derivative of claim 1, wherein Q is a group of -A-B in which a combination of A and B is a group selected from the class consisting of

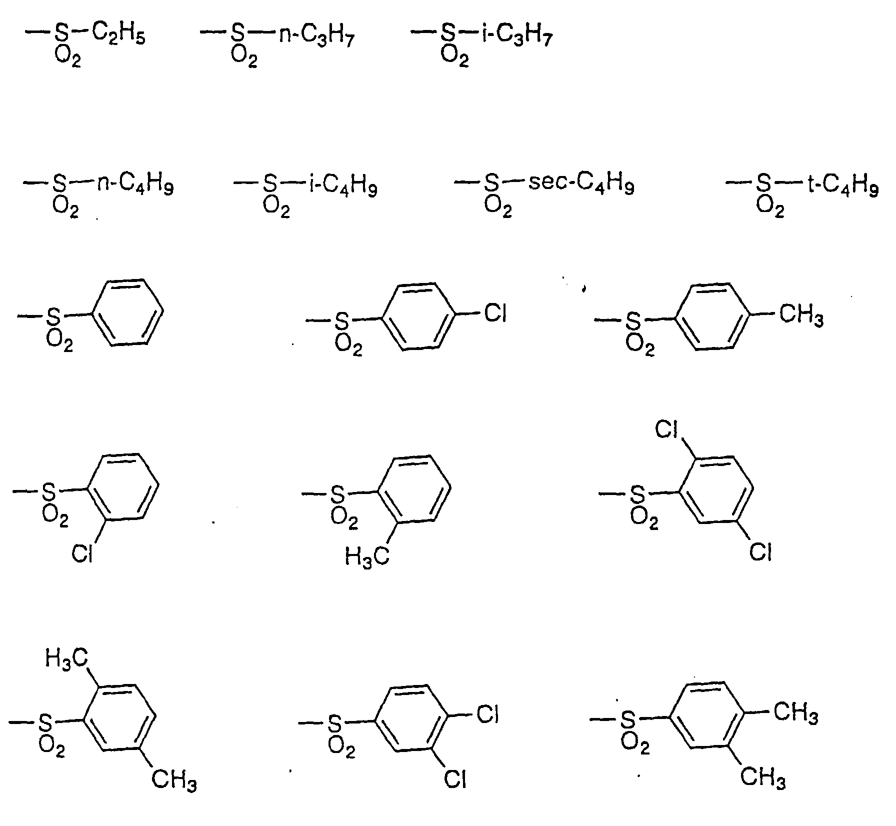

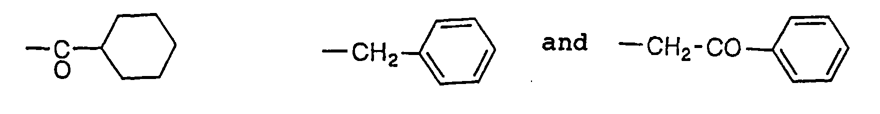

18. The pyrazole derivative of claim 17, wherein Q is a group of -A-B in which the combination of A and B is a group selected from the class consisting of

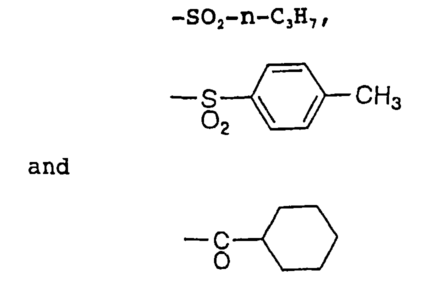

19. A carboxylic acid of the formula (III), 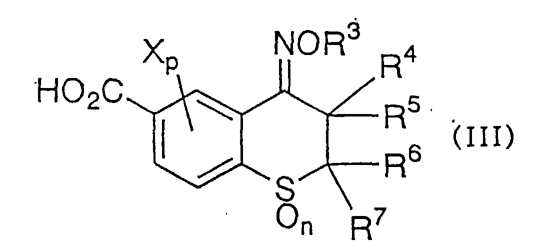 wherein:
X is a C₁∼C₄ alkyl group, a C₁∼C₄ haloalkyl group, a C₂-C₄ alkoxyalkyl group, a halogen atom, a C₁∼C₄ alkoxy group or a C₁∼C₄ haloalkoxy group;
p is an integer of 0, 1 or 2;
R³ is a C₁∼C₄ alkyl group, a C₁∼C₄ haloalkyl group, a C₃∼C₆ cycloalkyl group, a C₃∼C₆ alkenylalkyl group, a C₃∼C₆ alkynylalkyl group, a C₃∼C₆ haloalkenylalkyl group or a C₂∼C₄ alkoxyalkyl group;
each of R⁴, R⁵, R⁶ and R⁷ is independently a hydrogen atom, a C₁∼C₄ alkyl group, a C₁∼C₄ haloalkyl group or a C₂∼C₄ alkoxyalkyl group; and
n is an integer of 0, 1 or 2;
provided that compounds of the formula (III) wherein p is 1, R³ is a C₁∼C₄ alkyl group, X is a C₁∼C₄ alkyl group and all of R⁴, R⁵, R⁶ and R⁷ are hydrogen atoms and compounds of the formula (III) wherein p is 2, R³ is a C₁∼C₄ alkyl group, two Xs are both C₁∼C₄ alkyl groups and all of R⁴, R⁵, R⁶ and R⁷ are hydrogen atoms are excluded.

20. A herbicide containing, as an active ingredient, the pyrazole derivative of the general formula (I), recited in any one of claims 1 to 18.
